# EUROPEAN PATENT SPECIFICATION

(11) **EP 2 635 299 B1**
(45) Date of publication and mention of the grant of the patent: **31.07.2019**
(21) Application number: 11838771.1
(22) Date of filing: 02.11.2011
(51) Int. Cl.: A61P 17/14

(54) **METHODS FOR TREATING HAIR LOSS DISORDERS**
VERFAHREN ZUR BEHANDLUNG VON HAARAUSFALL
MÉTHODES DE TRAITEMENT DE TROUBLES CAPILLAIRES

(30) Priority: 02.11.2010 US 409509 P
(43) Date of publication of application: 11.09.2013
(73) Proprietor: The Trustees of Columbia University in the City of New York, New York, NY 10027 (US)
(72) Inventor: CHRISTIANO, Angela, M., Mahwah, NJ 07430 (US); CLYNES, Raphael, West Nyack, NY 10994 (US)
(74) Representative: Barker Brettell LLP
(86) International application number: PCT/US2011/059029
(87) International publication number: WO 2012/061537

(56) References cited:
- EP-A1- 1 176 140
- WO-A1-2010/093808
- US-A1- 2003 044 803
- DELAMERE F M ET AL: "Interventions for alopecia areata.", THE COCHRANE DATABASE OF SYSTEMATIC REVIEWS 2008, no. 2, 2008, page CD004413, XP009182992, ISSN: 1469-493X
- PETUKHOVA LYNN ET AL: "Genome-wide association study in alopecia areata implicates both innate and adaptive immunity.", NATURE 1 JUL 2010, vol. 466, no. 7302, 1 July 2010 (2010-07-01), pages 113-117, XP002736853, ISSN: 1476-4687
- ITO TAISUKE ET AL: "Maintenance of hair follicle immune privilege is linked to prevention of NK cell attack.", THE JOURNAL OF INVESTIGATIVE DERMATOLOGY MAY 2008, vol. 128, no. 5, May 2008 (2008-05), pages 1196-1206, XP002736854, ISSN: 1523-1747
- CAILLAT-ZUCMAN ET AL: "How NKG2D Ligands Trigger Autoimmunity?", HUMAN IMMUNOLOGY, NEW YORK, NY, US, vol. 67, no. 3, 1 March 2006 (2006-03-01), pages 204-207, XP024993277, ISSN: 0198-8859, DOI: 10.1016/J.HUMIMM.2006.02.013 [retrieved on 2006-03-01]
- HORNG TIFFANY ET AL: "NKG2D signaling is coupled to the interleukin 15 receptor signaling pathway.", NATURE IMMUNOLOGY DEC 2007, vol. 8, no. 12, December 2007 (2007-12), pages 1345-1352, XP002736855, ISSN: 1529-2916
- HORDINSKY MARIA K: "Treatment of alopecia areata: "What is new on the horizon?".", DERMATOLOGIC THERAPY 2011 MAY-JUN, vol. 24, no. 3, May 2011 (2011-05), pages 364-368, XP002736856, ISSN: 1529-8019
- XING LUZHOU ET AL: "Alopecia areata is driven by cytotoxic T lymphocytes and is reversed by JAK inhibition", NATURE MEDICINE, vol. 20, no. 9, 17 August 2014 (2014-08-17), pages 1043-1049, XP002736857, ISSN: 1078-8956
- SUMMARY OF THE 2010 ALOPECIA AREATA CLINICAL/TRANSLATIONAL RESEARCH SUMMIT. 22 October 2010, XP055106797 Retrieved from the Internet: <URL:http://www.naaf.org/site/PageServer?pa gename=2010> [retrieved on 2012-02-16]
- HAREL SIVAN ET AL: "Pharmacologic inhibition of JAK-STAT signaling promotes hair growth.", SCIENCE ADVANCES OCT 2015, vol. 1, no. 9, October 2015 (2015-10), page e1500973,
- Kerstin Foitzik ET AL: "Human Scalp Hair Follicles Are Both a Target and a Source of Prolactin, which Serves as an Autocrine and/or Paracrine Promoter of Apoptosis-Driven Hair Follicle Regression", AMERICAN JOURNAL OF PATHOLOGY., vol. 168, no. 3, 1 March 2006 (2006-03-01) , pages 748-756, XP055469860, US ISSN: 0002-9440, DOI: 10.2353/ajpath.2006.050468
- Yann F. Mahe ET AL: "Androgenetic alopecia and microinflammation", INTERNATIONAL JOURNAL OF DERMATOLOGY, vol. 39, no. 8, 1 August 2000 (2000-08-01) , pages 576-584, XP055096339, UK ISSN: 0011-9059, DOI: 10.1046/j.1365-4362.2000.00612.x

## Description

This application claims priority to United States Provisional Application No. 61/409,509, which was filed November 2, 2010.

### BACKGROUND OF THE INVENTION

The scope of the invention is defined by the appended claims. Alopecia Areata (AA) is one of the most highly prevalent autoimmune diseases, leading to hair loss due to the collapse of immune privilege of the hair follicle and subsequent autoimmune destruction. AA is a skin disease which leads to hair loss on the scalp and elsewhere. In some severe cases, it can progress to complete loss of hair on the head or body. Although Alopecia Areata is believed to be caused by autoimmunity, the gene level diagnosis and treatment are seldom reported. The genetic basis of AA is largely unknown.

### SUMMARY OF THE INVENTION

An aspect of the invention encompasses a Jak1 and/or Jak2 inhibitor selected from the group consisting of: AG490 with the CAS No. 133550-30-8; CYT387 with the CAS No. 1056634-68-4; SB1518 with the CAS No. 937272-79-2; TG101348 with the CAS No. 936091-26-8; BMS-911543 with the CAS No. 1271022-90-2; WP-1034 with the CAS No. 857064- 42-7; CEP-701 with the CAS No. 111358-88-4; INCB 018424 with the CAS No. 941678-49-5); and LY3009104 with the CAS No. 1187594-09-7 for use in the treatment of a hair-loss disorder selected from the group consisting of alopecia areata and androgenetic alopecia in a mammalian subject in need thereof. The disclosure relates to an inhibitor that is a Stat1 and/or a Stat2 inhibitor. In a further embodiment, the inhibitor is INCB 018424. The Jak1 and/or Jak2 for use according to the invention, wherein treatment is achieved by inducing hair growth in the subject, the disclosure relates to hair-loss disorders comprising, telogen effluvium, telogen effluvium, tinea capitis, hypotrichosis or hereditary hypotrichosis simplex. In one embodiment, the Jak1 and/or Jak2 for use according to the invention, wherein alopecia areata is alopecis universalis or alopecia totalis. In one embodiment, the Jak1 and/or Jak2 for use according to the invention, further comprising the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak1 or Jak2 protein; a siRNA that specifically targets the the gene that encodes the Jak1 or Jak2 protein; or a small molecule. The disclosure relates to an antisense RNA that specifically inhibits expression of the gene that encodes the Stat1 or Stat2 protein; a siRNA that specifically targets the the gene that encodes the Stat1 or Stat2 protein; or a small molecule. In one embodiment, the small molecule is AG490 with the CAS No. 133550-30-8; CYT387 with the CAS No. 1056634-68-4; SB1518 with the CAS No. 937272-79-2; TG101348 with the CAS No. 936091-26-8; BMS-911543 with the CAS No. 1271022-90-2; WP-1034 with the CAS No. 857064- 42-7; CEP-701 with the CAS No. 111358-88-4; INCB 018424 with the CAS No. 941678-49-5); and LY3009104 with the CAS No. 1187594-09-7. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NOS: 2, 4, 6 or 8. In some embodiments, the siRNA directed to a Jak1 gene is any one of the sequences listed in Table 11. The disclosure relates to an siRNA directed to a Stat1 gene that is any one of the sequences listed in Table 12.

The disclosure relates to a method for inducing hair growth in a subject where the method comprises administering to the subject an effective amount of an inhibitor of a protein tyrosine kinase (PTK) involved in cytokine signaling. In one embodiment, the inhibitor is a Jak/Stat inhibitor. In other embodiments, the inhibitor is INCB 018424. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak1 or Jak2 protein; a siRNA that specifically targets the the gene that encodes the Jak1 or Jak2 protein; or a small molecule. In another embodiment, the inhibitor is an antisense RNA that specifically inhibits expression of the gene that encodes the Stat1 or Stat2 protein; a siRNA that specifically targets the the gene that encodes the Stat1 or Stat2 protein; or a small molecule. In one embodiment, the small molecule is AG490, CYT387, SB1518, LY3009104, TG101348, BMS-911543, or CEP-701. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NOS: 2, 4, 6 or 8. In some embodiments, the siRNA directed to a Jak1 gene is any one of the sequences listed in Table 11. In other embodiments, the siRNA directed to a Stat1 gene is any one of the sequences listed in Table 12.

The disclosure relates to a method of treating a hair-loss disorder in a mammalian subject in need thereof, the method comprising administering to the subject a Jak1 inhibitor. In one embodiment, the inhibitor is an antibody or antibody fragment that is directed to SEQ ID NO: 1. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak1 protein; a siRNA that specifically targets the the gene that encodes the Jak1 protein; or a small molecule. In one embodiment, the small molecule is AG490, CYT387, SB1518, LY3009104, TG101348, BMS-911543, or CEP-701. In another embodiment, the siRNA is directed to a human nucleic acid sequence comprising SEQ ID NO: 2. In some embodiments, the siRNA directed to a Jak1 gene is any one of the sequences listed in Table 11.

The disclosure relates to a method for inducing hair growth in a subject where the method comprises administering to the subject an effective amount of a Jak1 inhibitor, thereby controlling hair growth in the subject. In one embodiment, the inhibitor comprises an antibody that specifically binds to a protein comprising SEQ ID NO: 1. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak1 protein; a siRNA that specifically targets the the gene that encodes the Jak1 protein; or a small molecule. In one embodiment, the small molecule is AG490, CYT387, SB1518, LY3009104, TG101348, BMS-911543, or CEP-701. In another embodiment, the siRNA is directed to a human nucleic acid sequence comprising SEQ ID NO: 2. In some embodiments, the siRNA directed to a Jak1 gene is any one of the sequences listed in Table 11.

The disclosure relates to a method of treating a hair-loss disorder in a mammalian subject in need thereof, the method comprising administering to the subject a Jak 2 inhibitor. In one embodiment, the inhibitor is an antibody or antibody fragment that is directed to SEQ ID NO: 3. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak2 protein; a siRNA that specifically targets the the gene that encodes the Jak2 protein; or a small molecule. In one embodiment, the small molecule is AG490, CYT387, SB1518, LY3009104, TG101348, BMS-911543, or CEP-701. In another embodiment, the siRNA is directed to a human nucleic acid sequence comprising SEQ ID NO: 4.

The disclosure relates to a method for inducing hair growth in a subject where the method comprises administering to the subject an effective amount of a Jak 2 inhibitor, thereby controlling hair growth in the subject. In one embodiment, the inhibitor comprises an antibody that specifically binds to a protein comprising SEQ ID NO: 3. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Jak2 protein; a siRNA that specifically targets the the gene that encodes the Jak2 protein; or a small molecule. In one embodiment, the small molecule is AG490, CYT387, SB1518, LY3009104, TG101348, BMS-911543, or CEP-701. In another embodiment, the siRNA is directed to a human nucleic acid sequence comprising SEQ ID NO: 4.

The discslosure relates to a method of treating a hair-loss disorder in a mammalian subject in need thereof, the method comprising administering to the subject a Stat 1 inhibitor. In one embodiment, the inhibitor is an antibody or antibody fragment that is directed to SEQ ID NO: 5. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Stat1 protein; a siRNA that specifically targets the the gene that encodes the Stat1 protein; or a small molecule. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NO: 6. In other embodiments, the siRNA directed to a Stat1 gene is any one of the sequences listed in Table 12.

The disclosure relates to a method for inducing hair growth in a subject where the method comprises administering to the subject an effective amount of a Stat 1 inhibitor, thereby controlling hair growth in the subject. In one embodiment, the inhibitor comprises an antibody that specifically binds to a protein comprising SEQ ID NO: 5. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Stat1 protein; a siRNA that specifically targets the the gene that encodes the Stat1 protein; or a small molecule. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NO: 6. In other embodiments, the siRNA directed to a Stat1 gene is any one of the sequences listed in Table 12.

The disclosure relates to a method of treating a hair-loss disorder in a mammalian subject in need thereof, the method comprising administering to the subject a Stat 2 inhibitor. In one embodiment, the inhibitor is an antibody or antibody fragment that is directed to SEQ ID NO: 7. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Stat2 protein; a siRNA that specifically targets the the gene that encodes the Stat2 protein; or a small molecule. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NO: 8.

The disclosure relates to a method for inducing hair growth in a subject where the method comprises administering to the subject an effective amount of a Stat 2 inhibitor, thereby controlling hair growth in the subject. In one embodiment, the inhibitor comprises an antibody that specifically binds to a protein comprising SEQ ID NO: 7. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis. In one embodiment, the method further comprises the step (b) determining whether the inhibitor administered induced hair growth in the subject afflicted with a hair loss disorder as compared to the subject's hair growth prior to treatment with the inhibitor. In one embodiment, the inhibitor is an antisense RNA that specifically inhibits expression of the gene that encodes the Stat2 protein; a siRNA that specifically targets the the gene that encodes the Stat2 protein; or a small molecule. In another embodiment, the small molecule is WP-1034 (Faderl et al., Anticancer Res. 2005 May-Jun;25(3B):1841-50), fludarabine (Fludara, Berlex, CA), epigallocatechin-3-gallate (EGCG), or Hyperforin. In another embodiment, the siRNA is directed to a human nucleic acid sequence comprising SEQ ID NO: 8.

In a further embodiment, the Jak1 and/or Jak2 inhibitor is in a composition suitable for subcutaneous, intra-muscular, intra-peritoneal, or intravenous injection; an infusion; oral, nasal, intradermal, subcutaneous, trans-mucosal, rectal or topical delivery; or a combination thereof. In some embodiments, the administering occurs daily, weekly, twice weekly, monthly, twice monthly, or yearly.

### BRIEF DESCRIPTION OF THE FIGURES

**FIG. 1** are photographic images of clinical manifestations of AA. In the upper panels (**FIGS. 1A-B**), patients with AA multiplex. In **FIG. 1B**, the patient is in regrowth phase. For patients with alopecia universalis, there is a complete lack of body hair and scalp hair **(****FIG. 1C****),** while patients with alopecia totalis only lack scalp hair **(FIG. ID).** In **FIG. ID,** hair regrowth is observed in the parietal region, while no regrowth in either occipital or temporal regions is evident.

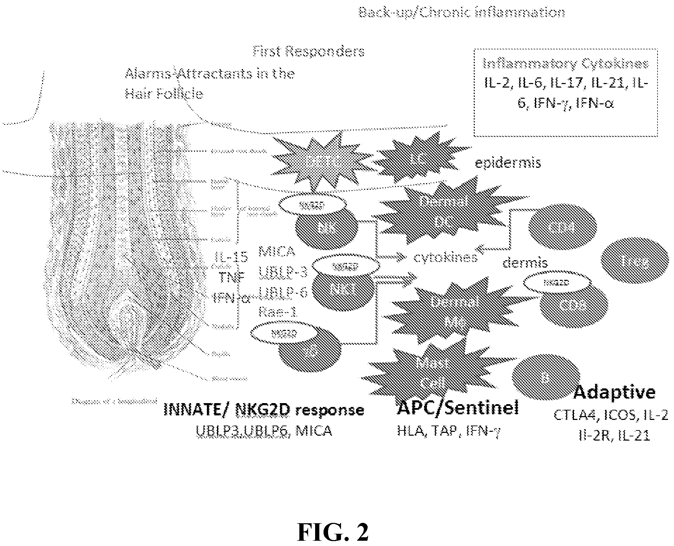

**FIG. 2** shows a diagram showing the inflammatory response underlying Alopecia Areata (AA).

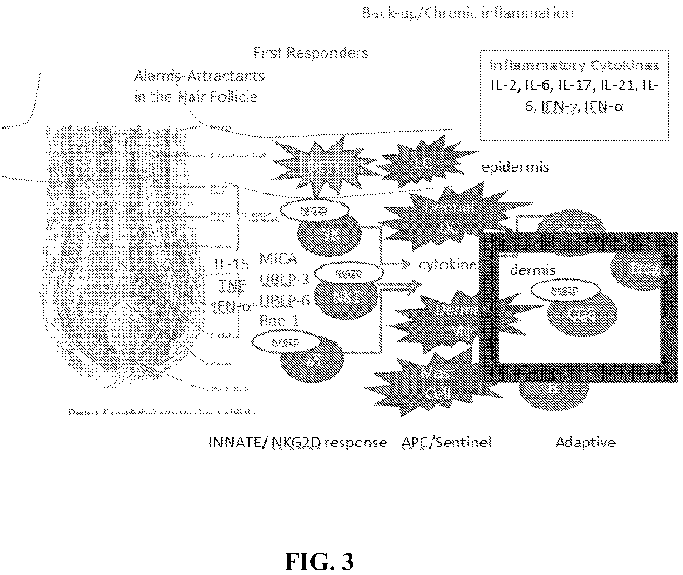

**FIG. 3** shows a diagram showing the inflammatory response underlying Alopecia Areata (AA).
**FIG. 4A** is a photograph of lymph nodes and spleen isolated from C3H mice afflicted with AA (top) and control mice (bottom).
**FIG. 4B** show the results from FACS analysis that NKG2D bearing CD8 and Th1 cells are expanded in murine AA cutaneous lymph nodes.
**FIG. 4C** is a bar graph that shows a 6-fold increase in the number of cytokine producing cells in C3H mice afflicted with AA as compared to control mice.

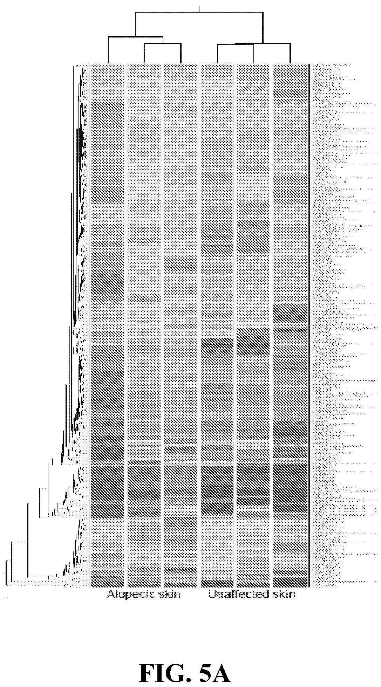

**FIG. 5A** is a RNA signature of alopecic skin in C3H mice. The heat map shows that 16 of the top 20 unregulated genes are Interferon-Response Genes. 16 of top 20 upregulated genes are Interferon Responsive Genes (IFN-γ but not Type I IFNs are elevevated)

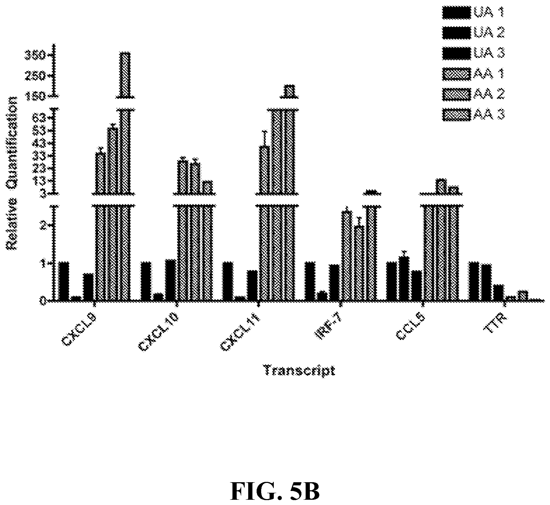

**FIG. 5B** shows transcriptional profiling of AA skin. qPCR verification of single genes picked from the list generated shows an average fold change over 3 experiments.

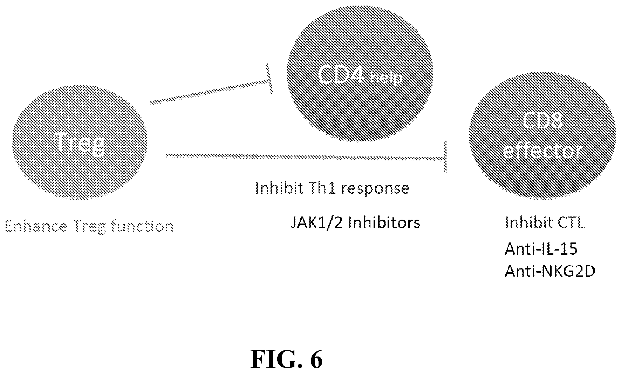

**FIG. 6** is a diagram showing the targets in AA.
**FIG. 7** shows a diagram showing the interferon γ pathway.

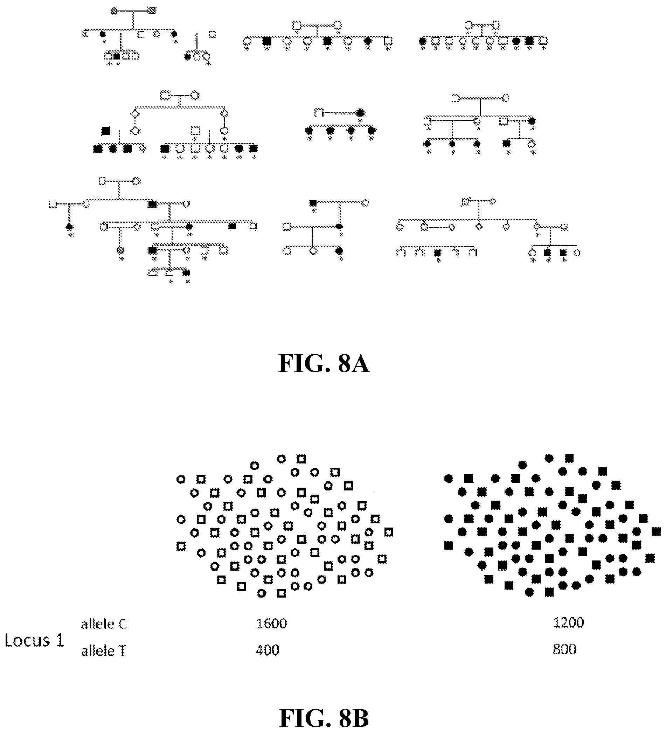

**FIGS. 8A-B** shows two gene mapping approaches. **FIG 8A** shows linkage analysis in families. **FIG. 8B** shows association analysis in a population cohort.
**FIG. 9** shows a Genomewide Association Study (GWAS) in AA.

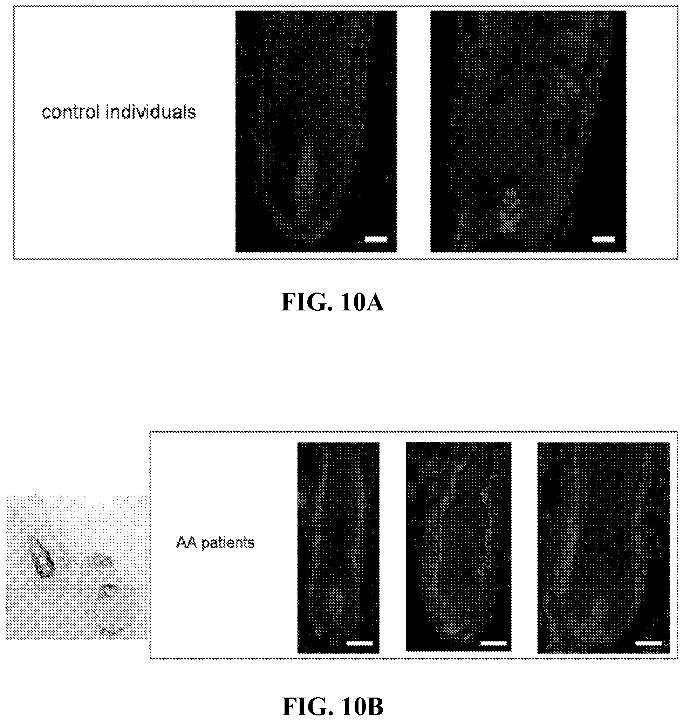

**FIGS. 10A-B** show danger signals: ULBP3 expression in the hair follicle in both control individuals (**FIG. 10A****)** and AA patients (**FIG. 10B**).

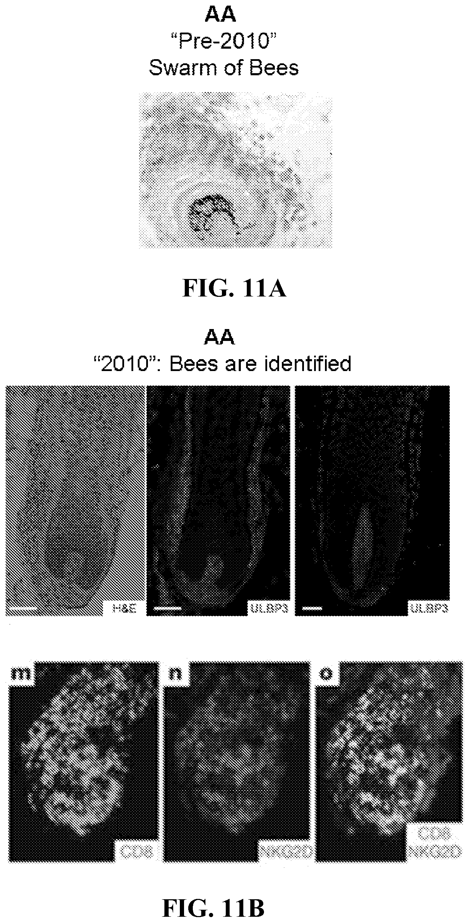

**FIGS. 11A-B** show that AA is caused by a "swarm of bees": Killer CD8 T cells attracted by NKG2DL. **FIG. 11A** shows "pre-2010" swarm of bees in AA. **FIG. 11B** shows "2012" identification of bees ((m) staining of CD8; (n) staining of NKG2D; and (o) colocalization of NKG2D and CD8).

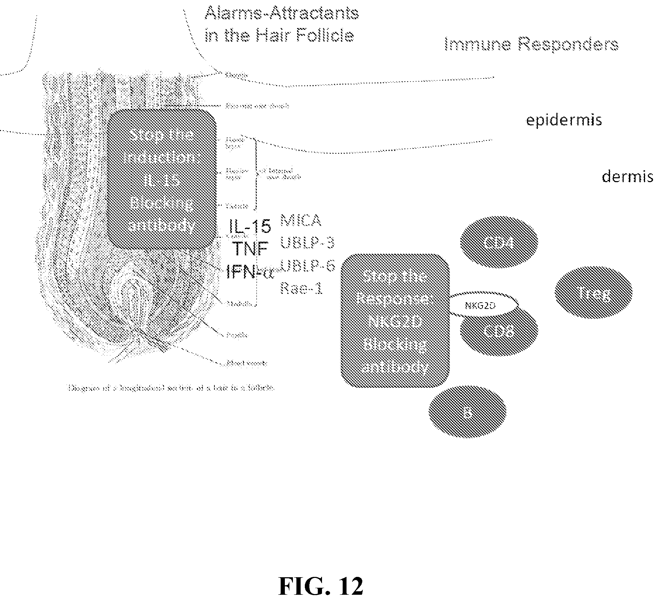

**FIG. 12** shows a diagram that shows the inflammatory response underlying AA.

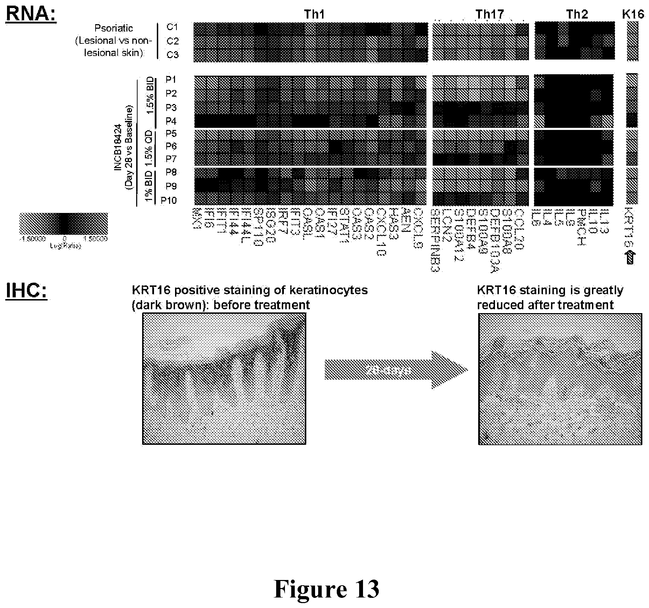

**FIG. 13** shows INCB18424-202: Th1 and Th17 transcript markers and keratin 16 are decreased with topical INCB 18424, a JAK1 and JAK2 inhibitor, treatment.

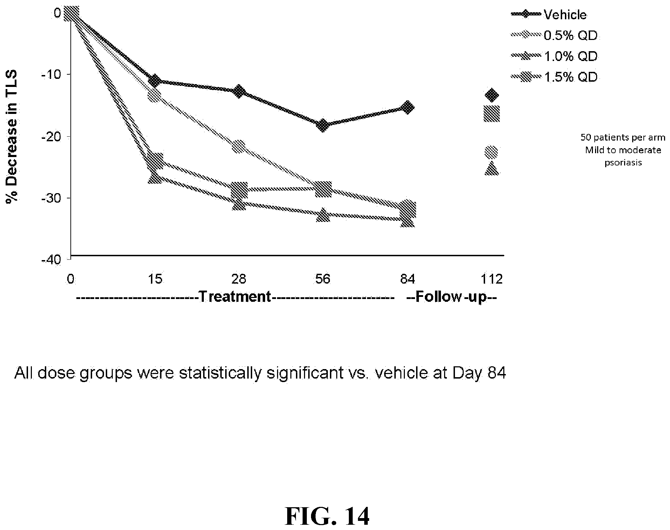

**FIG. 14** shows the Key Total Lesion Score results for INCB 18424-203. The results how that Total Lesion Score for all dose groups ≥ 2x decrease over vehicle, achieving significance after control for multiplicity

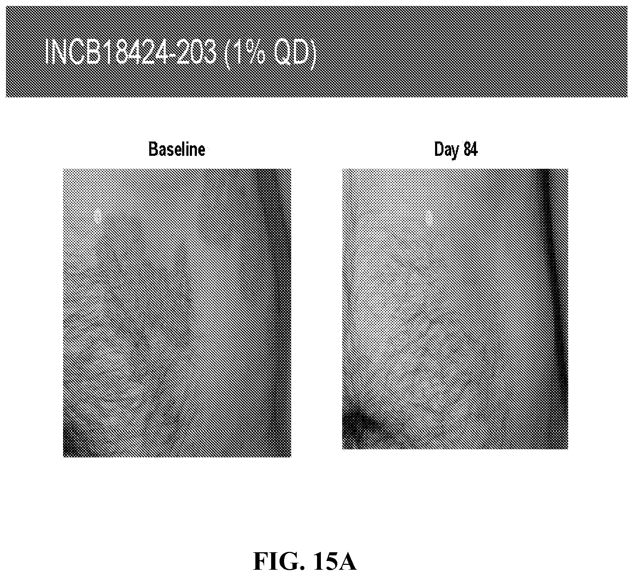

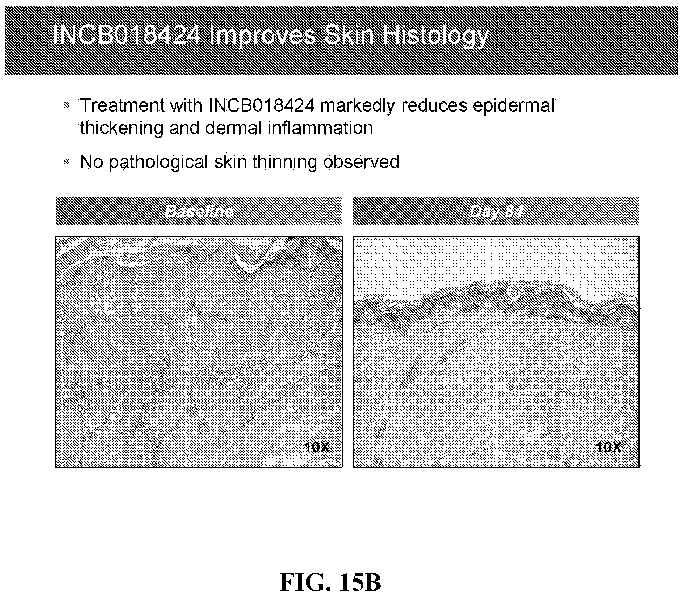

**FIGS. 15A-B** show that treatment with INCB018424 markedly reduces epidermal thickening and dermal inflammation. No pathological skin thinning is observed.

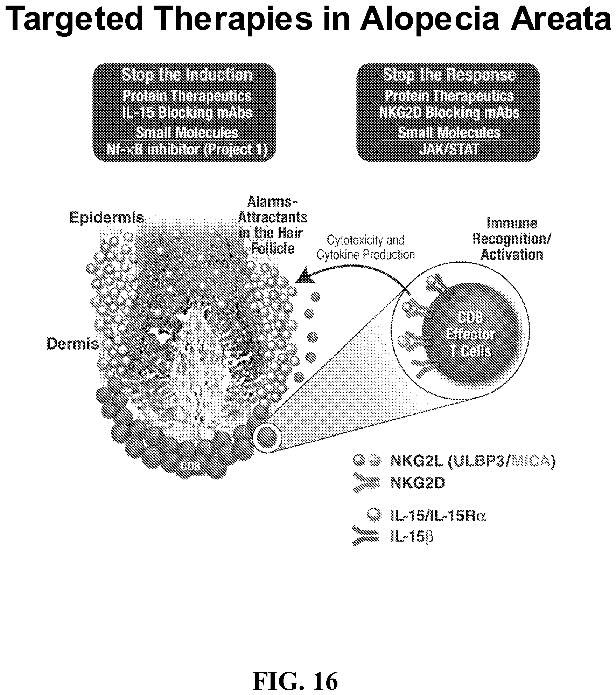

**FIG. 16** is a schematic that shows the pre-clinical strategies to interrupt AA pathogenesis are aimed at interruption of the induction and effector function of CD8 T cells and include blocking IFN-γ signals.
**FIG. 17** shows that CD8+ NKG2D+ T cells infiltrate AA hair follicles in both mouse and human. *Top panel:* Human Alopecia Areata (Pethukova et al.,2010, Nature, 466(7302):113-7); *bottom panel-* C3H/HeJ Alopecia Areata.
**FIG. 18** shows CD8+NKG2D+ T cells in alopecic C3H mice CD8 □ αβ ⁺NKG2D⁺ T cells are massively expanded in the skin, lymph node and blood of alopecic C3H mice. (NKG2D⁺ γδ T cells are present in both affected and unaffected skin).
**FIG. 19****.** shows CD8+NKG2D+ T cells in alopecic C3H mice. Immunophenotype of gated LN CD8α⁺NKG2D⁺ populations shows that they are uniformly CD8αβ⁺CD44⁺CXCR3⁺DX5⁺NKG2A/C/E+IFN-γ producing memory T cells.
**FIG. 20** shows spectratype analysis of C3H T cells in skin and LN: Similar TCR repertoires are shared by total skin and sorted CD8+NKG2D+ LN T cells identifying these as the relevant effectors.
**FIGS. 21A-B** show that IFN-γ producing CD8 T cells dominate human and mouse AA. **FIG. 21A****:** Human: T cells obtained from T cell crawl-outs from AA skin biopsies are uniformly CD8+NKG2D+ and produce IFN-γ upon PMA/Ionomycin stimulation. **FIG. 21B****:** Mouse: C3H/HeJ stimulated lymph node cells from alopecic mice contain 3 times more IFN-γ producing CD8 and CD4 T cells than unaffected mice.
**FIG. 22** show that Type I and II IFNs are upregulated in C3H mouse AA skin: fold changes in expression of interferon genes (normalized to 18S) between 3 affected and 3 non affected C3H/HeJ mice, utilizing an interferon signaling and response qPCR array (Stellarray Lonza Cat #00189608).
**FIG. 23** shows graphs that show elevated serum chemokines and cytokines in Human AA. Interferon-y and IFN-induced chemokines (e.g., IP-10/CXCL10) are elevated in the serum of human AA, in some cases correlating with disease severity, i.e.., patchy disease (AAP) vs. universalis (AU).
**FIG. 24** is a luminex analysis that shows elevated IL-13 in the serum of Human AA subjects, in some cases correlating with disease severity, i.e.., patchy disease (AAP) vs. universalis (AU).
**FIG. 25** shows flow cytometry data that were gathered on a LSRII using the following mAbs in one tube to stain the entire population of C3H dermal cells liberated from a 0.5 cm by 2 cm patch of alopecic skin after 30 minutes collagenase digestion: (MHC II, CD11b, CD11c, CD19, 120G8, CD3, CD4, CD8, DX5, NKG2D). γδNKG2D positive cells and CD8+DX5+NKG2D+ population are identified. These cells were also CD3+, with γδCD3 expression 100x higher than that seen in CD8+ NK+ T cells. >95% of the total CD3 positive cells were accounted.
**FIG. 26** is a graph that shows the evaluation of PBMCs from four AA subjects. NKG2D expression was increased in subsets of lymphocytes.
**FIG. 27** shows spectratyping in a C3H-HeJ mouse. RNA was Isolated from skin biopsies, and from sorted NKG2D- and NKG2D+ CD8+ T cells from cutaneous lymph nodes of C3H-HeJ mice. Spectratyping analysis shows CDR3 length skewing in several Vbeta families. Some of the skewed Vbeta families are shared between skin and NKG2D+ lymph node CD8+ T cell populations (e.g., Vbeta2, Vbeta5.2, Vbeta20) whereas additional Vbeta families are skewed in skin (reflecting the CD4+ T cell population).
**FIG. 28** is a schematic showing that human scalp skin procured from control or AA affected individuals can be used to establish primary cultures of individual cellular populations within the skin and hair follicle.

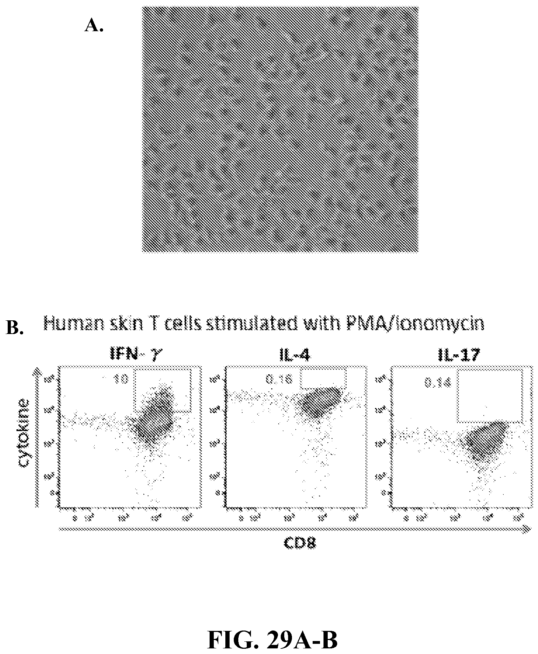

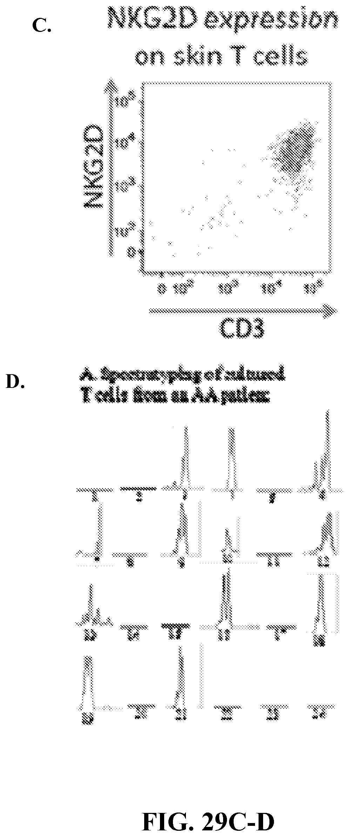

**FIG. 29** shows a T cell 'crawl-out' assay, in which cultured human dermis obtained from punch skin biopsies are used as source material for flow cytometric analysis. Trisected skin punch biopsies of a 6mm skin biopsy is obtained from alopecic lesional and non-lesional areas. After removing the fat, the finely minced skin is gently pressed in a Cellfoam matrix and then transferred, skin side-up, to a well of a 24-well plate containing 2 mls of Skin T medium with IL-2 (6ng/ml) and IL-15 (10 ng/ml). T cells crawl outs are shown in **FIG. 29A** after the matrix is removed 3 days later. Flow cytometry **(****FIGS. 29B-C****)** and spectratyping **(****FIG. 29D****)** of these cells are also shown.
**FIG. 30** shows that human peripheral blood mononuclear cells (PBMC), top panels or murine splenocytes were cultured in high dose IL-2 (1000/ml) to generate lymphokine-activated killer (LAK) cells. The CFSE green fluorescence labeled LAK cells were incubated with human hair follicle organ culture (top panels), or mouse hair follicle culture (bottom panels) from non-lesion region (bottom left two) or lesion.

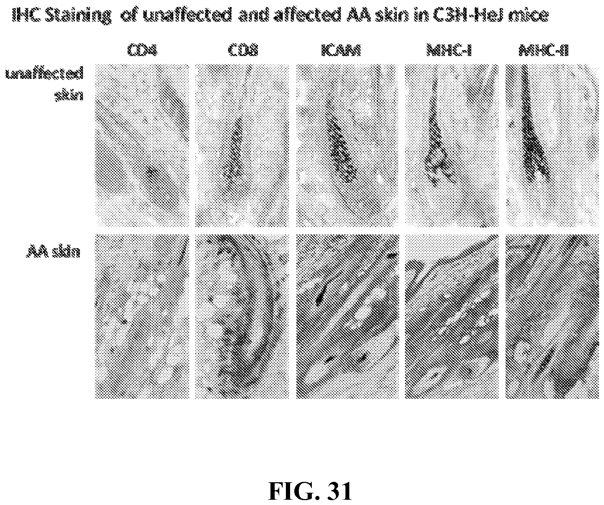

**FIG. 31** shows immunohistochemical (IHC) staining of unaffected and affected AA skin in C3H-HeJ mice.

### DETAILED DESCRIPTION OF THE INVENTION

The present disclosure provides for methods of treating a hair loss disorder (e.g., Alopecia Areata (AA), a common autoimmune form of hair loss) with an inhibitor of the Jak/Stat pathway, including Jak 1 and Jak 2, Stat 1 and Stat 2. Clinical research in AA has lagged behind its more heavily investigated "sister" autoimmune diseases in which this gene has been implicated (e.g., rheumatoid arthritis (RA), type 1 diabetes mellitus (T1D), multiple sclerosis (MS)). The disclosure relates to therapeutics previously untested in AA that can inform one about the clinical relevance of this pathway in AA and related diseases.

### Overview of the Integument and Hair Cells

The integument (or skin) is the largest organ of the body and is a highly complex organ covering the external surface of the body. It merges, at various body openings, with the mucous membranes of the alimentary and other canals. The integument performs a number of essential functions such as maintaining a constant internal environment via regulating body temperature and water loss; excretion by the sweat glands; but predominantly acts as a protective barrier against the action of physical, chemical and biologic agents on deeper tissues. Skin is elastic and except for a few areas such as the soles, palms, and ears, it is loosely attached to the underlying tissue. It also varies in thickness from 0.5 mm (0.02 inches) on the eyelids ("thin skin") to 4 mm (0.17 inches) or more on the palms and soles ("thick skin") (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

The skin is composed of two layers: a) the epidermis and b) the dermis. The epidermis is the outer layer, which is comparatively thin (0.1 mm). It is several cells thick and is composed of 5 layers: the stratum germinativum, stratum spinosum, stratum granulosum, stratum lucidum (which is limited to thick skin), and the stratum corneum. The outermost epidermal layer (the stratum corneum) consists of dead cells that are constantly shed from the surface and replaced from below by a single, basal layer of cells, called the stratum germinativum. The epidermis is composed predominantly of keratinocytes, which make up over 95% of the cell population. Keratinocytes of the basal layer (stratum germinativum) are constantly dividing, and daughter cells subsequently move upwards and outwards, where they undergo a period of differentiation, and are eventually sloughed off from the surface. The remaining cell population of the epidermis includes dendritic cells such as Langerhans cells and melanocytes. The epidermis is essentially cellular and non-vascular, containing little extracellular matrix except for the layer of collagen and other proteins beneath the basal layer of keratinocytes (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

The dermis is the inner layer of the skin and is composed of a network of collagenous extracellular material, blood vessels, nerves, and elastic fibers. Within the dermis are hair follicles with their associated sebaceous glands (collectively known as the pilosebaceous unit) and sweat glands. The interface between the epidermis and the dermis is extremely irregular and uneven, except in thin skin. Beneath the basal epidermal cells along the epidermal-dermal interface, the specialized extracellular matrix is organized into a distinct structure called the basement membrane (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

The mammalian hair fiber is composed of keratinized cells and develops from the hair follicle. The hair follicle is a peg of tissue derived from a downgrowth of the epidermis, which lies immediately underneath the skin's surface. The distal part of the hair follicle is in direct continuation with the external, cutaneous epidermis. Although a small structure, the hair follicle comprises a highly organized system of recognizably different layers arranged in concentric series. Active hair follicles extend down through the dermis, the hypodermis (which is a loose layer of connective tissue), and into the fat or adipose layer (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

At the base of an active hair follicle lies the hair bulb. The bulb consists of a body of dermal cells, known as the dermal papilla, contained in an inverted cup of epidermal cells known as the epidermal matrix. Irrespective of follicle type, the germinative epidermal cells at the very base of this epidermal matrix produce the hair fiber, together with several supportive epidermal layers. The lowermost dermal sheath is contiguous with the papilla basal stalk, from where the sheath curves externally around all of the hair matrix epidermal layers as a thin covering of tissue. The lowermost portion of the dermal sheath then continues as a sleeve or tube for the length of the follicle (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

Developing skin appendages, such as hair and feather follicles, rely on the interaction between the epidermis and the dermis, the two layers of the skin. In embryonic development, a sequential exchange of information between these two layers supports a complex series of morphogenetic processes, which results in the formation of adult follicle structures. However, in contrast to general skin dermal and epidermal cells, certain hair follicle cell populations, following maturity, retain their embryonic-type interactive, inductive, and biosynthetic behaviors. These properties can be derived from the very dynamic nature of the cyclical productive follicle, wherein repeated tissue remodeling necessitates a high level of dermal-epidermal interactive communication, which is vital for embryonic development and would be desirable in other forms of tissue reconstruction.

The hair fiber is produced at the base of an active follicle at a very rapid rate. For example, follicles produce hair fibers at a rate 0.4 mm per day in the human scalp and up to 1.5 mm per day in the rat vibrissa or whiskers, which means that cell proliferation in the follicle epidermis ranks amongst the fastest in adult tissues (Malkinson FD and JT Kearn, Int J Dermatol 1978, 17:536-551). Hair grows in cycles. The anagen phase is the growth phase, wherein up to 90% of the hair follicles said to be in anagen; catagen is the involuting or regressing phase which accounts for about 1-2% of the hair follicles; and telogen is the resting or quiescent phase of the cycle, which accounts for about 10-14% of the hair follicles. The cycle's length varies on different parts of the body.

Hair follicle formation and cycling is controlled by a balance of inhibitory and stimulatory signals. The signaling cues are potentiated by growth factors that are members of the TGFβ-BMP family. A prominent antagonist of the members of the TGFβ-BMP family is follistatin. Follistatin is a secreted protein that inhibits the action of various BMPs (such as BMP-2, -4, -7, and -11) and activins by binding to said proteins, and purportedly plays a role in the development of the hair follicle (Nakamura M, et al., FASEB J, 2003, 17(3):497-9; Patel K Intl J Biochem Cell Bio, 1998, 30:1087-93; Ueno N, et al., PNAS, 1987, 84:8282-86; Nakamura T, et al., Nature, 1990, 247:836-8; Iemura S, et al., PNAS, 1998, 77:649-52; Fainsod A, et al., Mech Dev, 1997, 63:39-50; Gamer LW, et al., Dev Biol, 1999, 208:222-32).

The deeply embedded end bulb, where local dermal-epidermal interactions drive active fiber growth, is the signaling center of the hair follicle comprising a cluster of mesencgymal cells, called the dermal papilla (DP). This same region is also central to the tissue remodeling and developmental changes involved in the hair fiber's or appendage's precise alternation between growth and regression phases. The DP, a key player in these activities, appears to orchestrate the complex program of differentiation that characterizes hair fiber formation from the primitive germinative epidermal cell source (Oliver RF, J Soc Cosmet Chem, 1971, 22:741-755; Oliver RF and CA Jahoda, Biology of Wool and Hair (eds Roger et al.), 1971, Cambridge University Press:51-67; Reynolds AJ and CA Jahoda, Development, 1992, 115:587-593; Reynolds AJ, et al., J Invest Dermatol, 1993, 101:634-38).

The lowermost dermal sheath (DS) arises below the basal stalk of the papilla, from where it curves outwards and upwards. This dermal sheath then externally encases the layers of the epidermal hair matrix as a thin layer of tissue and continues upward for the length of the follicle. The epidermally-derived outer root sheath (ORS) also continues for the length of the follicle, which lies immediately internal to the dermal sheath in between the two layers, and forms a specialized basement membrane termed the glassy membrane. The outer root sheath constitutes little more than an epidermal monolayer in the lower follicle, but becomes increasingly thickened as it approaches the surface. The inner root sheath (IRS) forms a mold for the developing hair shaft. It comprises three parts: the Henley layer, the Huxley layer, and the cuticle, with the cuticle being the innermost portion that touches the hair shaft. The IRS cuticle layer is a single cell thick and is located adjacent to the hair fiber. It closely interdigitates with the hair fiber cuticle layer. The Huxley layer can comprise up to four cell layers. The IRS Henley layer is the single cell layer that runs adjacent to the ORS layer (Ross MH, Histology: A text and atlas, 3rd edition, Williams and Wilkins, 1995: Chapter 14; Burkitt HG, et al, Wheater's Functional Histology, 3rd Edition, Churchill Livingstone, 1996: Chapter 9).

### Alopecia areata

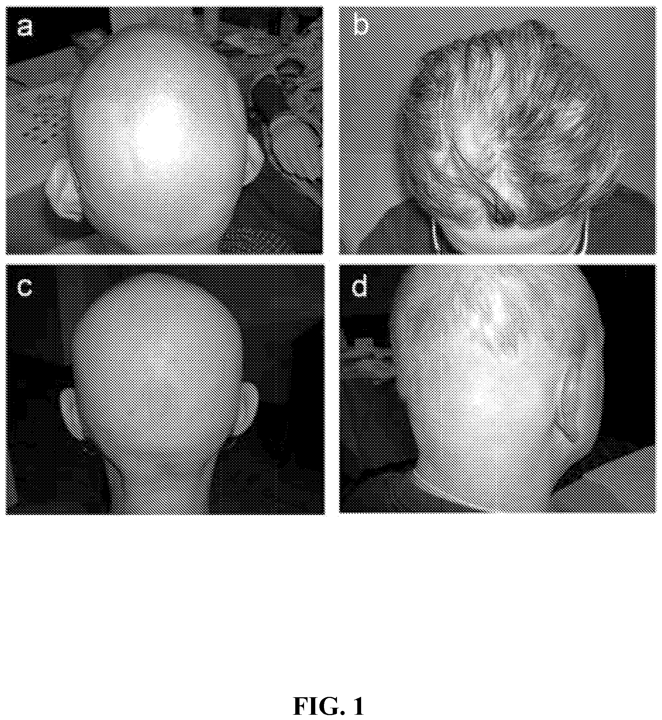

Alopecia areata (AA) is one of the most prevalent autoimmune diseases, affecting approximately 4.6 million people in the US alone, including males and females across all ethnic groups, with a lifetime risk of 1.7% (1) In AA, autoimmunity develops against the hair follicle, resulting in non-scarring hair loss that can begin as patches, which can coalesce and progress to cover the entire scalp (alopecia totalis, AT) or eventually the entire body (alopecia universalis, AU) (**FIG. 1**). AA was first described by Cornelius Celsus in 30 A.D., using the term "ophiasis", which means "snake", due to the sinuous path of hair loss as it spread slowly across the scalp. Hippocrates first used the Greek word 'alopekia' (fox mange), the modern day term "alopecia areata" was first used by Sauvages in his Nosologica Medica, published in 1760 in Lyons, France.

Curiously, AA preferentially affects pigmented hair follicles in the anagen (growth) phase of the hair cycle, and when the hair regrows in patches of AA, it frequently grows back white or colorless. The phenomenon of 'sudden whitening of the hair' is therefore ascribed to AA with an acute onset, and has been documented throughout history as having affected several prominent individuals at times of profound grief, stress or fear (2). Examples include Shahjahan, who upon the death of his wife in 1631 experienced acute whitening of his hair, and in his grief built the Taj Mahal in her honor. Sir Thomas More, author of *Utopia,* who on the eve of his execution in 1535 was said to have become 'white in both beard and hair'. The sudden whitening of the hair is believed to result from an acute attack upon the pigmented hair follicles, leaving behind the white hairs unscathed.

Several clinical aspects of AA remain unexplained but can hold important clues toward understanding pathogenesis. AA attacks hairs only around the base of the hair follicles, which are surrounded by dense clusters of lymphocytes, resulting in the pathognomic 'swarm of bees' appearance on histology. Based on these observations, it is postulated that a signal(s) in the pigmented, anagen hair follicle is emitted which invokes an acute or chronic immune response against the lower end of the hair follicle, leading to hair cycle perturbation, acute hair shedding, hair shaft anomalies and hair breakage. Despite these dramatic perturbations in the hair follicle, there is no permanent organ destruction and the possibility of hair regrowth remains if immune privilege can be restored.

Throughout history, AA has been considered at times to be a neurological disease brought on by stress or anxiety, or as a result of an infectious agent, or even hormonal dysfunction. The concept of a genetically-determined autoimmune mechanism as the basis for AA emerged during the 20^{th} century from multiple lines of evidence. AA hair follicles exhibit an immune infiltrate with activated Th, Tc and NK cells (3, 4) and there is a shift from a suppressive (Th2) to an autoimmune (Th1) cytokine response. The humanized model of AA, which involves transfer of AA patient scalp onto immune-deficient SCID mice illustrates the autoimmune nature of the disease, since transfer of donor T-cells causes hair loss only when co-cultured with hair follicle or human melanoma homogenate (5, 6). Regulatory T cells which serve to maintain immune tolerance are observed in lower numbers in AA tissue (7), and transfer of these cells to C3H/HeJ mice leads to resistance to AA (8). Although AA has long been considered exclusively as a T-cell mediated disease, in recent years, an additional mechanism of disease has been postulated. The hair follicle is defined as one of a select few immune privileged sites in the body, characterized by the presence of extracellular matrix barriers to impede immune cell trafficking, lack of antigen presenting cells, and inhibition of NK cell activity via the local production of immunosuppressive factors and reduced levels of MHC class I expression (9). Thus, the notion of a 'collapse of immune privilege' has also been invoked as part of the mechanism by which AA can arise. Support for a genetic basis for AA comes from multiple lines of evidence, including the observed heritability in first degree relatives (10, 11), twin studies (12), and most recently, from the results of our family-based linkage studies (13). Delamere F M et al., 2008 ("Interventions for alopecia areata.", THE COCHRANE DATABASE OF SYSTEMATIC REVIEWS 2008, (2008), no. 2, ISSN 1469-493X, page CD004413) discusses the effects of interventions used in the management of alopecia areata, alopecia totalis and alopecia universalis. Overall, none of the interventions showed significant treatment benefit in terms of hair growth when compared with placebo. Mahe et al., Androgenetic alopecia and microinflammation, International Journal of Dermatology (2000) 39:576-584A discloses a hypothesis postulating the role of "microinflammation" and inflammatory cytokines for triggering an inflammatory process ultimately resulting in androgenetic alopecia. Foitzik et al, American Journal of Pathology, Volume 168, Issue 3, March 2006, Pages 748-756 teaches that the JAK/STAT pathway plays some role in the progression of androgenetic alopecia.

### Treatment of Hair Loss Disorders

This disclosure relates to the discovery that a known therapeutic, for example an inhibitor of a protein tyrosine kinase (PTK) involved in cytokine signaling, such as JAK/STAT proteins Jak 1, Jak2, Stat 1 or Stat 2 (e.g., INCB 018424; Incyte), can be used for the the treatment of hair loss disorders. Non-limiting examples of hair loss disorders include: androgenetic alopecia, Alopecia areata, telogen effluvium, alopecia areata, alopecia totalis, and alopecia universalis.

An aspect of the invention encompasses a Jak1 and/or Jak2 inhibitor selected from the group consisting of: AG490 with the CAS No. 133550-30-8; CYT387 with the CAS No. 1056634-68-4; SB1518 with the CAS No. 937272-79-2; TG101348 with the CAS No. 936091-26-8; BMS-911543 with the CAS No. 1271022-90-2; WP-1034 with the CAS No. 857064- 42-7; CEP-701 with the CAS No. 111358-88-4; INCB 018424 with the CAS No. 941678-49-5); and LY3009104 with the CAS No. 1187594-09-7 for use in the treatment of a hair-loss disorder selected from the group consisting of alopecia areata and androgenetic alopecia in a mammalian subject in need thereof. In a further embodiment, the inhibitor is INCB 018424. In one embodiment, alopecia areata is alopecia totalis or alopecia universalis. The disclosure relates to a hair-loss disorder comprising telogen effluvium, telogen effluvium, tinea capitis, hypotrichosis or hereditary hypotrichosis simplex.

An aspect of the present disclosure provides for a method for inducing hair growth in a subject where the method comprises administering to the subject an effective amount of an inhibitor of a protein tyrosine kinase (PTK) involved in cytokine signaling. In one embodiment, the inhibitor is a Jak/Stat inhibitor. In other embodiments, the inhibitor is INCB 018424. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis.

The disclosure relates to a method of treating a hair-loss disorder in a mammalian subject in need thereof, the method comprising administering to the subject a Jak1 inhibitor. In one embodiment, the inhibitor is an antibody or antibody fragment that is directed to SEQ ID NO: 1. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis

The disclosure relates to a method for inducing hair growth in a subject where the method comprises administering to the subject an effective amount of a Jak1 inhibitor, thereby controlling hair growth in the subject. In one embodiment, the inhibitor comprises an antibody that specifically binds to a protein comprising SEQ ID NO: 1. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis.

The disclosure relates to a method of treating a hair-loss disorder in a mammalian subject in need thereof, the method comprising administering to the subject a Jak 2 inhibitor. In one embodiment, the inhibitor is an antibody or antibody fragment that is directed to SEQ ID NO: 3. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis

The disclosure relates to a method for inducing hair growth in a subject where the method comprises administering to the subject an effective amount of a Jak 2 inhibitor, thereby controlling hair growth in the subject. In one embodiment, the inhibitor comprises an antibody that specifically binds to a protein comprising SEQ ID NO: 3. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis.

The disclosure relates to a method of treating a hair-loss disorder in a mammalian subject in need thereof, the method comprising administering to the subject a Stat 1 inhibitor. In one embodiment, the inhibitor is an antibody or antibody fragment that is directed to SEQ ID NO: 5. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis

The disclosure relates to a method for inducing hair growth in a subject where the method comprises administering to the subject an effective amount of a Stat 1 inhibitor, thereby controlling hair growth in the subject. In one embodiment, the inhibitor comprises an antibody that specifically binds to a protein comprising SEQ ID NO: 5. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis.

The disclosure relates to a method of treating a hair-loss disorder in a mammalian subject in need thereof, the method comprising administering to the subject a Stat 2 inhibitor. In one embodiment, the inhibitor is an antibody or antibody fragment that is directed to SEQ ID NO: 7. In another embodiment, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis

The disclosure relates to a method for inducing hair growth in a subject where the method comprises administering to the subject an effective amount of a Stat 2 inhibitor, thereby controlling hair growth in the subject. In one embodiment, the inhibitor comprises an antibody that specifically binds to a protein comprising SEQ ID NO: 7. In some embodiments, the subject is afflicted with a hair-loss disorder. In other embodiments, the hair-loss disorder comprises androgenetic alopecia, telogen effluvium, alopecia areata, telogen effluvium, tinea capitis, alopecia totalis, hypotrichosis, hereditary hypotrichosis simplex, or alopecia universalis. In some embodiments, the modulating compound can also inhibit hair growth, thus it can be used for treatment of hair growth disorders, such as hypertrichosis.

### DNA and AminoAcid Manipulation Methods and Purification Thereof

The present invention utilizes conventional molecular biology, microbiology, and recombinant DNA techniques available to one of ordinary skill in the art. Such techniques are well known to the skilled worker and are explained fully in the literature. See, e.g., Maniatis, Fritsch & Sambrook, "Molecular Cloning: A Laboratory Manual" (1982): "DNA Cloning: A Practical Approach," Volumes I and II (D. N. Glover, ed., 1985); "Oligonucleotide Synthesis" (M. J. Gait, ed., 1984); "Nucleic Acid Hybridization" (B. D. Hames & S. J. Higgins, eds., 1985); "Transcription and Translation" (B. D. Hames & S. J. Higgins, eds., 1984); "Animal Cell Culture" (R. I. Freshney, ed., 1986); "Immobilized Cells and Enzymes" (IRL Press, 1986): B. Perbal, "A Practical Guide to Molecular Cloning" (1984), and Sambrook, et al., "Molecular Cloning: a Laboratory Manual" (1989).

One skilled in the art can obtain a protein in several ways, which include, but are not limited to, isolating the protein via biochemical means or expressing a nucleotide sequence encoding the protein of interest by genetic engineering methods.

A protein is encoded by a nucleic acid (including, for example, genomic DNA, complementary DNA (cDNA), synthetic DNA, as well as any form of corresponding RNA). For example, it can be encoded by a recombinant nucleic acid of a gene. The proteins can be obtained from various sources and can be produced according to various techniques known in the art. For example, a nucleic acid that encodes a protein can be obtained by screening DNA libraries, or by amplification from a natural source. A protein can be a fragment or portion thereof. The nucleic acids encoding aprotein can be produced via recombinant DNA technology and such recombinant nucleic acids can be prepared by conventional techniques, including chemical synthesis, genetic engineering, enzymatic techniques, or a combination thereof. For example, a Jak 1 protein is the polypeptide encoded by the nucleic acid having the nucleotide sequence shown in SEQ ID NO: 2. An example of a Jak 1 polypeptide has the amino acid sequence shown in SEQ ID NO: 1. A Jak 2 protein is the polypeptide encoded by the nucleic acid having the nucleotide sequence shown in SEQ ID NO: 4. An example of a Jak 2 polypeptide has the amino acid sequence shown in SEQ ID NO: 3. For example, a Stat 1 protein is the polypeptide encoded by the nucleic acid having the nucleotide sequence shown in SEQ ID NO: 6. An example of a Stat 1 polypeptide has the amino acid sequence shown in SEQ ID NO: 5. A Stat 2 protein is the polypeptide encoded by the nucleic acid having the nucleotide sequence shown in SEQ ID NO: 8. An example of a Stat 2 polypeptide has the amino acid sequence shown in SEQ ID NO: 7.

The polypeptide sequence of human Jak 1 is depicted in SEQ ID NO: 1. The nucleotide sequence of human Jak 1 is shown in SEQ ID NO: 2. Sequence information related to Jak 1 is accessible in public databases by GenBank Accession numbers NP_002218 (for protein) and NM_002227.2 (for nucleic acid).

SEQ ID NO: 1 is the human wild type amino acid sequence corresponding to Jak1 (residues 1 - 1154):

1 
61 
121 
181 
241 
301 
361 
421 
481 
541 
601 
661 
721 
781 
841 
901 
961 
1021 

1081 
1141 

SEQ ID NO: 2 is the human wild type nucleotide sequence corresponding to Jak1 (nucleotides 1-5053), wherein the underscored bolded "ATG" denotes the beginning of the open reading frame:

1 tgcagacagt gcgggcctgc gcccagtccc ggctgtcctc gccgcgaccc ctcctcagcc
61 ctgggcgcgc gcacgctggg gccccgcggg gctggccgcc tagcgagcct gccggtcgac
121 cccagccagc gcagcgacgg ggcgctgcct ggcccaggcg cacacggaag tgcgcttctc
181 tgaagtagct ttggaaagta gagaagaaaa tccagtttgc ttcttggaga acactggaca
241 gctgaataaa tgcagtatct aaatataaaa gaggactgca atgccatggc tttctgtgct
301 aaaatgagga gctccaagaa gactgaggtg aacctggagg cccctgagcc aggggtggaa
361 gtgatcttct atctgtcgga cagggagccc ctccggctgg gcagtggaga gtacacagca
421 gaggaactgt gcatcagggc tgcacaggca tgccgtatct ctcctctttg tcacaacctc
481 tttgccctgt atgacgagaa caccaagctc tggtatgctc caaatcgcac catcaccgtt
541 gatgacaaga tgtccctccg gctccactac cggatgaggt tctatttcac caattggcat
601 ggaaccaacg acaatgagca gtcagtgtgg cgtcattctc caaagaagca gaaaaatggc
661 tacgagaaaa aaaagattcc agatgcaacc cctctccttg atgccagctc actggagtat
721 ctgtttgctc agggacagta tgatttggtg aaatgcctgg ctcctattcg agaccccaag
781 accgagcagg atggacatga tattgagaac gagtgtctag ggatggctgt cctggccatc
841 tcacactatg ccatgatgaa gaagatgcag ttgccagaac tgcccaagga catcagctac
901 aagcgatata ttccagaaac attgaataag tccatcagac agaggaacct tctcaccagg
961 atgcggataa ataatgtttt caaggatttc ctaaaggaat ttaacaacaa gaccatttgt
1021 gacagcagcg tgtccacgca tgacctgaag gtgaaatact tggctacctt ggaaactttg
1081 acaaaacatt acggtgctga aatatttgag acttccatgt tactgatttc atcagaaaat
1141 gagatgaatt ggtttcattc gaatgacggt ggaaacgttc tctactacga agtgatggtg
1201 actgggaatc ttggaatcca gtggaggcat aaaccaaatg ttgtttctgt tgaaaaggaa
1261 aaaaataaac tgaagcggaa aaaactggaa aataaacaca agaaggatga ggagaaaaac
1321 aagatccggg aagagtggaa caatttttct tacttccctg aaatcactca cattgtaata
1381 aaggagtctg tggtcagcat taacaagcag gacaacaaga aaatggaact gaagctctct
1441 tcccacgagg aggccttgtc ctttgtgtcc ctggtagatg gctacttccg gctcacagca
1501 gatgcccatc attacctctg caccgacgtg gcccccccgt tgatcgtcca caacatacag
1561 aatggctgtc atggtccaat ctgtacagaa tacgccatca ataaattgcg gcaagaagga
1621 agcgaggagg ggatgtacgt gctgaggtgg agctgcaccg actttgacaa catcctcatg
1681 accgtcacct gctttgagaa gtctgagcag gtgcagggtg cccagaagca gttcaagaac
1741 tttcagatcg aggtgcagaa gggccgctac agtctgcacg gttcggaccg cagcttcccc
1801 agcttgggag acctcatgag ccacctcaag aagcagatcc tgcgcacgga taacatcagc
1861 ttcatgctaa aacgctgctg ccagcccaag ccccgagaaa tctccaacct gctggtggct
1921 actaagaaag cccaggagtg gcagcccgtc taccccatga gccagctgag tttcgatcgg
1981 atcctcaaga aggatctggt gcagggcgag caccttggga gaggcacgag aacacacatc
2041 tattctggga ccctgatgga ttacaaggat gacgaaggaa cttctgaaga gaagaagata
2101 aaagtgatcc tcaaagtctt agaccccagc cacagggata tttccctggc cttcttcgag
2161 gcagccagca tgatgagaca ggtctcccac aaacacatcg tgtacctcta tggcgtctgt
2221 gtccgcgacg tggagaatat catggtggaa gagtttgtgg aagggggtcc tctggatctc
2281 ttcatgcacc ggaaaagcga tgtccttacc acaccatgga aattcaaagt tgccaaacag
2341 ctggccagtg ccctgagcta cttggaggat aaagacctgg tccatggaaa tgtgtgtact
2401 aaaaacctcc tcctggcccg tgagggcatc gacagtgagt gtggcccatt catcaagctc
2461 agtgaccccg gcatccccat tacggtgctg tctaggcaag aatgcattga acgaatccca
2521 tggattgctc ctgagtgtgt tgaggactcc aagaacctga gtgtggctgc tgacaagtgg
2581 agctttggaa ccacgctctg ggaaatctgc tacaatggcg agatcccctt gaaagacaag
2641 acgctgattg agaaagagag attctatgaa agccggtgca ggccagtgac accatcatgt
2701 aaggagctgg ctgacctcat gacccgctgc atgaactatg accccaatca gaggcctttc
2761 ttccgagcca tcatgagaga cattaataag cttgaagagc agaatccaga tattgtttca
2821 gaaaaaaaac cagcaactga agtggacccc acacattttg aaaagcgctt cctaaagagg
2881 atccgtgact tgggagaggg ccactttggg aaggttgagc tctgcaggta tgaccccgaa
2941 ggggacaata caggggagca ggtggctgtt aaatctctga agcctgagag tggaggtaac

3001 cacatagctg atctgaaaaa ggaaatcgag atcttaagga acctctatca tgagaacatt
3061 gtgaagtaca aaggaatctg cacagaagac ggaggaaatg gtattaagct catcatggaa
3121 tttctgcctt cgggaagcct taaggaatat cttccaaaga ataagaacaa aataaacctc
3181 aaacagcagc taaaatatgc cgttcagatt tgtaagggga tggactattt gggttctcgg
3241 caatacgttc accgggactt ggcagcaaga aatgtccttg ttgagagtga acaccaagtg
3301 aaaattggag acttcggttt aaccaaagca attgaaaccg ataaggagta ttacaccgtc
3361 aaggatgacc gggacagccc tgtgttttgg tatgctccag aatgtttaat gcaatctaaa
3421 ttttatattg cctctgacgt ctggtctttt ggagtcactc tgcatgagct gctgacttac
3481 tgtgattcag attctagtcc catggctttg ttcctgaaaa tgataggccc aacccatggc
3541 cagatgacag tcacaagact tgtgaatacg ttaaaagaag gaaaacgcct gccgtgccca
3601 cctaactgtc cagatgaggt ttatcaactt atgaggaaat gctgggaatt ccaaccatcc
3661 aatcggacaa gctttcagaa ccttattgaa ggatttgaag cacttttaaa ataagaagca
3721 tgaataacat ttaaattcca cagattatca agtccttctc ctgcaacaaa tgcccaagtc
3781 attttttaaa aatttctaat gaaagaagtt tgtgttctgt ccaaaaagtc actgaactca
3841 tacttcagta catatacatg tataaggcac actgtagtgc ttaatatgtg taaggacttc
3901 ctctttaaat ttggtaccag taacttagtg acacataatg acaaccaaaa tatttgaaag
3961 cacttaagca ctcctccttg tggaaagaat ataccaccat ttcatctggc tagttcacca
4021 tcacaactgc attaccaaaa ggggattttt gaaaacgagg agttgaccaa aataatatct
4081 gaagatgatt gcttttccct gctgccagct gatctgaaat gttttgctgg cacattaatc
4141 atagataaag aaagattgat ggacttagcc ctcaaatttc agtatctata cagtactaga
4201 ccatgcattc ttaaaatatt agataccagg tagtatatat tgtttctgta caaaaatgac
4261 tgtattctct caccagtagg acttaaactt tgtttctcca gtggcttagc tcctgttcct
4321 ttgggtgatc actagcaccc atttttgaga aagctggttc tacatggggg gatagctgtg
4381 gaatagataa tttgctgcat gttaattctc aagaactaag cctgtgccag tgctttccta
4441 agcagtatac ctttaatcag aactcattcc cagaacctgg atgctattac acatgctttt
4501 aagaaacgtc aatgtatatc cttttataac tctaccactt tggggcaagc tattccagca
4561 ctggttttga atgctgtatg caaccagtct gaataccaca tacgctgcac tgttcttaga
4621 gggtttccat acttaccacc gatctacaag ggttgatccc tgtttttacc atcaatcatc
4681 accctgtggt gcaacacttg aaagacccgg ctagaggcac tatggacttc aggatccact
4741 agacagtttt cagtttgctt ggaggtagct gggtaatcaa aaatgtttag tcattgattc
4801 aatgtgaacg attacggtct ttatgaccaa gagtctgaaa atctttttgt tatgctgttt
4861 agtattcgtt tgatattgtt acttttcacc tgttgagccc aaattcagga ttggttcagt
4921 ggcagcaatg aagttgccat ttaaatttgt tcatagccta catcaccaag gtctctgtgt
4981 caaacctgtg gccactctat atgcactttg tttactcttt atacaaataa atatactaaa
5041 gactttacat gca

The polypeptide sequence of human Jak 2 is depicted in SEQ ID NO: 3. The nucleotide sequence of human Jak 2 is shown in SEQ ID NO: 4. Sequence information related to Jak 2 is accessible in public databases by GenBank Accession numbers NP_004963 (for protein) and NM_004972.3 (for nucleic acid).

SEQ ID NO: 3 is the human wild type amino acid sequence corresponding to Jak2 (residues 1 - 1132):

1 
61 
121 
181 
241 
301 
361 
421 
481 
541 

601 
661 
721 
781 
841 
901 
961 
1021 
1081 

SEQ ID NO: 4 is the human wild type nucleotide sequence corresponding to Jak2 (nucleotides 1-5285), wherein the underscored bolded "ATG" denotes the beginning of the open reading frame:

1 ctgcaggaag gagagaggaa gaggagcaga agggggcagc agcggacgcc gctaacggcc
61 tccctcggcg ctgacaggct gggccggcgc ccggctcgct tgggtgttcg cgtcgccact
121 tcggcttctc ggccggtcgg gcccctcggc ccgggcttgc ggcgcgcgtc ggggctgagg
181 gctgctgcgg cgcagggaga ggcctggtcc tcgctgccga gggatgtgag tgggagctga
241 gcccacactg gagggccccc gagggcccag cctggaggtc gttcagagcc gtgcccgtcc
301 cggggcttcg cagaccttga cccgccgggt aggagccgcc cctgcgggct cgagggcgcg
361 ctctggtcgc ccgatctgtg tagccggttt cagaagcagg caacaggaac aagatgtgaa
421 ctgtttctct tctgcagaaa aagaggctct tcctcctcct cccgcgacgg caaatgttct
481 gaaaaagact ctgcatggga atggcctgcc ttacgatgac agaaatggag ggaacatcca
541 cctcttctat atatcagaat ggtgatattt ctggaaatgc caattctatg aagcaaatag
601 atccagttct tcaggtgtat ctttaccatt cccttgggaa atctgaggca gattatctga
661 cctttccatc tggggagtat gttgcagaag aaatctgtat tgctgcttct aaagcttgtg
721 gtatcacacc tgtgtatcat aatatgtttg ctttaatgag tgaaacagaa aggatctggt
781 atccacccaa ccatgtcttc catatagatg agtcaaccag gcataatgta ctctacagaa
841 taagatttta ctttcctcgt tggtattgca gtggcagcaa cagagcctat cggcatggaa
901 tatctcgagg tgctgaagct cctcttcttg atgactttgt catgtcttac ctctttgctc
961 agtggcggca tgattttgtg cacggatgga taaaagtacc tgtgactcat gaaacacagg
1021 aagaatgtct tgggatggca gtgttagata tgatgagaat agccaaagaa aacgatcaaa
1081 ccccactggc catctataac tctatcagct acaagacatt cttaccaaaa tgtattcgag
1141 caaagatcca agactatcat attttgacaa ggaagcgaat aaggtacaga tttcgcagat
1201 ttattcagca attcagccaa tgcaaagcca ctgccagaaa cttgaaactt aagtatctta
1261 taaatctgga aactctgcag tctgccttct acacagagaa atttgaagta aaagaacctg
1321 gaagtggtcc ttcaggtgag gagatttttg caaccattat aataactgga aacggtggaa
1381 ttcagtggtc aagagggaaa cataaagaaa gtgagacact gacagaacag gatttacagt
1441 tatattgcga ttttcctaat attattgatg tcagtattaa gcaagcaaac caagagggtt
1501 caaatgaaag ccgagttgta actatccata agcaagatgg taaaaatctg gaaattgaac
1561 ttagctcatt aagggaagct ttgtctttcg tgtcattaat tgatggatat tatagattaa
1621 ctgcagatgc acatcattac ctctgtaaag aagtagcacc tccagccgtg cttgaaaata
1681 tacaaagcaa ctgtcatggc ccaatttcga tggattttgc cattagtaaa ctgaagaaag
1741 caggtaatca gactggactg tatgtacttc gatgcagtcc taaggacttt aataaatatt
1801 ttttgacttt tgctgtcgag cgagaaaatg tcattgaata taaacactgt ttgattacaa
1861 aaaatgagaa tgaagagtac aacctcagtg ggacaaagaa gaacttcagc agtcttaaag
1921 atcttttgaa ttgttaccag atggaaactg ttcgctcaga caatataatt ttccagttta
1981 ctaaatgctg tcccccaaag ccaaaagata aatcaaacct tctagtcttc agaacgaatg
2041 gtgtttctga tgtaccaacc tcaccaacat tacagaggcc tactcatatg aaccaaatgg
2101 tgtttcacaa aatcagaaat gaagatttga tatttaatga aagccttggc caaggcactt
2161 ttacaaagat ttttaaaggc gtacgaagag aagtaggaga ctacggtcaa ctgcatgaaa
2221 cagaagttct tttaaaagtt ctggataaag cacacagaaa ctattcagag tctttctttg
2281 aagcagcaag tatgatgagc aagctttctc acaagcattt ggttttaaat tatggagtat
2341 gtgtctgtgg agacgagaat attctggttc aggagtttgt aaaatttgga tcactagata
2401 catatctgaa aaagaataaa aattgtataa atatattatg gaaacttgaa gttgctaaac
2461 agttggcatg ggccatgcat tttctagaag aaaacaccct tattcatggg aatgtatgtg
2521 ccaaaaatat tctgcttatc agagaagaag acaggaagac aggaaatcct cctttcatca

2581 aacttagtga tcctggcatt agtattacag ttttgccaaa ggacattctt caggagagaa
2641 taccatgggt accacctgaa tgcattgaaa atcctaaaaa tttaaatttg gcaacagaca
2701 aatggagttt tggtaccact ttgtgggaaa tctgcagtgg aggagataaa cctctaagtg
2761 ctctggattc tcaaagaaag ctacaatttt atgaagatag gcatcagctt cctgcaccaa
2821 agtgggcaga attagcaaac cttataaata attgtatgga ttatgaacca gatttcaggc
2881 cttctttcag agccatcata cgagatctta acagtttgtt tactccagat tatgaactat
2941 taacagaaaa tgacatgtta ccaaatatga ggataggtgc cctggggttt tctggtgcct
3001 ttgaagaccg ggatcctaca cagtttgaag agagacattt gaaatttcta cagcaacttg
3061 gcaagggtaa ttttgggagt gtggagatgt gccggtatga ccctctacag gacaacactg
3121 gggaggtggt cgctgtaaaa aagcttcagc atagtactga agagcaccta agagactttg
3181 aaagggaaat tgaaatcctg aaatccctac agcatgacaa cattgtaaag tacaagggag
3241 tgtgctacag tgctggtcgg cgtaatctaa aattaattat ggaatattta ccatatggaa
3301 gtttacgaga ctatcttcaa aaacataaag aacggataga tcacataaaa cttctgcagt
3361 acacatctca gatatgcaag ggtatggagt atcttggtac aaaaaggtat atccacaggg
3421 atctggcaac gagaaatata ttggtggaga acgagaacag agttaaaatt ggagatttg
3481 ggttaaccaa agtcttgcca caagacaaag aatactataa agtaaaagaa cctggtgaaa
3541 gtcccatatt ctggtatgct ccagaatcac tgacagagag caagttttct gtggcctcag
3601 atgtttggag ctttggagtg gttctgtatg aacttttcac atacattgag aagagtaaaa
3661 gtccaccagc ggaatttatg cgtatgattg gcaatgacaa acaaggacag atgatcgtgt
3721 tccatttgat agaacttttg aagaataatg gaagattacc aagaccagat ggatgcccag
3781 atgagatcta tatgatcatg acagaatgct ggaacaataa tgtaaatcaa cgcccctcct
3841 ttagggatct agctcttcga gtggatcaaa taagggataa catggctgga tgaaagaaat
3901 gaccttcatt ctgagaccaa agtagattta cagaacaaag ttttatattt cacattgctg
3961 tggactatta ttacatatat cattattata taaatcatga tgctagccag caaagatgtg
4021 aaaatatctg ctcaaaactt tcaaagttta gtaagttttt cttcatgagg ccaccagtaa
4081 aagacattaa tgagaattcc ttagcaagga ttttgtaaga agtttcttaa acattgtcag
4141 ttaacatcac tcttgtctgg caaaagaaaa aaaatagact ttttcaactc agctttttga
4201 gacctgaaaa aattattatg taaattttgc aatgttaaag atgcacagaa tatgtatgta
4261 tagtttttac cacagtggat gtataatacc ttggcatctt gtgtgatgtt ttacacacat
4321 gagggctggt gttcattaat actgttttct aatttttcca tagttaatct ataattaatt
4381 acttcactat acaaacaaat taagatgttc agataattga ataagtacct ttgtgtcctt
4441 gttcatttat atcgctggcc agcattataa gcaggtgtat acttttagct tgtagttcca
4501 tgtactgtaa atatttttca cataaaggga acaaatgtct agttttattt gtataggaaa
4561 tttccctgac cctaaataat acattttgaa atgaaacaag cttacaaaga tataatctat
4621 tttattatgg tttcccttgt atctatttgt ggtgaatgtg ttttttaaat ggaactatct
4681 ccaaattttt ctaagactac tatgaacagt tttcttttaa aattttgaga ttaagaatgc
4741 caggaatatt gtcatccttt gagctgctga ctgccaataa cattcttcga tctctgggat
4801 ttatgctcat gaactaaatt taagcttaag ccataaaata gattagattg ttttttaaaa
4861 atggatagct cattaagaag tgcagcaggt taagaatttt ttcctaaaga ctgtatattt
4921 gaggggtttc agaattttgc attgcagtca tagaagagat ttatttcctt tttagagggg
4981 aaatgaggta aataagtaaa aaagtatgct tgttaatttt attcaagaat gccagtagaa
5041 aattcataac gtgtatcttt aagaaaaatg agcatacatc ttaaatcttt tcaattaagt
5101 ataaggggtt gttcgttgtt gtcatttgtt atagtgctac tccactttag acaccatagc
5161 taaaataaaa tatggtgggt tttgtgtgtg tgtgtgtgtg tgtgtgtgtg tgtgtgtgtg
5221 tgttatttat acaaaactta aaatacttgc tgttttgatt aaaaagaaaa tagtttctta
5281 cttta

The polypeptide sequence of human Stat 1 is depicted in SEQ ID NO: 5. The nucleotide sequence of human Stat 1 is shown in SEQ ID NO: 6. Sequence information related to Stat 1 is accessible in public databases by GenBank Accession numbers ADA59516 (for protein) and GU211347.1 (for nucleic acid).

SEQ ID NO: 5 is the human wild type amino acid sequence corresponding to Stat1 (residues 1 - 750):

1 
61 
121 
181 
241 
301 
361 
421 
481 
541 
601 
661 
721 

SEQ ID NO: 6 is the human wild type nucleotide sequence corresponding to Stat1 (nucleotides 1-2353), wherein the underscored bolded "ATG" denotes the beginning of the open reading frame:

1 gtgctgtgcg tagctgctcc tttggttgaa tccccaggcc cttgttgggg cacaaggtgg
61 caggatgtct cagtggtacg aacttcagca gcttgactca aaattcctgg agcaggttca
121 ccagctttat gatgacagtt ttcccatgga aatcagacag tacctggcac agtggttaga
181 aaagcaagac tgggagcaca ctgccaatga tgtttcattt gccaccatcc gttttcatga
241 cctcctgtca cagctggatg atcaatatag tcgcttttct ttggagaata acttcttgct
301 acagcataac ataaggaaaa gcaagcgtaa tcttcaggat aattttcagg aagacccaat
361 ccagatgtct atgatcattt acagctgtct gaaggaagaa aggaaaattc tggaaaacgc
421 ccagagattt aatcaggctc agtcggggaa tattcagagc acagtgatgt tagacaaaca
481 gaaagagctt gacagtaaag tcagaaatgt gaaggacaag gttatgtgta tagagcatga
541 aatcaagagc ctggaagatt tacaagatga atatgacttc aaatgcaaaa ccttgcagaa
601 cagagaacac gagaccaatg gtgtggcaaa gagtgatcag aaacaagaac agctgttact
661 caagaagatg tatttaatgc ttgacaataa gagaaaggaa gtagttcaca aaataataga
721 gttgctgaat gtcactgaac ttacccagaa tgccctgatt aatgatgaac tagtggagtg
781 gaagcggaga cagcagagcg cctgtattgg ggggccgccc aatgcttgct tggatcagct
841 gcagaactgg ttcactatag ttgcggagag tctgcagcaa gttcggcagc agcttaaaaa
901 gttggaggaa ttggaacaga aatacaccta cgaacatgac cctatcacaa aaaacaaaca
961 agtgttatgg gaccgcacct tcagtctttt ccagcagctc attcagagct cgtttgtggt
1021 ggaaagacag ccctgcatgc caacgcaccc tcagaggccg ctggtcttga agacaggggt
1081 ccagttcact gtgaagttga gactgttggt gaaattgcaa gagctgaatt ataatttgaa
1141 agtcaaagtc ttatttgata aagatgtgaa tgagagaaat acagtaaaag gatttaggaa
1201 gttcaacatt ttgggcacgc acacaaaagt gatgaacatg gaggagtcca ccaatggcag
1261 tctggcggct gaatttcggc acctgcaatt gaaagaacag aaaaatgctg gcaccagaac
1321 gaatgagggt cctctcatcg ttactgaaga gcttcactcc cttagttttg aaacccaatt
1381 gtgccagcct ggtttggtaa ttgacctcga gacgacctct ctgcccgttg tggtgatctc
1441 caacgtcagc cagctcccga gcggttgggc ctccatcctt tggtacaaca tgctggtggc
1501 ggaacccagg aatctgtcct tcttcctgac tccaccatgt gcacgatggg ctcagctttc
1561 agaagtgctg agttggcagt tttcttctgt caccaaaaga ggtctcaatg tggaccagct
1621 gaacatgttg ggagagaagc ttcttggtcc taacgccagc cccgatggtc tcattccgtg
1681 gacgaggttt tgtaaggaaa atataaatga taaaaatttt cccttctggc tttggattga
1741 aagcatccta gaactcatta aaaaacacct gctccctctc tggaatgatg ggtgcatcat
1801 gggcttcatc agcaaggagc gagagcgtgc cctgttgaag gaccagcagc cggggacctt
1861 cctgctgcgg ttcagtgaga gctcccggga aggggccatc acattcacat gggtggagcg
1921 gtcccagaac ggaggcgaac ctgacttcca tgcggttgaa ccctacacga agaaagaact
1981 ttctgctgtt actttccctg acatcattcg caattacaaa gtcatggctg ctgagaatat
2041 tcctgagaat cccctgaagt atctgtatcc aaatattgac aaagaccatg cctttggaaa
2101 gtattactcc aggccaaagg aagcaccaga gccaatggaa cttgatggcc ctaaaggaac
2161 tggatatatc aagactgagt tgatttctgt gtctgaagtt cacccttcta gacttcagac
2221 cacagacaac ctgctcccca tgtctcctga ggagtttgac gaggtgtctc ggatagtggg
2281 ctctgtagaa ttcgacagta tgatgaacac agtatagagc atgaattttt ttcatcttct
2341 ctggcgacag ttt

The polypeptide sequence of human Stat 2 is depicted in SEQ ID NO: 7. The nucleotide sequence of human Stat 2 is shown in SEQ ID NO: 8. Sequence information related to Stat 2 is accessible in public databases by GenBank Accession numbers AAA98760 (for protein) and U18671.1 (for nucleic acid).

SEQ ID NO: 7 is the human wild type amino acid sequence corresponding to Stat2 (residues 1 - 851):

1 
61 
121 
181 
241 
301 
361 
421 
481 
541 
601 
661 
721 
781 
841 

SEQ ID NO: 8 is the human wild type nucleotide sequence corresponding to Stat2 (nucleotides 1-18648), wherein the underscored bolded "ATG" denotes the beginning of the open reading frame:

1 tcaagatcag cctgggcaac atggcgaaac cccgtctcta caataaatac aaaaaaatta
61 tcctggcgga gttatgcacg ttgtagtccc aactacctgg gaggctgagg cgggagaatc
121 acctgagcct gggaggtcga ggctgcagcg agccgagatc ggccgctgca ttccagcctg
181 ggtgacagag cgagaccatg tctcaaaaaa taaaaattaa aaaaaaattg ttttcattac
241 ctcagccctc ctcttcctat cccaaggcgt cgaaattccg gtcccacccc ttcccatgga
301 gcccttggcg tctccaggct cctcaagcta gtttcggttc cgggctcacg cgcgggttct
361 cgaaaatcag ctgtttcagt cttgggctag tccactaatt ggactcctcc cctcgtagaa
421 agtgcctact tgaacttctc caccaatcgc tgaagctgca ggtgtggttt cggctcagct
481 tgtcccgccc tggcggaggg gcggagttgc ggcggcgcca gtgagctcgc agtctgggaa
541 gggcttgact gaatggcagc cagtgtcggg gtggcggctg ggaatggggg ccgctccgga
601 cttccgctgc caactacaag ggggcgggtc cgaggggggt tagccgaagt tgtaggcggg
661 gcgcgaggtt ctagtacccg agctcatact agggacggga agtcgcgacc agagccattg
721 gagggcgcgg ggactgcaac cctaatcagg tacgggccct gagagggtgt gctggggtag
781 gggtgggggt gagagtgaga gttcctccga gggaagggcg actggcccag gggttacccc
841 ctggagaggg tagcttcctt ccccagattg aaataggagc tgtcgcctgc tcggtcctcg
901 atcttcttct gtccagccta tctccctaac cctaatgccc ctctcccaaa actgccctgc
961 agcttccgag acccggaatc tggcattgtt atgttggttc ggtatctgac gtttttccct
1021 ctgctctgca ttatttttta tcttcaccaa aaaacgatgt tcaaagatag ataaatctaa
1081 aaacaaagat agataaatct attacccttg tttcgtaaaa agtataagct actgaaagat
1141 gaaacgattg cctaaggtca cacacaaaat tcagttcatt tcagaaaagc ttcttgagtg
1201 caaaatatgt gcctaagaat gagagataat gagaaaaaat tgtttcagcc ccttaacctc

1261 agtgtttgca atccatttgg ggagaccagg tttttgtttt ttgttttcat atttgaatct
1321 ttgctgactt gctcctttaa tatcagacac ttaaatcctc agatgggact catcatattt
1381 tttttgagat ggaatcttca ctatgttgct caagcttggt ctgcaactcc tggctcaagc
1441 catcctctcg tcttgttggg cctctcgtct tgtgggcctg cacaaagtgc tgggattaca
1501 ggcatgagcc attcatgccc tgggcgcacc ttggattgcg atgtgtgtgt gttgtgaagc
1561 tttttttttt ggtatcataa aagcaataca gatacatagt tttaaaaatc aagcagctac
1621 taaaagagtt aaaatgaaaa tagcccctcc caatccctcc cttgttcctg ctggaggtag
1681 aaaggcagct gatgttattc atgttagtag aagactctcc caccccaagc atttctcttt
1741 attttgtaat aaaatcatgt gaccttttta gaccacaaat atgcatgaat tctgttctgt
1801 taggctcagg ctgcaacaag ataagtttca gtttcctaaa tagacaccag ctggcagtga
1861 gcagggaaca gtggggagaa agatgcatgg gacagcctgc ttggtgacag gcaaaaaccg
1921 gtttgttgtt cttttagaga cagagtcttg ctttgtcacc caggctggag tgtagtgatg
1981 tgatctctgc ttactgcaac cctgcctctg ggtacaagcc attctcctgc ctcagcctct
2041 tgagtagctg ggattacagg caacaatttt aagtgaagtg aagtttcagg atctcgagca
2101 aagttgtata acctataatc atattcaaga ttcacaggtc ataaacgtgt catattcttg
2161 ggattgagcg acccattgca cagcatttag atgtgcttct agaatggagc tcctccttcc
2221 tatatggagg gcagtttata tggtgtactt acctgaccac caaaaagatt tggctctaaa
2281 aaagcttcag gtggccgggc atggtggttc acccctgtaa tccagcactt tgggaggcag
2341 gtgggcagat cacctgaggt cagaagttca gacagctgga catatggtga aacctcatct
2401 ctactaaaaa tacaaaaatt agactgggca tggtagtggg cgcctgtaat cccagctagt
2461 cgggaggctg aggcaggaga atcccttcaa ctcggacggc agagtttgca gtgaggccga
2521 gatcgtgtca ctgcagtcca gcctgggtga cagagcaaga ctccatctca aaaaaagtaa
2581 aaaaaaaaaa aagaaaaaaa aaagcttcag agccagcagg gatcatgctg taataaatac
2641 ttaacatcaa cactgatctt taaatgcttt agcacaatca aatataaata acaaacacac
2701 acataaatgc aaaataaatg aattagggag atagatgaaa taagattgtg gaaatagtaa
2761 tgtttgttaa agctggatgg tgatccttgt actattcact ctactctagt gtgtatttga
2821 aaattaccat taggctggtt atggtggctc atgcctgtta atcccggcat tttggaaggc
2881 tgaggcaggc ggattacttg agctcaggag tttagagtct gcctgggcaa catggcaaaa
2941 tcccatctct acaaaaaatt agctggcatg atggcacact cctgtagtcc cagctccttg
3001 aggggctgag gcagagaatg gcttgaacct gagaggctaa agctgcagtg agccaagatc
3061 atgccactgc actccagcct gggtgaccaa gtgagaccct gtctcaaaaa aaaaaaaaaa
3121 aaaaagaaaa gaaaattccc attaaagcac aaaggcccac ttattgaagc tattaaaata
3181 caggttgggg ccggctgggc atcgcgtcac gcctgtaatc ccagcacttt ggaaggccga
3241 ggtaggcgag tcacgagttc aggagatcga gaccatcctg gctaacacgg tgaaacccca
3301 tctctactaa aaatacaaaa aaaaaaatca gccgggcatg gtggcgggag cctatagtcc
3361 cagctactcg ggaggctgag gcaggagaat ggcatgagcc cgggaggcgg agcttgcagt
3421 gagccaaaat cacaccactg cactccagcc tgggcaacag atcgagactc catctgaaga
3481 aaaaaaaaat acaggttggg accacagtgg ctcatgcctg taatcctagt actttgggag
3541 tccgaagtag gtggatcacc tgaggtcagg actttgagac cagcctggcc aacatggcaa
3601 aaccccatct ctactaaaaa atatacaaaa attagctggg cgtggtggtg ggtgcctgta
3661 atcccagcta ctcaggaggc tgaggcagaa gaatcacaac aaccaggggg atggtggttg
3721 caatgagcca agatcatctc cacttcactc cggcccaggc aaaagagtga gagtcatctt
3781 aaaaaaaaaa aaaaaaaaaa aaaaaaaata cagattaggc attcctaatc tgaaaaattt
3841 ggctccaaaa tgctccagtc gagcatttcc tttgagtgtc atgtgggtgc tcaaaaagtt
3901 agatttttgg accattttca gatttcagag ttttggatta gggatgctcg actggtaagt
3961 aatcgagata ttccaaaaat ctggacaaat ctgaaatcca aaatgcttgg aatagcagat
4021 actcaactgg tagcactccc tggaagaata tgcaccaaac tgatagcagt ggttaccttc
4081 tggtgaggag gggaaagaac caagattagc agtaggatca acatatattt taatgttttc
4141 tgtattttta ttacttgtat aatttaaaca ttttaaatta gtaataatga acaatcatga
4201 aactatggat gatttagtcc agcaaaatat ccaattggga accctcatcc ttctgcagag
4261 cccaaatggc gcagtgggaa atgctgcaga atcttgacag cccctttcag gatcagctgc
4321 accagcttta ctcgcacagc ctcctgcctg tggacattcg acagtacttg gctgtctgga
4381 ttgaagacca gaactggtga ggccttcagg aagttggggg aatgaaaaag gtggccttcc
4441 acttctgggc ccccgggatc ctggaatcat taatggcagg aaggggttgg aaagcctcag
4501 gactacagta acactgcaga gacactaata cttcttattc ctggtcccag gcaggaagct
4561 gcacttggga gtgatgattc caaggctacc atgctattct tccacttctt ggatcagctg
4621 aactatgagt gtggccgttg cagccaggac ccagagtcct tgttgctgca gcacaatttg
4681 cggaaattct gccgggacat tcaggtactt ggaacggttg ggagtgatgg ggtagcactg
4741 ggagcagagc atagaggagt aaggtttgga gaatagaata gtacctggag gtggcaaggg
4801 agacgggaac aaatgtgggg aaaggaggac agagtctgga cttggggaat cactagcaga
4861 gagaagggtt gcatatacgt gacactgttg ggaggatgct atggtgaaaa gacaaagggc

4921 taagaacccc gaaggaggag gaaatactgt ggacattggt ggggagggtc tagggcaata
4981 ggtcattgag agtggttgaa ttggatcaat cctttctgtt tacctttctg ttagcccttt
5041 tcccaggatc ctacccagtt ggctgagatg atctttaacc tccttctgga agaaaaaaga
5101 attttgatcc aggctcagag ggcccaattg gtgaggacaa ttcagtggta atgttggaaa
5161 ctcctgaagt agagaggaac catggaaagg actcagggag ttgtctcaga acaggatccc
5221 cccgacatcc tgtggtataa tttcaggcct gaacttaagg catgaaaggc cagagttaaa
5281 acgtgctcag agcctctttt ttcaggaaca aggagagcca gttctcgaaa cacctgtgga
5341 gagccagcaa catgagattg aatcccggat cctggattta agggctatga tggaggttag
5401 tagatgtggt aggagttagg gttgacagtg ttcagcctaa cacctccctg agaagcagcc
5461 tcatcggggt cctctcccct ctgcagaagc tggtaaaatc catcagccaa ctgaaagacc
5521 agcaggatgt cttctgcttc cgatataaga tccaggccaa aggtaggaag cacattgagg
5581 ggctggagaa agataagtgc ctgctgagaa gccggagctg gaagtgaaca ggagaaagct
5641 ccgatgagca gtagtcactg tcagacacac cccactgact acagtcctgc tgccgtgcaa
5701 agctggaatc gtgctttgtg gaggctgagc tggaggtgac agctgagaga cagtaaattg
5761 ttgaggaaat gcatggaaaa ctaacagtgt tttatttgag gggtgtctgg gtccaagatg
5821 accacttcag aatttgcctg gagggtccca caggtgcctg tgctttgctt ggtttccctt
5881 tcttcctccg ccacaaaatt cctccttcct gactctgact gagaccccag tcaggaagga
5941 gaggaaagaa cccctggact gactcctgtt cccaccatcc agggaagaca ccctctctgg
6001 acccccatca gaccaaagag cagaagattc tgcaggaaac tctcaatgaa ctggacaaaa
6061 ggagaaaggt gggaggcagc agaacagaac atgtgggcaa caaggacctg aaaaaatgag
6121 ggatgttggg aaccctggta atctagcgct ggcttctttc tttcttcatc cccagttggg
6181 tggtggaggg tgaaagggag agatgctcaa cactcacatt atctctttcc caggaggtgc
6241 tggatgcctc caaagcactg ctaggccgat taactaccct aatcgagcta ctgctgccaa
6301 agttggagga gtggaaggcc cagcagcaaa aagcctgcat cagagctccc attgaccacg
6361 ggttggaaca gctggagaca tggtgagagg taccacccca accctcgtcc tcgccatgcg
6421 ctgtgatttg taagttgcag tgccctgcat atagcaagag atactgttct ctatttgtct
6481 ctgctcccca gaatagagcc ctgctccctg cctgactgca gctctattct gcctcctcag
6541 cctcaccacg cagggaagcc cagaagtccc agtctccttc agggaaagga atgaattaac
6601 ccacaatctg gttttgcttc ttttttttaa tcacccagaa atatatatat atgtattttt
6661 tttttactgc aacgaataca atgacaagaa aggaagggaa ggaaggaagg aagagaaaat
6721 tacctattac ctagcttatt aaacaaaaat ggaatcatat tgtccatact attttgaaat
6781 ccatggggtt ttttttaagc ttaacagtat tttatatata tatatatata tatatatata
6841 tatatatata tatatatata tatatttttt tttttttttt tttttttttt ttttgagacg
6901 gagtctctct ctgttccctg gctggcggag cggagtcggc acgatctcag ctcactgcaa
6961 cttccaactc ccacggttca agccaattct cctgtctcag cctcccgagc ctgggattac
7021 caggcacaca ccagcctggc tagttttttt gattttttag tagagacgat gtttctccat
7081 gttggccagg ctggtctcaa actcctgact tcaggtgatc cacccaactt gggctcccaa
7141 agtgctggga ttacaggcgt gacgaccatg cccggccaac agtatattat atttatccat
7201 gttatttctt atgtccacac aacagtcccc tatatggtgg taacataatt taattaatga
7261 actcctattt tcagctattt aggttatttt caatttcttg ttaccttttg ccaggaaacg
7321 tatattttat ggtaattata ttgtgttgta gaaaaatcac tagtctagtc caacttgctt
7381 gaaaaatagc tactttttaa ctattttctc atttaaaaat ttattataat ttagtctttt
7441 agaaatatac caggccaggc atggcgtctc atgcctgtta tcctagtact ttggaaggct
7501 gaggacggag gatcacttca gtcttggggt ttgagaccag cccgggaaac ataacaagac
7561 cccatctcta caaaaaaaaa aaattgtttt taattaggca tgtccgacac agtggctcac
7621 acatgtggcc agcactgtgg gaaggccaag gtgggtggat cacttgaggg tcaggagttc
7681 aagaccagcc tggccaatgt ggtgaaaccc catctctact aaaaatacaa aaatttgcca
7741 ggtgtggtgg cgcatgcctg tattcccagc tactcaggag gctaaggcag gaaatcactt
7801 gaactcggag gcagaggttg cagtgagctg tgacaatgcc actgtactcc agcctgggtg
7861 acagagcgag ctccgtctca aaaaaaaaaa aaaaagatta ggcatggtgg cacacgcctg
7921 tagaccctag ctactcagga ggctgaggtg ggaggattgc ttgagcccag gtgttggagg
7981 ctgcagtgag ccatgattat accactgtag tccagcctgg acaacagaac gagaccctgt
8041 ctctaaaagt atatatgtac acataccata atacccagct actgaggagg ctgaggcaga
8101 aagagtgctt gagtccagga gtttgatgtc agcctgagca atatagcaag accctcacct
8161 cttaaaaaaa tttaaagtag attaaaaaaa taccacaatt gctcaggtag attaaaaaaa
8221 taccacaatt gctcaggtag attattgaaa aacaggcata tagtacttat ggtacaggac
8281 cagcatgcat gcatgcatgc attgattgat tgattgattg attgattgag acagggtctc
8341 tctctgtctc ccaggctgga gtgcctggcc ttaagtgatc tgcccacctt tgcttcccaa
8401 agtgctgaga ttacaggtgt gagccaccat gtcagctggc gaggcttttt aaaagatagt
8461 tccaagtgtt acagctcttt taggatttgt ctagcaggct ttcaggtttt tgccagaaac
8521 cacccccacc cccaccaaaa aaaaaaaaaa aaaaaagata tgtacaagtt cccagatagt

8581 gttcccaact gaatctattt ctcatgtgta gtgtatggtt gttttcctgt caccacattg
8641 ctgattatta ttatttttaa ttatagagac agtaaagtac agtagttaaa aatgtgagtt
8701 ggggctgggt gcagtggctc acacctgtaa tcccagcact ttgggaggcc aaggtgggcg
8761 gatcacctga ggtcaggagt tcaagaccag cttggccaac atggcaaaac cccgtctcga
8821 ctaaaaatat atatatataa gttagccggg cgtggtggca acattacctg taatcccagc
8881 tactcgggag gccaacaggc aggagaatct cttgaatcca ggaggtggag gttgcagtga
8941 gccagatcac accattgcac tccagcctgg atgacaagag agtgagactg tctaaaaaaa
9001 aaaaacaaag tgtgagttgt acaatgagac tgcctgggat cacatacaag cttcatccct
9061 tactagttgt attgacccta aagcaagtca ctaacctttc tgtgccctcc agttttatca
9121 tctgtaatgt ggggaaaata atagtacctg cctcagaggg ttgttttgag gattaaatgc
9181 attaatatgt ggaaagggct taatataagt tgtacatagc atatgaaaac tgttatgtta
9241 aatctattag cagttttata tgtgaaaata gctttgattt tcatttcttg gattatgaat
9301 catgttgaat aatcctttat atgcttcctg gattcttttt ttttcttccc cccagtcagt
9361 ttctgactct tctcatattt atagagagat cttggaacct ggatggggga atccaggaaa
9421 ctcatggatt ccttcttcct gaattttatc acccaggttc acagctggag caaagctgtt
9481 gtttcacctg aggcagctgc tgaaggagct gaagggactg agttgcctgg ttagctatca
9541 ggatgaccct ctgaccaaag gggtggacct acgcaacgcc caggtcacag agttgctaca
9601 gcgtctgctc cacaggtcta gaggccaggc aggaaccctg ggggaaagaa ggaacaaggg
9661 aagccattct tacacatact gagctatata ttctctccac acctctctct cctcgagcct
9721 ttgtggtaga aacccagccc tgcatgcccc aaactcccca tcgacccctc atcctcaaga
9781 ctggcagcaa gttcaccgtc cgaacaaggt tggcattcca gaactcattc ccacttcctt
9841 tttccaaccc tgccactgtg tattttctgg ctttacagct actgcccact cttggctttt
9901 tcagtctttc ctgaatctcc ctacctcgtt gataccccat cgtcctcttt ttcaaacacc
9961 tagcctatac aaaagccgac tccgaccaca tttccctata ccccttgact tccccaggct
10021 gctggtgaga ctccaggaag gcaatgagtc actgactgtg gaagtctcca ttgacaggta
10081 aattggagca ggtgaagggt ggccaggaca cgggctgctg gggtggagga gatactcact
10141 cttcacaaca gggccctagg gctatatcct tcctccttcc aatcctacct cacagaaatt
10201 ataattcatt tcttttgttg aacacttact ttgtgacatg cagcatgtca gctactcatt
10261 taattgtcac accaacccca tgaataaact attaccagtg cactgtacaa acaaagatac
10321 aggcttagag agactgatta catctcttct caaggccaca tagctagtga gctcaagtcg
10381 ggtttgaacc gaggtctgtc tgatcccaaa gacgaaactc ctaacttcca tactcttttg
10441 cccaatgatt ttttttaaat ttatttcttt tcaggaatcc tcctcaatta caagggtagg
10501 tgcttgacaa ggacactgca aacatctgta cagtgtatga cctgcagaac cgggggattt
10561 gggaaatgga caaagggaga tggcgagatc tgaaatggaa gtggaacttc agtttttttt
10621 ttttctgctg agtttttaca ataattccat tccttgtctc catgtatctt cctcctggaa
10681 cagcttccgg aagttcaaca ttctgacttc aaaccagaaa actttgaccc ccgagaaggg
10741 gcagagtcag ggtttgattt gggactttgg ttacctggta agaatagttt gtgacctatg
10801 cttttattac tatttttatt ttttcgagac ggagtctcac tctgtccccc aggctggagt
10861 gcagtggtgc catcttggct cacaggaacc tccgccctcc ccggttcaag caattcttct
10921 gtctcagcct cctgagtacg tagagctata ggcagcacac caccatgccc ggctaatttt
10981 tgtattttta gtagagatag ggtttcacca tattggtcgg gctggtctcg aactcctgac
11041 ctcaggtgat ccgacccgcc tcagcctccc aaagtgctgg gatcacaggc atgagccacc
11101 atagctggcc tgcttttagt ccaaaggaac aggggttggg ggaagttccc aggcttgag
11161 aggtcttgaa gccaaacagg ggttccaggg agactagggt gcccactctg gcatttctc
11221 tccttccctt caattcacag actctggtgg agcaacgttc aggtggttca ggaaagggca
11281 gcaataaggt gagatctgga cagaggactc gaggcagggg gagcttgcca aagagccttc
11341 tgatgactat gtctttgcct gtcccagagg ggccactagg tgtgacagag gaactgcaca
11401 tcatcagctt cacggtcaaa tatacctacc agggtctgaa gcaggagctg aaagtgagtg
11461 aaaatggagg gcaaggagag agaaagcagc tttggaagaa ggcataagaa ggggataaac
11521 agaagcctct tggggagggt tagcactcct ttcctctaac aaatacctgc agctagaaac
11581 atcacatccc tctctgtgac tcctgtcttc tccccacaca cggacaccct ccctgtggtg
11641 attatttcca acatgaacca gctctcaatt gcctgggctt cagttctctg gttcaatttg
11701 ctcagcccaa accttcaggt aggggagtgg ggccgacagg tcccggcgcg agagcagggg
11761 tgtggaagct tggtgtgata ggttgcttct gagccagcct acactgctcc cacccctgca
11821 gaaccagcag ttcttctcca acccccccaa ggccccctgg agcttgctgg gccctgctct
11881 cagttggcag ttctcctcct atgttggccg aggcctcaac tcagaccagc tgagcatgct
11941 gagaaacaag ctgttcggta cagatttcct tttctctcag cctttcccca gccttagtct
12001 tttctgtccc tctgtcctat ctatcccagg acccctggct tccctcacat atctgtggct
12061 atctgtccca cagggcagaa ctgtaggact gaggatccat tattgtcctg ggctgacttc
12121 actaaggtaa ctccctgaat cctgtggagc tgctggatct agccccacat tccaaatact
12181 ggccttccca cgtgccctcc ttccctacac cagaggcaac tcctcagctt ttgctacctt

12241 tccattcctc cagcgagaga gccctcctgg caagttacca ttctggacat ggctggacaa
12301 aattctggag ttggtacatg accacctgaa ggatctctgg aatgatgggt aaggccttgg
12361 tcacccttcc ctcatgggct tgtgcttccg ggcttgagag tggagtctct gcaccctcac
12421 gtggcaagca gggagagaga gcaaagcacg gtgcaggcca cgtctcctca catttgttaa
12481 gaataataag gccgggtgtg gtggctcaca cctgtaatcc cagcactttg ggaggccgag
12541 gcgggcggat catgaggtca ggagatcgag accatcctgg ctaacacggt gaaaccccgt
12601 ctctactcta aaaatacaaa aaattagccg ggcgtggagg cagacaccct gtagtcccag
12661 ctactcagga ggctgaggca ggaaaatggc gtgaacctgg gagatggagc ttgcagtgag
12721 ccgagattgc gtcactgccc tccagccttg gggtgacgta gcaagactcc gtctcaaaaa
12781 aaaaaaaaaa aaacaaccaa taatagccat aaacagtgtt tttgtgaagc actcctacat
12841 tccagagctt gatgggtgct cttcattaat tctctcatct catccttaca accatgctga
12901 gtggtgggtt ttgccagctt catttcatgt gaggaaactg agtttcagag aagttaaaga
12961 acttacccaa gggacacagt tgatattcaa atccaggcct atgtgactcc aagcccatgc
13021 tctttccacc acactgccta ccaacttgtg tagcatttgg cttttaaaag tgctattcat
13081 gaccaggcac gatggctcac gccttgtaat cccagcattt tgggaggccg aggtgggtgg
13141 atcacctgag gtcaggagtt tgagaccagc ctggccaaca tggcgaaacc ccatctctat
13201 taaaaataca aaaattagcc gggtgtggtg gtgggcgcct gtaatcccag ctactcagga
13261 ggctgaggag gagaatcgct tgaatttagg agagaaggtt acagtgagcc aagatcgtgc
13321 cattgcactc cagcctgggt gacagagcaa gactctgtct caaaacaaaa ccaaaaaaaa
13381 gtgctatttg tggccaggcg tggttgctca tgcctgtaat cctagcattt ttggggaggc
13441 tgaggagtac agatcacttg agcccaggag ttcaaaacta ccctgggcca cgtggtgaaa
13501 ccccaaaccc cgtctctacg aaaaatacaa aagttagcca ggatgggtgg tgtgcacctg
13561 tggtcccagc tactctggag gctgagaggt ggggaagatt gcttgagccc gggaggtcga
13621 ggtggcagtg agctgtgatc atgccactat tctccagcct gggtgacaga atacaccctg
13681 tctccctgtc tcccagaaaa aaaaaaaagt gctgttcatc tgtgtgatct cactgaatct
13741 tcgtacttca aaccctcgga aggtggctat tgtcagcaaa gtgaagtgac ttgtaaaaga
13801 taaaaaaaag ctaagtggca gggcttggtc caaagcctgg attccaaacc tgggctgttt
13861 ctccatacaa ggggagcagg gaggcagggg cctggggggg cagggtgttg ggcggtgtca
13921 cacgtgacac actgtgctcc agacgcatca tgggctttgt gagtcggagc caggagcgcc
13981 ggctgctgaa gaagaccatg tctggcacct ttctactgcg cttcagtgaa tcgtcagaag
14041 ggggcattac ctgctcctgg gtggagcacc aggatgatgg tagctgctct gccctgccat
14101 tcccacagcc tctcctttct gcctggctct cctctggccc ctctgcctgc cttgcttcgc
14161 tggctctgaa ctgaatgctc agtggtttgg gactgggcag ccagagagtc agagagctcc
14221 aaggcccggc ctcttccctc aagcccgcct gttcctgcat tcactctcca gacaaggtgc
14281 tcatctactc tgtgcaaccg tacacgaagg aggtgctgca gtcactcccg ctgactgaaa
14341 tcatccgcca ttaccagttg ctcactgagg agaatatacc tgaaaaccca ctgcgcttcc
14401 tctatccccg aatcccccgg gatgaagctt ttgggtgcta ctaccaggag aaaggtggga
14461 atcgttgaca tacttcattg ctagattgca gagatctacc agacatccat agatcccact
14521 ccttccttta aagcatggga aaactgatat ctagaggaat taagggattc gtccatggga
14581 tactgctggt tactatgggg atgagactgc caggaccatc tgcactaggg gaaaacctca
14641 ggctatatgt ctggcccact gatcttctct gcttcttgta tatgttcctc acagttaatc
14701 tccaggaacg gaggaaatac ctgaaacaca ggctcattgt ggtctctaat agagtgagat
14761 atgaactgtt cattcatcct ccctaatcct tattggctct gcttcagtga atcgtcaaaa
14821 gggggcatta ccttctcctg ggtggagcac caggatgatg gtcagctgct ctgccctgcc
14881 attcccacag cctctccttt ctgccttctc ctaagctgcc cctattccag tctccccagc
14941 cttccctccc tcctagcccc actctagttt tttctggttc tagtctctcc tatctcatat
15001 ttttctgctg ccatccttag gttgtctcca caggggtttc tggataataa tgatcataat
15061 cactggtgtt aaggggtacc tacttgatgc aagcatggag cttttttttt ttccagacag
15121 ggttttgttc tgtcgcccag gctggagtgc agtggtgtga tcctggctca ctgcagcctc
15181 gacctcctga gctcaagcaa tacaggcatg catcaccaaa ctcagctaat tttttttgta
15241 ttttttgtag agatggggtc ttaccatgtt gacgcatcag gctgttctga actcctggac
15301 tcaagcaatc cacccacctt ggcctcccaa aagtcaggga ttacaggcgt gcgaccacac
15361 cccgcatata tatatttttt tttttttttt tttttttttt tttttgagac agggtctctg
15421 ttatccaggc tggagttgca gtggataata tgactacgag ccttgaccta ggggttgaag
15481 caatgctcct gcctcagcca ccaagtgctg agactacagg cacacgccaa tctacactca
15541 atcacactca gctaattttt taaatttttt gtagggatgg ggtatcactg tgtttgccca
15601 ggctggtctt gaactcctgg cctcaagcag tctcctgcct tggcctccca aattgccggg
15661 attgtaggaa tgagccatgg cacttggctg ggggatagaa tttttttttt tttttttttt
15721 tttttttttt ttgagacagt ctcactctca ttgcccgggc tggagtgcag tggtgcaatt
15781 tcagctcact gcaacctctg cctcccaggc tcaagcaatt ctcctgcctc agcctataga
15841 gtagctggga ttacaggcga gcgccaccca tgcctggtta atttttgttt tttttttgag

15901 acagagtctc gccctgttgc ccaggctgga gtgcagtggc acgatctcag ctcactgcaa
15961 cctctgcctc ccaggctcaa gcaattctcc tgcctcagcc tcctgagtac tgggactaca
16021 agcgcgcaca accaccacac ctggtaattt ttgtattttt agtagagaca gggtttacc
16081 atattggcca ggctggtctc aaactcctga cctcatgatc cgacccacct tggcctccca
16141 aagtgcaggg attacaggcg tgagcctctg cacccggcct aacttttgta tttttagtag
16201 aaacagggtt tcaccatgtt ggccaggctg gtcatgagct cctggcctca agtgatctgc
16261 ccgcctcagc ctcccaaagt gcttggatta caggtgtgag ccacctggcc tgagagttta
16321 ttatgcgcca ggcactaggc aaatggtttg catttatttt ctcattttat tgaatctaca
16381 aaatagtcct gtgaagtaaa cactgttact gttttcagct aaggaactgg atttagagta
16441 gtcaagtttt gtacctaagg tacgtggcta atgatacagg tctgttagat tccgtagccc
16501 tgattttaac caccctactg cctctcaaga attactaggt attgttctca tttatagatg
16561 ataaatctga ggctcagaaa agttaggcca cttgcctaag gtcccccagc caggattcaa
16621 actccaggag gcctgattcc aaacccatgc tctttagccc tccgccctac tgccttctta
16681 gactagcttc tgcttattct accattcctg atttcatttg aaccactgag ccctgcccct
16741 ttgtctgtct ttgggtatcc aggcaggtgg atgaactgca acaaccgctg gagcttaagc
16801 cagagccaga gctggagtca ttagagctgg aactagggct ggtgccagag ccagagctca
16861 gcctggactt agagccactg ctgaaggcag ggctggatct ggggccagag ctagagtctg
16921 tgctggagtc cactctggag cctgtgatag agcccacact atgcatggta tcacaaacag
16981 tgccagagcc agaccaagga cctgtatcac agccagtgcc agagccagat ttgccctgtg
17041 atctgagaca tttgaacact gagccaatgg aaagtaagtg atgagatgga gtggcacaca
17101 ttccctttcc tacctcttct ccctctccca ttacagaaaa agctgaactc caagctcctc
17161 attggagaga ggtccatctg tgattccttt ttttaggaat tacacatgcc ttcccccacc
17221 tccctgctct ttcatcccac aagttcccac tcaggctctt cccaggcctt tcctgccatc
17281 ctccctccct tgggctgctg ggttgggaac tcctaactaa gatcggggcc tcacttttct
17341 ctctggatta cctagtcttc agaaactgtg taaagattga agaaatcatg ccgaatggtg
17401 acccactgtt ggctggccag aacaccgtgg atgaggttta cgtctcccgc cccagccact
17461 tctacactga tggacccttg atgccttctg acttctagga accacatttc ctctgttctt
17521 ttcatatctc tttgcccttc ctactcctca tagcatgata ttgttctcca aggatgggaa
17581 tcaggcatgt gtcccttcca agctgtgtta actgttcaaa ctcaggcctg tgtgactcca
17641 ttggggtgag aggtgaaagc ataacatggg tacagagggg acaacaatga atcagaacag
17701 atgctgagcc ataggtctaa ataggatcct ggaggctgcc tgctgtgctg ggaggtatag
17761 gggtcctggg ggcaggccag ggcagttgac aggtacttgg agggctcagg gcagtggctt
17821 ctttccagta tggaaggatt tcaacatttt aatagttggt taggctaaac tggtgcatac
17881 tggcattggc cttggtgggg agcacagaca caggatagga ctccatttct ttcttccatt
17941 ccttcatgtc taggataact tgctttcttc tttcctttac tcctggctca agccctgaat
18001 ttcttctttt cctgcagggg ttgagagctt tctgccttag cctaccatgt gaaactctac
18061 cctgaagaaa gggatggata ggaagtagac ctctttttct taccagtctc ctcccctact
18121 ctgcccccta agctggctgt acctgttcct cccccataaa atgatcctgc caatctaatg
18181 tgagtgtgaa gtttgcacac tagtttatgc tacctagtct ccactttctc aatgcttagg
18241 agacagatca ctcctggagg ctggggatgg taggattgct ggggattttt tttttttaa
18301 agagggtctc actctgttgc ccaggctaga gtgcaatggt gcaatcacag ctcactgcag
18361 cctcaacctc ctgggttcaa gcaatcctcc tacctcagcc tcctgggtag ctagcaccat
18421 ggcatcgcca ccatgcccta tttttttttt ttaaagacag ggtcttgcta tattgcccag
18481 gctggtcttg aactgggctc aagtgatcct cacgccttgc ctcccaaagt gctgggatta
18541 taggcatgag ccactgtgct tggccaggat tttttttttt ttttttttga gatggagttt
18601 ctctcttgtt gtccaggctg gagtgcaatg gtgtgatccg gggaattc

Protein Variants: Protein variants can include amino acid sequence modifications. For example, amino acid sequence modifications fall into one or more of three classes: substitutional, insertional or deletional variants. Insertions can include amino and/or carboxyl terminal fusions as well as intrasequence insertions of single or multiple amino acid residues. Insertions ordinarily will be smaller insertions than those of amino or carboxyl terminal fusions, for example, on the order of one to four residues. Deletions are characterized by the removal of one or more amino acid residues from the protein sequence. These variants ordinarily are prepared by site-specific mutagenesis of nucleotides in the DNA encoding the protein, thereby producing DNA encoding the variant, and thereafter expressing the DNA in recombinant cell culture.

Techniques for making substitution mutations at predetermined sites in DNA having a known sequence are well known, for example M13 primer mutagenesis and PCR mutagenesis. Amino acid substitutions can be single residues, but can occur at a number of different locations at once. In one non-limiting embodiment, insertions can be on the order of about from 1 to about 10 amino acid residues, while deletions can range from about 1 to about 30 residues. Deletions or insertions can be made in adjacent pairs (for example, a deletion of about 2 residues or insertion of about 2 residues). Substitutions, deletions, insertions, or any combination thereof can be combined to arrive at a final construct. The mutations cannot place the sequence out of reading frame and should not create complementary regions that can produce secondary mRNA structure. Substitutional variants are those in which at least one residue has been removed and a different residue inserted in its place.

Substantial changes in function or immunological identity are made by selecting residues that differ more significantly in their effect on maintaining (a) the structure of the polypeptide backbone in the area of the substitution, for example as a sheet or helical conformation, (b) the charge or hydrophobicity of the molecule at the target site or (c) the bulk of the side chain. The substitutions that can produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

Minor variations in the amino acid sequences of proteins are provided by the present disclosure. The variations in the amino acid sequence can be when the sequence maintains at least about 30%, at least about 40%, at least about 50%, at least about 60%, at least about 70%, at least about 75%, at least about 80%, at least about 90%, at least about 95%, or at least about 99% identity to SEQ ID NO: 1, 3, 5, or 7. For example, conservative amino acid replacements can be utilized. Conservative replacements are those that take place within a family of amino acids that are related in their side chains, wherein the interchangeability of residues have similar side chains.

Genetically encoded amino acids are generally divided into families: (1) acidic amino acids are aspartate, glutamate; (2) basic amino acids are lysine, arginine, histidine; (3) non-polar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. The hydrophilic amino acids include arginine, asparagine, aspartate, glutamine, glutamate, histidine, lysine, serine, and threonine. The hydrophobic amino acids include alanine, cysteine, isoleucine, leucine, methionine, phenylalanine, proline, tryptophan, tyrosine and valine. Other families of amino acids include (i) a group of amino acids having aliphatic-hydroxyl side chains, such as serine and threonine; (ii) a group of amino acids having amide-containing side chains, such as asparagine and glutamine; (iii) a group of amino acids having aliphatic side chains such as glycine, alanine, valine, leucine, and isoleucine; (iv) a group of amino acids having aromatic side chains, such as phenylalanine, tyrosine, and tryptophan; and (v) a group of amino acids having sulfur-containing side chains, such as cysteine and methionine. Useful conservative amino acids substitution groups are: valine-leucine-isoleucine, phenylalanine-tyrosine, lysine-arginine, alanine valine, glutamic-aspartic, and asparagine-glutamine.

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gin; Ser, Thr; Lys, Arg; and Phe, Tyr. Substitutional or deletional mutagenesis can be employed to insert sites for N-glycosylation (Asn-X-Thr/Ser) or O-glycosylation (Ser or Thr). Deletions of cysteine or other labile residues also can be desirable. Deletions or substitutions of potential proteolysis sites, e.g. Arg, is accomplished for example by deleting one of the basic residues or substituting one by glutaminyl or histidyl residues.

Bacterial and Yeast Expression Systems. In bacterial systems, a number of expression vectors can be selected. For example, when a large quantity of a protein encoded by a gene, such as Jak 1, Jak 2, Stat 1or Stat 2, is needed for the induction of antibodies, vectors which direct high level expression of proteins that are readily purified can be used. Non-limiting examples of such vectors include multifunctional *E. coli* cloning and expression vectors such as BLUESCRIPT (Stratagene). pIN vectors or pGEX vectors (Promega, Madison, Wis.) also can be used to express foreign polypeptide molecules as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

Plant and Insect Expression Systems. If plant expression vectors are used, the expression of sequences encoding a Jak 1, Jak 2, Stat 1or Stat 2 protein can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters, can be used. These constructs can be introduced into plant cells by direct DNA transformation or by pathogen-mediated transfection.

An insect system also can be used to express Jak 1, Jak 2, Stat 1or Stat 2 proteins. For example, in one such system *Autographa californica* nuclear polyhedrosis virus (AcNPV) is used as a vector to express foreign genes in *Spodoptera frugiperda* cells or in *Trichoplusia* larvae. Sequences encoding a polypeptide of Jak 1, Jak 2, Stat 1or Stat 2 can be cloned into a non-essential region of the virus, such as the polyhedrin gene, and placed under control of the polyhedrin promoter. Successful insertion of nucleic acid sequences, such as a sequence corresponding to a gene, such as a Jak 1, Jak 2, Stat 1or Stat 2 gene, will render the polyhedrin gene inactive and produce recombinant virus lacking coat protein. The recombinant viruses can then be used to infect *S. frugiperda* cells or *Trichoplusia* larvae in which the protein or a variant thereof can be expressed.

Mammalian Expression Systems. An expression vector can include a nucleotide sequence that encodes a Jak 1, Jak 2, Stat 1or Stat 2 polypeptide linked to at least one regulatory sequence in a manner allowing expression of the nucleotide sequence in a host cell. A number of viral-based expression systems can be used to express a Jak 1, Jak 2, Stat 1or Stat 2 protein or a variant thereof in mammalian host cells. For example, if an adenovirus is used as an expression vector, sequences encoding a protein can be ligated into an adenovirus transcription/translation complex comprising the late promoter and tripartite leader sequence. Insertion into a non-essential E1 or E3 region of the viral genome can be used to obtain a viable virus which expresses a Jak 1, Jak 2, Stat 1or Stat 2 protein in infected host cells. Transcription enhancers, such as the Rous sarcoma virus (RSV) enhancer, can also be used to increase expression in mammalian host cells.

Regulatory sequences are well known in the art, and can be selected to direct the expression of a protein or polypeptide of interest in an appropriate host cell as described in Goeddel, Gene Expression Technology: Methods in Enzymology 185, Academic Press, San Diego, Calif. (1990). Non-limiting examples of regulatory sequences include: polyadenylation signals, promoters (such as CMV, ASV, SV40, or other viral promoters such as those derived from bovine papilloma, polyoma, and Adenovirus 2 viruses (Fiers, et al., 1973, Nature 273:113; Hager GL, et al., Curr Opin Genet Dev, 2002, 12(2):137-41) enhancers, and other expression control elements.

Enhancer regions, which are those sequences found upstream or downstream of the promoter region in non-coding DNA regions, are also known in the art to be important in optimizing expression. If needed, origins of replication from viral sources can be employed, such as if a prokaryotic host is utilized for introduction of plasmid DNA. However, in eukaryotic organisms, chromosome integration is a common mechanism for DNA replication.

For stable transfection of mammalian cells, a small fraction of cells can integrate introduced DNA into their genomes. The expression vector and transfection method utilized can be factors that contribute to a successful integration event. For stable amplification and expression of a desired protein, a vector containing DNA encoding a protein of interest is stably integrated into the genome of eukaryotic cells (for example mammalian cells, such as cells from the end bulb of the hair follicle), resulting in the stable expression of transfected genes. An exogenous nucleic acid sequence can be introduced into a cell (such as a mammalian cell, either a primary or secondary cell) by homologous recombination as disclosed in U.S. Patent 5,641,670.

A gene that encodes a selectable marker (for example, resistance to antibiotics or drugs, such as ampicillin, neomycin, G418, and hygromycin) can be introduced into host cells along with the gene of interest in order to identify and select clones that stably express a gene encoding a protein of interest. The gene encoding a selectable marker can be introduced into a host cell on the same plasmid as the gene of interest or can be introduced on a separate plasmid. Cells containing the gene of interest can be identified by drug selection wherein cells that have incorporated the selectable marker gene will survive in the presence of the drug. Cells that have not incorporated the gene for the selectable marker die. Surviving cells can then be screened for the production of the desired protein molecule (for example, a protein encoded by a gene, such as Jak 1, Jak 2, Stat 1or Stat 2).

### Cell Transfection

A eukaryotic expression vector can be used to transfect cells in order to produce proteins encoded by nucleotide sequences of the vector. Mammalian cells (such as isolated cells from the hair bulb; for example dermal sheath cells and dermal papilla cells) can contain an expression vector (for example, one that contains a gene encoding a Jak 1, Jak 2, Stat 1or Stat 2 protein or polypeptide) via introducing the expression vector into an appropriate host cell via methods known in the art.

A host cell strain can be chosen for its ability to modulate the expression of the inserted sequences or to process the expressed polypeptide encoded by a gene, such as a Jak 1, Jak 2, Stat 1or Stat 2 gene, in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, Va. 20110-2209) and can be chosen to ensure the correct modification and processing of the foreign protein.

An exogenous nucleic acid can be introduced into a cell via a variety of techniques known in the art, such as lipofection, microinjection, calcium phosphate or calcium chloride precipitation, DEAE-dextran-mediated transfection, or electroporation. Electroporation is carried out at approximate voltage and capacitance to result in entry of the DNA construct(s) into cells of interest (such as cells of the end bulb of a hair follicle, for example dermal papilla cells or dermal sheath cells). Other transfection methods also include modified calcium phosphate precipitation, polybrene precipitation, liposome fusion, and receptor-mediated gene delivery.

Cells that will be genetically engineered can be primary and secondary cells obtained from various tissues, and include cell types which can be maintained and propagated in culture. Non-limiting examples of primary and secondary cells include epithelial cells (for example, dermal papilla cells, hair follicle cells, inner root sheath cells, outer root sheath cells, sebaceous gland cells, epidermal matrix cells), neural cells, endothelial cells, glial cells, fibroblasts, muscle cells (such as myoblasts) keratinocytes, formed elements of the blood (e.g., lymphocytes, bone marrow cells), and precursors of these somatic cell types.

Vertebrate tissue can be obtained by methods known to one skilled in the art, such a punch biopsy or other surgical methods of obtaining a tissue source of the primary cell type of interest. In one embodiment, a punch biopsy or removal can be used to obtain a source of keratinocytes, fibroblasts, endothelial cells, or mesenchymal cells (for example, hair follicle cells or dermal papilla cells). In another embodiment, removal of a hair follicle can be used to obtain a source of fibroblasts, keratinocytes, endothelial cells, or mesenchymal cells (for example, hair follicle cells or dermal papilla cells). A mixture of primary cells can be obtained from the tissue, using methods readily practiced in the art, such as explanting or enzymatic digestion (for examples using enzymes such as pronase, trypsin, collagenase, elastase dispase, and chymotrypsin). Biopsy methods have also been described in United States Patent Application Publication 2004/0057937 and PCT application publication WO 2001/32840.

Primary cells can be acquired from the individual to whom the genetically engineered primary or secondary cells are administered. However, primary cells can also be obtained from a donor, other than the recipient, of the same species. The cells can also be obtained from another species (for example, rabbit, cat, mouse, rat, sheep, goat, dog, horse, cow, bird, or pig). Primary cells can also include cells from an isolated vertebrate tissue source grown attached to a tissue culture substrate (for example, flask or dish) or grown in a suspension; cells present in an explant derived from tissue; both of the aforementioned cell types plated for the first time; and cell culture suspensions derived from these plated cells. Secondary cells can be plated primary cells that are removed from the culture substrate and replated, or passaged, in addition to cells from the subsequent passages. Secondary cells can be passaged one or more times. These primary or secondary cells can contain expression vectors having a gene that encodes a protein of interest (for example, a Jak 1, Jak 2, Stat 1or Stat 2 protein or polypeptide).

### Cell Culturing

Various culturing parameters can be used with respect to the host cell being cultured. Appropriate culture conditions for mammalian cells are well known in the art (Cleveland WL, et al., J Immunol Methods, 1983, 56(2): 221-234) or can be determined by the skilled artisan (see, for example, Animal Cell Culture: A Practical Approach 2nd Ed., Rickwood, D. and Hames, B. D., eds. (Oxford University Press: New York, 1992)). Cell culturing conditions can vary according to the type of host cell selected. Commercially available medium can be utilized. Non-limiting examples of medium include, for example, Minimal Essential Medium (MEM, Sigma, St. Louis, Mo.); Dulbecco's Modified Eagles Medium (DMEM, Sigma); Ham's F10 Medium (Sigma); HyClone cell culture medium (HyClone, Logan, Utah); RPMI-1640 Medium (Sigma); and chemically-defined (CD) media, which are formulated for various cell types, e.g., CD-CHO Medium (Invitrogen, Carlsbad, Calif.).

The cell culture media can be supplemented as necessary with supplementary components or ingredients, including optional components, in appropriate concentrations or amounts, as necessary or desired. Cell culture medium solutions provide at least one component from one or more of the following categories: (1) an energy source, usually in the form of a carbohydrate such as glucose; (2) all essential amino acids, and usually the basic set of twenty amino acids plus cysteine; (3) vitamins and/or other organic compounds required at low concentrations; (4) free fatty acids or lipids, for example linoleic acid; and (5) trace elements, where trace elements are defined as inorganic compounds or naturally occurring elements that can be required at very low concentrations, usually in the micromolar range.

The medium also can be supplemented electively with one or more components from any of the following categories: (1) salts, for example, magnesium, calcium, and phosphate; (2) hormones and other growth factors such as, serum, insulin, transferrin, and epidermal growth factor; (3) protein and tissue hydrolysates, for example peptone or peptone mixtures which can be obtained from purified gelatin, plant material, or animal byproducts; (4) nucleosides and bases such as, adenosine, thymidine, and hypoxanthine; (5) buffers, such as HEPES; (6) antibiotics, such as gentamycin or ampicillin; (7) cell protective agents, for example pluronic polyol; and (8) galactose. In one embodiment, soluble factors can be added to the culturing medium.

The mammalian cell culture that can be used with the present disclosure is prepared in a medium suitable for the type of cell being cultured. In one embodiment, the cell culture medium can be any one of those previously discussed (for example, MEM) that is supplemented with serum from a mammalian source (for example, fetal bovine serum (FBS)). In another embodiment, the medium can be a conditioned medium to sustain the growth of epithelial cells or cells obtained from the hair bulb of a hair follicle (such as dermal papilla cells or dermal sheath cells). For example, epithelial cells can be cultured according to Barnes and Mather in Animal Cell Culture Methods (Academic Press, 1998). In a further embodiment, epithelial cells or hair follicle cells can be transfected with DNA vectors containing genes that encode a polypeptide or protein of interest (for example, a Jak 1 Jak 2, Stat 1or Stat 2 protein or polypeptide). In other embodiments, cells are grown in a suspension culture (for example, a three-dimensional culture such as a hanging drop culture) in the presence of an effective amount of enzyme, wherein the enzyme substrate is an extracellular matrix molecule in the suspension culture. For example, the enzyme can be a hyaluronidase. Epithelial cells or hair follicle cells can be cultivated according to methods practiced in the art, for example, as those described in PCT application publication WO 2004/044188 and in U.S. Patent Application Publication No. 2005/0272150, or as described by Harris in Handbook in Practical Animal Cell Biology: Epithelial Cell Culture (Cambridge Univ. Press, Great Britain; 1996; see Chapter 8),.

A suspension culture is a type of culture wherein cells, or aggregates of cells (such as aggregates of DP cells), multiply while suspended in liquid medium. A suspension culture comprising mammalian cells can be used for the maintenance of cell types that do not adhere or to enable cells to manifest specific cellular characteristics that are not seen in the adherent form. Some types of suspension cultures can include three-dimensional cultures or a hanging drop culture. A hanging-drop culture is a culture in which the material to be cultivated is inoculated into a drop of fluid attached to a flat surface (such as a coverglass, glass slide, Petri dish, flask, and the like), and can be inverted over a hollow surface. Cells in a hanging drop can aggregate toward the hanging center of a drop as a result of gravity. However, according to the methods disclosed herein, cells cultured in the presence of a protein that degrades the extracellular matrix (such as collagenase, chondroitinase, hyaluronidase, and the like) will become more compact and aggregated within the hanging drop culture, for degradation of the ECM will allow cells to become closer in proximity to one another since less of the ECM will be present. See also International PCT Publication No. WO2007/100870.

Cells obtained from the hair bulb of a hair follicle (such as dermal papilla cells or dermal sheath cells) can be cultured as a single, homogenous population (for example, comprising DP cells) in a hanging drop culture so as to generate an aggregate of DP cells. Cells can also be cultured as a heterogeneous population (for example, comprising DP and DS cells) in a hanging drop culture so as to generate a chimeric aggregate of DP and DS cells. Epithelial cells can be cultured as a monolayer to confluency as practiced in the art. Such culturing methods can be carried out essentially according to methods described in Chapter 8 of the Handbook in Practical Animal Cell Biology: Epithelial Cell Culture (Cambridge Univ. Press, Great Britain; 1996); Underhill CB, J Invest Dermatol, 1993, 101(6):820-6); in Armstrong and Armstrong, (1990) J Cell Biol 110:1439-55; or in Animal Cell Culture Methods (Academic Press, 1998).

Three-dimensional cultures can be formed from agar (such as Gey's Agar), hydrogels (such as matrigel, agarose, and the like; Lee et al., (2004) Biomaterials 25: 2461-2466) or polymers that are cross-linked. These polymers can comprise natural polymers and their derivatives, synthetic polymers and their derivatives, or a combination thereof. Natural polymers can be anionic polymers, cationic polymers, amphipathic polymers, or neutral polymers. Non-limiting examples of anionic polymers can include hyaluronic acid, alginic acid (alginate), carageenan, chondroitin sulfate, dextran sulfate, and pectin. Some examples of cationic polymers, include but are not limited to, chitosan or polylysine. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73). Examples of amphipathic polymers can include, but are not limited to collagen, gelatin, fibrin, and carboxymethyl chitin. Non-limiting examples of neutral polymers can include dextran, agarose, or pullulan. (Peppas et al., (2006) Adv Mater. 18: 1345-60; Hoffman, A. S., (2002) Adv Drug Deliv Rev. 43: 3-12; Hoffman, A. S., (2001) Ann NY Acad Sci 944: 62-73).

Cells suitable for culturing according to methods disclosed herein can harbor introduced expression vectors, such as plasmids. The expression vector constructs can be introduced via transformation, microinjection, transfection, lipofection, electroporation, or infection. The expression vectors can contain coding sequences, or portions thereof, encoding the proteins for expression and production. Expression vectors containing sequences encoding the produced proteins and polypeptides, as well as the appropriate transcriptional and translational control elements, can be generated using methods well known to and practiced by those skilled in the art. These methods include synthetic techniques, *in vitro* recombinant DNA techniques, and *in vivo* genetic recombination which are described in J. Sambrook et al., 2001, Molecular Cloning, A Laboratory Manual, Cold Spring Harbor Press, Plainview, N.Y. and in F. M. Ausubel et al., 1989, Current Protocols in Molecular Biology, John Wiley & Sons, New York, N.Y.

### Obtaining and Purifying Polypeptides

A polypeptide molecule encoded by a gene, such as a Jak 1, Jak 2, Stat 1or Stat 2 gene, or a variant thereof, can be obtained by purification from human cells expressing a protein or polypeptide encoded by a Jak 1, Jak 2, Stat 1or Stat 2 gene via in vitro or in vivo expression of a nucleic acid sequence encoding a Jak 1, Jak 2, Stat 1or Stat 2 protein or polypeptide; or by direct chemical synthesis.

Detecting Polypeptide Expression. Host cells which contain a nucleic acid encoding a Jak 1, Jak 2, Stat 1or Stat 2 protein or polypeptide, and which subsequently express a protein encoded by a Jak 1, Jak 2, Stat 1or Stat 2 gene, can be identified by various procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a nucleic acid encoding a Jak 1, Jak 2, Stat 1or Stat 2 protein or polypeptide can be detected by DNA-DNA or DNA-RNA hybridization or amplification using probes or fragments of nucleic acids encoding a Jak 1, Jak 2, Stat 1or Stat 2 protein or polypeptide. In one embodiment, a fragment of a nucleic acid of a Jak 1, Jak 2, Stat 1or Stat 2 gene can encompass any portion of at least about 8 consecutive nucleotides of SEQ ID NO: 2, 4, 6, or 8. In another embodiment, the fragment can comprise at least about 10 consecutive nucleotides, at least about 15 consecutive nucleotides, at least about 20 consecutive nucleotides, or at least about 30 consecutive nucleotides of SEQ ID NO: 2, 4, 6, or 8. Fragments can include all possible nucleotide lengths between about 8 and about 100 nucleotides, for example, lengths between about 15 and about 100 nucleotides, or between about 20 and about 100 nucleotides. Nucleic acid amplification-based assays involve the use of oligonucleotides selected from sequences encoding a polypeptide encoded by a Jak 1, Jak 2, Stat 1or Stat 2 gene to detect transformants which contain a nucleic acid encoding a Jak 1, Jak 2, Stat 1or Stat 2 protein or polypeptide.

Protocols for detecting and measuring the expression of a polypeptide encoded by a gene, such as a Jak 1, Jak 2, Stat 1or Stat 2 gene, using either polyclonal or monoclonal antibodies specific for the polypeptide are well established. Non-limiting examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on a polypeptide encoded by a gene, such as a Jak 1, Jak 2, Stat 1or Stat 2 gene, can be used, or a competitive binding assay can be employed.

Labeling and conjugation techniques are known by those skilled in the art and can be used in various nucleic acid and amino acid assays. Methods for producing labeled hybridization or PCR probes for detecting sequences related to nucleic acid sequences encoding a protein, such as Jak 1, Jak 2, Stat 1or Stat 2, include, but are not limited to, oligolabeling, nick translation, end-labeling, or PCR amplification using a labeled nucleotide. Alternatively, nucleic acid sequences encoding a polypeptide encoded by a gene, such as a Jak 1, Jak 2, Stat 1or Stat 2 gene, can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, and/or magnetic particles.

Expression and Purification of Polypeptides. Host cells transformed with a nucleic acid sequence encoding a polypeptide, such as Jak 1, Jak 2, Stat 1or Stat 2, can be cultured under conditions suitable for the expression and recovery of the protein from cell culture. The polypeptide produced by a transformed cell can be secreted or contained intracellularly depending on the sequence and/or the vector used. Expression vectors containing a nucleic acid sequence encoding a polypeptide, such as Jak 1, Jak 2, Stat 1or Stat 2, can be designed to contain signal sequences which direct secretion of soluble polypeptide molecules encoded by a gene, such as a Jak 1, Jak 2, Stat 1or Stat 2 gene, or a variant thereof, through a prokaryotic or eukaryotic cell membrane or which direct the membrane insertion of membrane-bound a polypeptide molecule encoded by a Jak 1, Jak 2, Stat 1or Stat 2 gene or a variant thereof.

Other constructions can also be used to join a gene sequence encoding a Jak 1, Jak 2, Stat 1or Stat 2 polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Including cleavable linker sequences (i.e., those specific for Factor Xa or enterokinase (Invitrogen, San Diego, Calif.)) between the purification domain and a polypeptide encoded by a Jak 1, Jak 2, Stat 1or Stat 2 gene also can be used to facilitate purification. One such expression vector provides for expression of a fusion protein containing a polypeptide encoded by a Jak 1, Jak 2, Stat 1or Stat 2 gene and 6 histidine residues preceding a thioredoxin or an enterokinase cleavage site. The histidine residues facilitate purification by immobilized metal ion affinity chromatography, while the enterokinase cleavage site provides a means for purifying the polypeptide encoded by a Jak 1, Jak 2, Stat 1or Stat 2 gene.

A Jak 1, Jak 2, Stat 1or Stat 2 polypeptide can be purified from any human or non-human cell which expresses the polypeptide, including those which have been transfected with expression constructs that express a Jak 1, Jak 2, Stat 1or Stat 2 protein. A purified Jak 1, Jak 2, Stat 1or Stat 2 protein can be separated from other compounds which normally associate with a protein encoded by a Jak 1, Jak 2, Stat 1or Stat 2 gene in the cell, such as certain proteins, carbohydrates, or lipids, using methods practiced in the art. Non-limiting methods include size exclusion chromatography, ammonium sulfate fractionation, ion exchange chromatography, affinity chromatography, and preparative gel electrophoresis.

Chemical Synthesis. Nucleic acid sequences comprising a gene, such as a Jak 1, Jak 2, Stat 1or Stat 2 gene, that encodes a polypeptide can be synthesized, in whole or in part, using chemical methods known in the art. Alternatively, a polypeptide, such as Jak 1, Jak 2, Stat 1or Stat 2, can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of Jak 1, Jak 2, Stat 1or Stat 2 polypeptides can be separately synthesized and combined using chemical methods to produce a full-length molecule. In one embodiment, a fragment of a nucleic acid sequence that comprises a Jak 1, Jak 2, Stat 1or Stat 2 gene can encompass any portion of at least about 8 consecutive nucleotides of SEQ ID NO: 2, 4, 6, or 8. In one embodiment, the fragment can comprise at least about 10 nucleotides, at least about 15 nucleotides, at least about 20 nucleotides, or at least about 30 nucleotides of SEQ ID NO: 2, 4, 6, or 8. Fragments include all possible nucleotide lengths between about 8 and about 100 nucleotides, for example, lengths between about 15 and about 100 nucleotides, or between about 20 and about 100 nucleotides.

A Jak 1, Jak 2, Stat 1or Stat 2 fragment can be a fragment of a protein, such as Jak 1, Jak 2, Stat 1or Stat 2. For example, the Jak 1, Jak 2, Stat 1or Stat 2 fragment can encompass any portion of at least about 8 consecutive amino acids of SEQ ID NO: 1, 3, 5, or 7. The fragment can comprise at least about 10 consecutive amino acids, at least about 20 consecutive amino acids, at least about 30 consecutive amino acids, at least about 40 consecutive amino acids, a least about 50 consecutive amino acids, at least about 60 consecutive amino acids, at least about 70 consecutive amino acids, or at least about 75 consecutive amino acids of SEQ ID NO: 1, 3, 5, or 7. Fragments include all possible amino acid lengths between about 8 and 100 about amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids.

A synthetic peptide can be substantially purified via high performance liquid chromatography (HPLC). The composition of a synthetic polypeptide of Jak 1, Jak 2, Stat 1or Stat 2 can be confirmed by amino acid analysis or sequencing. Additionally, any portion of an amino acid sequence comprising a protein encoded by a Jak 1, Jak 2, Stat 1or Stat 2 gene can be altered during direct synthesis and/or combined using chemical methods with sequences from other proteins to produce a variant polypeptide or a fusion protein.

### Identifying Jak 1, Jak 2, Stat 1or Stat 2 Modulating Compounds

The disclosure relates to methods for identifying compounds which can be used for controlling and/or regulating hair growth (for example, hair density) or hair pigmentation in a subject. Since the disclosure has provided the identification of the genes listed herein as genes associated with a hair loss disorder, the disclosure also provides methods for identifiying compounds that modulate the expression or activity of a gene and/or protein of Jak 1, Jak 2, Stat 1or Stat 2. In addition, the disclosure provides methods for identifying compounds which can be used for the treatment of a hair loss disorder. The disclosure also relates to methods for identifying compounds which can be used for the treatment of hypotrichosis (for example, hereditary hypotrichosis simplex (HHS)). Non-limiting examples of hair loss disorders include: androgenetic alopecia, Alopecia areata, telogen effluvium, alopecia areata, alopecia totalis, and alopecia universalis. The methods can comprise the identification of test compounds or agents (e.g., peptides (such as antibodies or fragments thereof), small molecules, nucleic acids (such as siRNA or antisense RNA), or other agents) that can bind to a polypeptide molecule encoded by a Jak 1, Jak 2, Stat 1or Stat 2 gene and/or have a stimulatory or inhibitory effect on the biological activity of a protein encoded by a Jak 1, Jak 2, Stat 1or Stat 2 gene or its expression, and subsequently determining whether these compounds can regulate hair growth in a subject or can have an effect on symptoms associated with the hair loss disorders in an *in vivo* assay (i.e., examining an increase or reduction in hair growth).

As used herein, a "Jak 1, Jak 2, Stat 1or Stat 2 modulating compound" refers to a compound that interacts with a Jak 1, Jak 2, Stat 1or Stat 2 gene or a Jak 1, Jak 2, Stat 1or Stat 2 protein or polypeptide and modulates its activity and/or its expression. The compound can either increase the activity or expression of a protein encoded by a Jak 1, Jak 2, Stat 1or Stat 2 gene. Conversely, the compound can decrease the activity or expression of a protein encoded by a Jak 1, Jak 2, Stat 1or Stat 2 gene. The compound can be a Jak 1, Jak 2, Stat 1or Stat 2 agonist or a Jak 1, Jak 2, Stat 1or Stat 2 antagonist (e.g., a Jak1 inhibitor, a Jak2 inhibitor, a Stat1 inhibitor, or a Stat2 inhibitor). Some non-limiting examples of Jak 1, Jak 2, Stat 1or Stat 2 modulating compounds include peptides (such as peptide fragments comprising a polypeptide encoded by a Jak 1, Jak 2, Stat 1or Stat 2 gene, or antibodies or fragments thereof), small molecules, and nucleic acids (such as siRNA or antisense RNA specific for a nucleic acid comprising a Jak 1, Jak 2, Stat 1or Stat 2 gene). Agonists of a Jak 1, Jak 2, Stat 1or Stat 2 protein can be molecules which, when bound to a Jak 1, Jak 2, Stat 1or Stat 2 protein, increase or prolong the activity of the Jak 1, Jak 2, Stat 1or Stat 2 protein. Jak 1, Jak 2, Stat 1or Stat 2 agonists include, but are not limited to, proteins, nucleic acids, small molecules, or any other molecule which activates a Jak 1, Jak 2, Stat 1or Stat 2 protein. Antagonists of a Jak 1, Jak 2, Stat 1or Stat 2 protein can be molecules which, when bound to a Jak 1, Jak 2, Stat 1or Stat 2 protein decrease the amount or the duration of the activity of the Jak 1, Jak 2, Stat 1or Stat 2 protein. Antagonists include proteins, nucleic acids, antibodies, small molecules, or any other molecule which decrease the activity of a Jak 1, Jak 2, Stat 1or Stat 2 protein.

The term "modulate," as it appears herein, refers to a change in the activity or expression of a gene or protein of Jak 1, Jak 2, Stat 1or Stat 2. For example, modulation can cause an increase or a decrease in protein activity, binding characteristics, or any other biological, functional, or immunological properties of a Jak 1, Jak 2, Stat 1or Stat 2 protein.

In one embodiment, a Jak 1, Jak 2, Stat 1or Stat 2 modulating compound can be a peptide fragment of a Jak 1, Jak 2, Stat 1or Stat 2 protein that binds to the protein. For example, the Jak 1, Jak 2, Stat 1or Stat 2 polypeptide can encompass any portion of at least about 8 consecutive amino acids of SEQ ID NO: 1, 3, 5, or 7. The fragment can comprise at least about 10 consecutive amino acids, at least about 20 consecutive amino acids, at least about 30 consecutive amino acids, at least about 40 consecutive amino acids, at least about 50 consecutive amino acids, at least about 60 consecutive amino acids, or at least about 75 consecutive amino acids of SEQ ID NO: 1, 3, 5, or 7. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England). The Jak 1, Jak 2, Stat 1or Stat 2 peptide fragments can be isolated from a natural source, genetically engineered, or chemically prepared. These methods are well known in the art.

A Jak 1, Jak 2, Stat 1or Stat 2 modulating compound can be a protein, such as an antibody (monoclonal, polyclonal, humanized, chimeric, or fully human), or a binding fragment thereof, directed against a polypeptide encoded by a Jak 1, Jak 2, Stat 1or Stat 2 gene. An antibody fragment can be a form of an antibody other than the full-length form and includes portions or components that exist within full-length antibodies, in addition to antibody fragments that have been engineered. Antibody fragments can include, but are not limited to, single chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see*, Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (Janeway et al., (2001) Immunobiology, 5th ed., Garland Publishing). In one embodiment, an antibody or binding fragment thereof is directed against SEQ ID NO: 1, 3, 5 or 7. Antibodies can be obtained commercially, custom generated, or synthesized against an antigen of interest according to methods established in the art (Janeway et al., (2001) Immunobiology, 5th ed., Garland Publishing). For example, antibodies directed to Jak1, Jak2, Stat1, or Stat2 can be obtained commercially from Abcam, Santa Cruz Biotechnology, Abnova Corp., BD Biosciences, Antigenix America Inc., etc. Human antibodies directed to either Jak1, Jak2, Stat1, or Stat2 (such as monoclonal, humanized, or chimeric antibodies) can be useful antibody therapeutics for use in humans.

Inhibition of RNA encoding a polypeptide encoded by a Jak 1, Jak 2, Stat 1or Stat 2 gene can effectively modulate the expression of a Jak 1, Jak 2, Stat 1or Stat 2 gene from which the RNA is transcribed. Inhibitors are selected from the group comprising: siRNA; interfering RNA or RNAi; dsRNA; RNA Polymerase III transcribed DNAs; ribozymes; and antisense nucleic acids, which can be RNA, DNA, or an artificial nucleic acid.

Antisense oligonucleotides, including antisense DNA, RNA, and DNA/RNA molecules, act to directly block the translation of mRNA by binding to targeted mRNA and preventing protein translation. For example, antisense oligonucleotides of at least about 15 bases and complementary to unique regions of the DNA sequence encoding a polypeptide encoded by a Jak 1, Jak 2, Stat 1or Stat 2 gene can be synthesized, e.g., by conventional phosphodiester techniques (Dallas et al., (2006) Med. Sci. Monit. 12(4):RA67-74; Kalota et al., (2006) Handb. Exp. Pharmacol. 173:173-96; Lutzelburger et al., (2006) Handb. Exp. Pharmacol. 173:243-59). Antisense nucleotide sequences include, but are not limited to: morpholinos, 2'-O-methyl polynucleotides, DNA, RNA and the like.

siRNA comprises a double stranded structure containing from about 15 to about 50 base pairs, for example from about 21 to about 25 base pairs, and having a nucleotide sequence identical or nearly identical to an expressed target gene or RNA within the cell. The siRNA comprise a sense RNA strand and a complementary antisense RNA strand annealed together by standard Watson-Crick base-pairing interactions. The sense strand comprises a nucleic acid sequence which is substantially identical to a nucleic acid sequence contained within the target miRNA molecule. "Substantially identical" to a target sequence contained within the target mRNA refers to a nucleic acid sequence that differs from the target sequence by about 3% or less. The sense and antisense strands of the siRNA can comprise two complementary, single-stranded RNA molecules, or can comprise a single molecule in which two complementary portions are base-paired and are covalently linked by a single-stranded "hairpin" area. See also, McMnaus and Sharp (2002) Nat Rev Genetics, 3:737-47, and Sen and Blau (2006) FASEB J., 20:1293-99.

The siRNA can be altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, or modifications that make the siRNA resistant to nuclease digestion, or the substitution of one or more nucleotides in the siRNA with deoxyribo-nucleotides. One or both strands of the siRNA can also comprise a 3' overhang. As used herein, a 3' overhang refers to at least one unpaired nucleotide extending from the 3'-end of a duplexed RNA strand. For example, the siRNA can comprise at least one 3' overhang of from 1 to about 6 nucleotides (which includes ribonucleotides or deoxyribonucleotides) in length, or from 1 to about 5 nucleotides in length, or from 1 to about 4 nucleotides in length, or from about 2 to about 4 nucleotides in length. For example, each strand of the siRNA can comprise 3' overhangs of dithymidylic acid ("TT") or diuridylic acid ("uu").

siRNA can be produced chemically or biologically, or can be expressed from a recombinant plasmid or viral vector (for example, see U.S. Patent No. 7,294,504 and U.S. Patent No. 7,422,896). Exemplary methods for producing and testing dsRNA or siRNA molecules are described in U.S. Patent Application Publication No. 2002/0173478 to Gewirtz, U.S. Patent Application Publication No. 2007/0072204 to Hannon et al., and in U.S. Patent Application Publication No.2004/0018176 to Reich et al.

In one embodiment, an siRNA directed to a human nucleic acid sequence comprising a Jak 1, Jak 2, Stat 1or Stat 2 gene can be generated against any one of SEQ ID NOS: 2, 4, 6 or 8. In another embodiment, an siRNA directed to a human nucleic acid sequence comprising a Jak1 gene can comprise any one of the sequences listed in Table 11. In another embodiment, an siRNA directed to a human nucleic acid sequence comprising a Stat1 gene can comprise any one of the sequences listed in Table 12.

In another embodiment, the siRNA directed to Jak1 is listed in Table 11.

| Table 11. | | |
|---|---|---|
| | siRNA SEQUENCES for Jak1 | |
| | CCACATAGCTGATCTGAAA CGGGAAGCCTTAAGGAATA CTGAAGAGAAGAAGATAAA ACAAGAAGGATGAGGAGAA CCAAGAAGACTGAGGTGAA CAGAATACGCCATCAATAA GCACAGAAGACGGAGGAAA GCGATATATTCCAGAAACA TGAGCTACTTGGAGGATAA GAGTGAACACCAAGTGAAA CAAGAAGGATGAGGAGAAA CGGGAAGAGTGGAACAATT GCATTAACAAGCAGGACAA TTTGGAAAGTAGAGAAGAA GGAAAGTAGAGAAGAAAAT GGAACCACGCTCTGGGAAA TGATTGAGAAAGAGAGATT ACATTACGGTGCTGAAATA GGGAAGAGTGGAACAATTT AGAATATCATGGTGGAAGA GGAAATGTGTGTACTAAAA ATGAGAACATTGTGAAGTA CACAGAAGACGGAGGAAAT GCTGGTGGCTACTAAGAAA | TCTGAAGAGAAGAAGATAA GCAAGAATGCATTGAACGA AAGAAAGATTGATGGACTT AAAATAAACTGAAGCGGAA ATGAGGAAATGCTGGGAAT AAAGAAAGATTGATGGACT GGACTTAGCCCTCAAATTT ACACTGGACAGCTGAATAA CTGAATAAATGCAGTATCT AAGAATGCATTGAACGAAT CCAGTAACTTAGTGACACA TTAACAAGCAGGACAACAA GGGAGCAGGTGGCTGTTAA AAATCGAGATCTTAAGGAA GGAAATGGTATTAAGCTCA CCAGAATGTTTAATGCAAT GGACAAGCTTTCAGAACCT ACACATAATGACAACCAAA GCTTGGAGGTAGCTGGGTA CAGCAGAGGAACTGTGCAT CCAGAAACATTGAATAAGT CTGAAATATTTGAGACTTC GTACAGAATACGCCATCAA GCACCGACTTTGACAACAT GAGAAGAAGATAAAAGTGA GGAAATTCAAAGTTGCCAA |

In another embodiment, the siRNA directed to Stat1 is listed in Table 12.

| Table 12. | | |
|---|---|---|
| | siRNA SEQUENCES for Stat1 | |
| | AGAAAGAGCTTGACAGTAA AGGAATTGGAACAGAAATA CAGCATAACATAAGGAAAA TCAGAAATGTGAAGGACAA GGCAAAGAGTGATCAGAAA GCACAGTGATGTTAGACAA AGTCATGGCTGCTGAGAAT GTATAGAGCATGAAATCAA GGTTATGTGTATAGAGCAT GAAAGGAAGTAGTTCACAA GATGTGAATGAGAGAAATA ACAGAGAACACGAGACCAA AAGATGTGAATGAGAGAAA AGGACAAGGTTATGTGTAT CCTGATTAATGATGAACTA ACACAAAAGTGATGAACAT GCAATTGAAAGAACAGAAA CGAGAGCTGTCTAGGTTAA GAACATGACCCTATCACAA AGACAAACAGAAAGAGCTT GGACAAGGTTATGTGTATA TGACAATAAGAGAAAGGAA GGAAGTAGTTCACAAAATA | TGAAATTGCAAGAGCTGAA AGAAATACACCTACGAACA CCCTAAAGGAACTGGATAT GGAGGAATTGGAACAGAAA CCTAAAGGAACTGGATATA AGCGTAATCTTCAGGATAA TCTGAAGGAAGAAAGGAAA GGAAGATTTACAAGATGAA TGGCAAAGAGTGATCAGAA AGAGAAAGGAAGTAGTTCA TAATAGAGTTGCTGAATGT TGGAGGAATTGGAACAGAA AGTTGAGACTGTTGGTGAA GATAAAGATGTGAATGAGA CATCGTTACTGAAGAGCTT TGAAGTATCTGTATCCAAA GCACAAGGTGGCAGGATGT TGTCACAGCTGGATGATCA GAAAGAGCTTGACAGTAAA TCAAGAGCCTGGAAGATTT TGGAAGATTTACAAGATGA ATGAACTAGTGGAGTGGAA TGAGACTGTTGGTGAAATT TTGCAAGAGCTGAATTATA GGATTTAGGAAGTTCAACA GGAACTTGATGGCCCTAAA GGAACTGGATATATCAAGA |

RNA polymerase III transcribed DNAs contain promoters, such as the U6 promoter. These DNAs can be transcribed to produce small hairpin RNAs in the cell that can function as siRNA or linear RNAs that can function as antisense RNA. The Jak 1, Jak 2, Stat 1or Stat 2 modulating compound can contain ribonucleotides, deoxyribonucleotides, synthetic nucleotides, or any suitable combination such that the target RNA and/or gene is inhibited. In addition, these forms of nucleic acid can be single, double, triple, or quadruple stranded, (see for example Bass (2001) Nature, 411, 428 429; Elbashir et al., (2001) Nature, 411, 494 498; and PCT Publication Nos. WO 00/44895, WO 01/36646, WO 99/32619, WO 00/01846, WO 01/29058, WO 99/07409, WO 00/44914).

A Jak 1, Jak 2, Stat 1or Stat 2 modulating compound can be a small molecule that binds to a Jak 1, Jak 2, Stat 1or Stat 2 protein and disrupts its function, or conversely, enhances its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that modulate a Jak 1, Jak 2, Stat 1or Stat 2 protein can be identified via *in silico* screening or high-throughput (HTP) screening of combinatorial libraries. Most conventional pharmaceuticals, such as aspirin, penicillin, and many chemotherapeutics, are small molecules, can be obtained commercially, can be chemically synthesized, or can be obtained from random or combinatorial libraries as described below (Werner et al., (2006) Brief Funct. Genomic Proteomic 5(1):32-6).

Non-limiting examples of Jak1/Jak2 inhibitors include: AG490 (Caceres-Cortes, Anticancer Agents Med Chem. 2008 Oct;8(7):717-22); CYT387 (Pardanani et al., Leukemia. 2009 Aug;23(8):1441-5; Monaghan et al., Leukemia. 2011 Jul 26. doi: 10.1038/leu.2011.175. [Epub ahead of print]); SB1518 (William et al., J Med Chem. 2011 Jul 14;54(13):4638-58; Hart et al., Leukemia. 2011 Jun 21. doi: 10.1038/leu.2011.148. [Epub ahead of print]); LY3009104 (INCB28050) (Incyte and Lilly); TG101348 (Wernig et al., Cancer Cell. 2008 Apr;13(4):311-20; Pardanani et al., J Clin Oncol. 2011 Mar 1;29(7):789-96); and BMS-911543 (Purandare et al., Leukemia. 2011 Oct 21. doi: 10.1038/leu.2011.292. [Epub ahead of print]).

JAK1/2 inhibitors in clinical development include a) INCB018424, topical and oral; 5nM activity (Incyte); b) CEP-701 (Cephalon); and c) TG101348.
α) 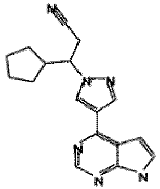
b) 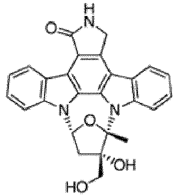
c)

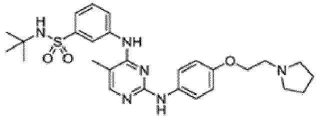

Non-limiting examples of Stat inhibitors include: WP-1034 (Faderl et al., Anticancer Res. 2005 May-Jun;25(3B):1841-50), fludarabine (Fludara, Berlex, CA), epigallocatechin-3-gallate (EGCG), and Hyperforin. Other compounds directed to Jak/Stat signaling are described in Ivanenkov et al., Mini Rev Med Chem. 2011 Jan;11(1):55-78.

Knowledge of the primary sequence of a molecule of interest, such as a polypeptide encoded by a Jak 1, Jak 2, Stat 1or Stat 2 gene, and the similarity of that sequence with proteins of known function, can provide information as to the inhibitors or antagonists of the protein of interest in addition to agonists. Identification and screening of agonists and antagonists is further facilitated by determining structural features of the protein, e.g., using X-ray crystallography, neutron diffraction, nuclear magnetic resonance spectrometry, and other techniques for structure determination. These techniques provide for the rational design or identification of agonists and antagonists.

Test compounds, such as Jak 1, Jak 2, Stat 1or Stat 2 modulating compounds, can be screened from large libraries of synthetic or natural compounds (see Wang et al., (2007) Curr Med Chem, 14(2):133-55; Mannhold (2006) Curr Top Med Chem, 6 (10):1031-47; and Hensen (2006) Curr Med Chem 13(4):361-76). Numerous means are currently used for random and directed synthesis of saccharide, peptide, and nucleic acid based compounds. Synthetic compound libraries are commercially available from Maybridge Chemical Co. (Trevillet, Cornwall, UK), AMRI (Albany, NY), ChemBridge (San Diego, CA), and MicroSource (Gaylordsville, CT). A rare chemical library is available from Aldrich (Milwaukee, Wis.). Alternatively, libraries of natural compounds in the form of bacterial, fungal, plant and animal extracts are available from e.g. Pan Laboratories (Bothell, Wash.) or MycoSearch (N.C.), or are readily producible. Additionally, natural and synthetically produced libraries and compounds are readily modified through conventional chemical, physical, and biochemical means (Blondelle et al., (1996) Tib Tech 14:60).

Methods for preparing libraries of molecules are well known in the art and many libraries are commercially available. Libraries of interest in the disclosure include peptide libraries, randomized oligonucleotide libraries, synthetic organic combinatorial libraries, and the like. Degenerate peptide libraries can be readily prepared in solution, in immobilized form as bacterial flagella peptide display libraries or as phage display libraries. Peptide ligands can be selected from combinatorial libraries of peptides containing at least one amino acid. Libraries can be synthesized of peptoids and non-peptide synthetic moieties. Such libraries can further be synthesized which contain non-peptide synthetic moieties, which are less subject to enzymatic degradation compared to their naturally-occurring counterparts. For example, libraries can also include, but are not limited to, peptide-on-plasmid libraries, synthetic small molecule libraries, aptamer libraries, in vitro translation-based libraries, polysome libraries, synthetic peptide libraries, neurotransmitter libraries, and chemical libraries.

Examples of chemically synthesized libraries are described in Fodor et al., (1991) Science 251:767-773; Houghten et al., (1991) Nature 354:84-86; Lam et al., (1991) Nature 354:82-84; Medynski, (1994) BioTechnology 12:709-710; Gallop et al., (1994) J. Medicinal Chemistry 37(9):1233-1251; Ohlmeyer et al., (1993) Proc. Natl. Acad. Sci. USA 90:10922-10926; Erb et al., (1994) Proc. Natl. Acad. Sci. USA 91:11422-11426; Houghten et al., (1992) Biotechniques 13:412; Jayawickreme et al., (1994) Proc. Natl. Acad. Sci. USA 91:1614-1618; Salmon et al., (1993) Proc. Natl. Acad. Sci. USA 90:11708-11712; PCT Publication No. WO 93/20242, dated Oct. 14, 1993; and Brenner et al., (1992) Proc. Natl. Acad. Sci. USA 89:5381-5383.

Examples of phage display libraries are described in Scott et al., (1990) Science 249:386-390; Devlin et al., (1990) Science, 249:404-406; Christian, et al., (1992) J. Mol. Biol. 227:711-718; Lenstra, (1992) J. Immunol. Meth. 152:149-157; Kay et al., (1993) Gene 128:59-65; and PCT Publication No. WO 94/18318.

*In vitro* translation-based libraries include but are not limited to those described in PCT Publication No. WO 91/05058; and Mattheakis et al., (1994) Proc. Natl. Acad. Sci. USA 91:9022-9026.

As used herein, the term "ligand source" can be any compound library described herein, or tissue extract prepared from various organs in an organism's system, that can be used to screen for compounds that would act as an agonist or antagonist of a Jak 1, Jak 2, Stat 1or Stat 2 protein. Screening compound libraries listed herein [also see U.S. Patent Application Publication No. 2005/0009163, in combination with *in vivo* animal studies, functional and signaling assays described below can be used to identify Jak 1, Jak 2, Stat 1or Stat 2 modulating compounds that regulate hair growth or treat hair loss disorders.

Screening the libraries can be accomplished by any variety of commonly known methods. See, for example, the following references, which disclose screening of peptide libraries: Parmley and Smith, (1989) Adv. Exp. Med. Biol. 251:215-218; Scott and Smith, (1990) Science 249:386-390; Fowlkes et al., (1992) BioTechniques 13:422-427; Oldenburg et al., (1992) Proc. Natl. Acad. Sci. USA 89:5393-5397; Yu et al., (1994) Cell 76:933-945; Staudt et al., (1988) Science 241:577-580; Bock et al., (1992) Nature 355:564-566; Tuerk et al., (1992) Proc. Natl. Acad. Sci. USA 89:6988-6992; Ellington et al., (1992) Nature 355:850-852; U.S. Patent Nos. 5,096,815; 5,223,409; and 5,198,346, all to Ladner et al.; Rebar et al., (1993) Science 263:671-673; and PCT Pub. WO 94/18318.

Small molecule combinatorial libraries can also be generated and screened. A combinatorial library of small organic compounds is a collection of closely related analogs that differ from each other in one or more points of diversity and are synthesized by organic techniques using multi-step processes. Combinatorial libraries include a vast number of small organic compounds. One type of combinatorial library is prepared by means of parallel synthesis methods to produce a compound array. A compound array can be a collection of compounds identifiable by their spatial addresses in Cartesian coordinates and arranged such that each compound has a common molecular core and one or more variable structural diversity elements. The compounds in such a compound array are produced in parallel in separate reaction vessels, with each compound identified and tracked by its spatial address. Examples of parallel synthesis mixtures and parallel synthesis methods are provided in U.S. Ser. No. 08/177,497, filed Jan. 5, 1994 and its corresponding PCT published patent application WO95/18972, published Jul. 13, 1995 and U.S. Pat. No. 5,712,171 granted Jan. 27, 1998 and its corresponding PCT published patent application WO96/22529.

In one non-limiting example, non-peptide libraries, such as a benzodiazepine library (see e.g., Bunin et al., (1994) Proc. Natl. Acad. Sci. USA 91:4708-4712), can be screened. Peptoid libraries, such as that described by Simon et al., (1992) Proc. Natl. Acad. Sci. USA 89:9367-9371, can also be used. Another example of a library that can be used, in which the amide functionalities in peptides have been permethylated to generate a chemically transformed combinatorial library, is described by Ostresh et al. (1994), Proc. Natl. Acad. Sci. USA 91:11138-11142.

Computer modeling and searching technologies permit the identification of compounds, or the improvement of already identified compounds, that can modulate the expression or activity of a Jak 1, Jak 2, Stat 1or Stat 2 protein. Having identified such a compound or composition, the active sites or regions of a Jak 1, Jak 2, Stat 1or Stat 2 protein can be subsequently identified via examining the sites to which the compounds bind. These sites can be ligand binding sites and can be identified using methods known in the art including, for example, from the amino acid sequences of peptides, from the nucleotide sequences of nucleic acids, or from study of complexes of the relevant compound or composition with its natural ligand. In the latter case, chemical or X-ray crystallographic methods can be used to find the active site by finding where on the factor the complexed ligand is found.

The three dimensional geometric structure of a site, for example that of a polypeptide encoded by a Jak 1, Jak 2, Stat 1 or Stat 2 gene, can be determined by known methods in the art, such as X-ray crystallography, which can determine a complete molecular structure. Solid or liquid phase NMR can be used to determine certain intramolecular distances. Any other experimental method of structure determination can be used to obtain partial or complete geometric structures. The geometric structures can be measured with a complexed ligand, natural or artificial, which can increase the accuracy of the active site structure determined.

Other methods for preparing or identifying peptides that bind to a target are known in the art. Molecular imprinting, for instance, can be used for the de novo construction of macromolecular structures such as peptides that bind to a molecule. See, for example, Kenneth J. Shea, Molecular Imprinting of Synthetic Network Polymers: The De Novo synthesis of Macromolecular Binding and Catalytic Sites, TRIP Vol. 2, No. 5, May 1994; Mosbach, (1994) Trends in Biochem. Sci., 19(9); and Wulff, G., in Polymeric Reagents and Catalysts (Ford, W. T., Ed.) ACS Symposium Series No. 308, pp 186-230, American Chemical Society (1986). One method for preparing mimics of a Jak 1, Jak 2, Stat 1 or Stat 2 modulating compound involves the steps of: (i) polymerization of functional monomers around a known substrate (the template) that exhibits a desired activity; (ii) removal of the template molecule; and then (iii) polymerization of a second class of monomers in, the void left by the template, to provide a new molecule which exhibits one or more desired properties which are similar to that of the template. In addition to preparing peptides in this manner other binding molecules such as polysaccharides, nucleosides, drugs, nucleoproteins, lipoproteins, carbohydrates, glycoproteins, steroids, lipids, and other biologically active materials can also be prepared. This method is useful for designing a wide variety of biological mimics that are more stable than their natural counterparts, because they are prepared by the free radical polymerization of functional monomers, resulting in a compound with a nonbiodegradable backbone. Other methods for designing such molecules include for example drug design based on structure activity relationships, which require the synthesis and evaluation of a number of compounds and molecular modeling.

### Screening Assays

Jak 1, Jak 2, Stat 1 or Stat 2 Modulating Compounds. A Jak 1, Jak 2, Stat 1 or Stat 2 modulating compound can be a compound that affects the activity and/or expression of a Jak 1, Jak 2, Stat 1 or Stat 2 protein *in vivo* and/or *in vitro.* Jak 1, Jak 2, Stat 1 or Stat 2 modulating compounds can be agonists and antagonists of a Jak 1, Jak 2, Stat 1 or Stat 2 protein, and can be compounds that exert their effect on the activity of a Jak 1, Jak 2, Stat 1 or Stat 2 protein via the expression, via post-translational modifications, or by other means.

Test compounds or agents which bind to a Jak 1, Jak 2, Stat 1 or Stat 2 protein, and/or have a stimulatory or inhibitory effect on the activity or the expression of a Jak 1, Jak 2, Stat 1 or Stat 2 protein, can be identified by two types of assays: (a) cell-based assays which utilize cells expressing a Jak 1, Jak 2, Stat 1 or Stat 2 protein or a variant thereof on the cell surface; or (b) cell-free assays, which can make use of isolated Jak 1, Jak 2, Stat 1 or Stat 2 proteins. These assays can employ a biologically active fragment of a Jak 1, Jak 2, Stat 1 or Stat 2 protein, full-length proteins, or a fusion protein which includes all or a portion of a polypeptide encoded by a Jak 1, Jak 2, Stat 1 or Stat 2 gene. A Jak 1, Jak 2, Stat 1 or Stat 2 protein can be obtained from any suitable mammalian species (e.g., human, rat, chick, xenopus, equine, bovine or murine). The assay can be a binding assay comprising direct or indirect measurement of the binding of a test compound. The assay can also be an activity assay comprising direct or indirect measurement of the activity of a Jak 1, Jak 2, Stat 1 or Stat 2 protein. The assay can also be an expression assay comprising direct or indirect measurement of the expression of Jak 1, Jak 2, Stat 1 or Stat 2 mRNA nucleic acid sequences or a protein encoded by a Jak 1, Jak 2, Stat 1 or Stat 2 gene. The various screening assays can be combined with an *in vivo* assay comprising measuring the effect of the test compound on the symptoms of a hair loss disorder or disease in a subject (for example, androgenetic alopecia, alopecia areata, alopecia totalis, or alopecia universalis), loss of hair pigmentation in a subject, or even hypotrichosis.

An *in vivo* assay can also comprise assessing the effect of a test compound on regulating hair growth in known mammalian models that display defective or aberrant hair growth phenotypes or mammals that contain mutations in the open reading frame (ORF) of nucleic acid sequences comprising a Jak 1, Jak 2, Stat 1 or Stat 2 gene that affects hair growth regulation or hair density, or hair pigmentation. In one embodiment, controlling hair growth can comprise an induction of hair growth or density in the subject. Here, the compound's effect in regulating hair growth can be observed either visually via examining the organism's physical hair growth or loss, or by assessing protein or mRNA expression using methods known in the art.

Assays for screening test compounds that bind to or modulate the activity of a Jak 1, Jak 2, Stat 1 or Stat 2 protein can also be carried out. The test compound can be obtained by any suitable means, such as from conventional compound libraries. Determining the ability of the test compound to bind to a membrane-bound form of the Jak 1, Jak 2, Stat 1 or Stat 2 protein can be accomplished via coupling the test compound with a radioisotope or enzymatic label such that binding of the test compound to the cell expressing a Jak 1, Jak 2, Stat 1 or Stat 2 protein can be measured by detecting the labeled compound in a complex. For example, the test compound can be labeled with ³H, ¹⁴C, ³⁵S, or ¹²⁵I, either directly or indirectly, and the radioisotope can be subsequently detected by direct counting of radioemmission or by scintillation counting. Alternatively, the test compound can be enzymatically labeled with, for example, horseradish peroxidase, alkaline phosphatase, or luciferase, and the enzymatic label detected by determination of conversion of an appropriate substrate to product.

Cell-based assays can comprise contacting a cell expressing Jak 1, Jak 2, Stat 1 or Stat 2 with a test agent and determining the ability of the test agent to modulate (such as increase or decrease) the activity or the expression of the membrane-bound molecule. Determining the ability of the test agent to modulate the activity of the membrane-bound Jak 1, Jak 2, Stat 1 or Stat 2 molecule can be accomplished by any method suitable for measuring the activity of such a molecule, such as monitoring downstream signaling events (e.g., You et al., Ann N Y Acad Sci. 2008 Dec;1150:300-10; Posadas et al., Expert Rev Clin Immunol. 2009 Jan;5(1):9-17; Korhonen et al., Basic Clin Pharmacol Toxicol. 2009 Apr;104(4):276-84; Vital et al., Ther Clin Risk Manag. 2006 Dec;2(4):365-75; Malek and Castro, Immunity. 2010 Aug 27;33(2):153-65; Cheng et al., Immunol Rev. 2011 May;241(1):63-76; Lanier, Nat Immunol. 2008 May;9(5):495-502; Lowell, Cold Spring Harb Perspect Biol. 2011 Mar 1;3(3). pii: a002352; Mócsai et al., Nat Rev Immunol. 2010 Jun;10(6):387-402; Bradshaw, Cell Signal. 2010 Aug;22(8):1175-84; Ivanenkov et al., Mini Rev Med Chem. 2011 Jan;11(1):55-78; Himpe et al., Biofactors. 2009 Jan-Feb;35(1):76-81).

A Jak 1, Jak 2, Stat 1 or Stat 2 protein or the target of a Jak 1, Jak 2, Stat 1 or Stat 2 protein can be immobilized to facilitate the separation of complexed from uncomplexed forms of one or both of the proteins. Binding of a test compound to a Jak 1, Jak 2, Stat 1 or Stat 2 protein or a variant thereof, or interaction of a Jak 1, Jak 2, Stat 1 or Stat 2 protein with a target molecule in the presence and absence of a test compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix (for example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtiter plates).

A Jak 1, Jak 2, Stat 1 or Stat 2 protein, or a variant thereof, can also be immobilized via being bound to a solid support. Non-limiting examples of suitable solid supports include glass or plastic slides, tissue culture plates, microtiter wells, tubes, silicon chips, or particles such as beads (including, but not limited to, latex, polystyrene, or glass beads). Any method known in the art can be used to attach a polypeptide (or polynucleotide) corresponding to Jak 1, Jak 2, Stat 1 or Stat 2 or a variant thereof, or test compound to a solid support, including use of covalent and non-covalent linkages, or passive absorption.

The expression of a Jak 1, Jak 2, Stat 1 or Stat 2 protein can also be monitored. For example, regulators of the expression of a Jak 1, Jak 2, Stat 1 or Stat 2 protein can be identified via contacting a cell with a test compound and determining the expression of a protein encoded by a Jak 1, Jak 2, Stat 1 or Stat 2 gene or Jak 1, Jak 2, Stat 1 or Stat 2 mRNA nucleic acid sequences in the cell. The expression level of a protein encoded by a Jak 1, Jak 2, Stat 1 or Stat 2 gene or Jak 1, Jak 2, Stat 1 or Stat 2 mRNA nucleic acid sequences in the cell in the presence of the test compound is compared to the protein or mRNA expression level in the absence of the test compound. The test compound can then be identified as a regulator of the expression of a Jak 1, Jak 2, Stat 1 or Stat 2 protein based on this comparison. For example, when expression of a protein encoded by a Jak 1, Jak 2, Stat 1 or Stat 2 gene or Jak 1, Jak 2, Stat 1 or Stat 2 mRNA nucleic acid sequences in the cell is statistically or significantly greater in the presence of the test compound than in its absence, the test compound is identified as a stimulator/enhancer of expression of a protein encoded by a Jak 1, Jak 2, Stat 1 or Stat 2 gene or Jak 1, Jak 2, Stat 1 or Stat 2 mRNA nucleic acid sequences in the cell. The test compound can be said to be a Jak 1, Jak 2, Stat 1 or Stat 2 modulating compound (such as an agonist).

Alternatively, when expression of a protein encoded by a Jak 1, Jak 2, Stat 1 or Stat 2 gene or Jak 1, Jak 2, Stat 1 or Stat 2 mRNA nucleic acid sequences in the cell is statistically or significantly less in the presence of the test compound than in its absence, the compound is identified as an inhibitor of the expression of a protein encoded by a Jak 1, Jak 2, Stat 1 or Stat 2 gene or Jak 1, Jak 2, Stat 1 or Stat 2 mRNA nucleic acid sequences in the cell. The test compound can also be said to be a Jak 1, Jak 2, Stat 1 or Stat 2 modulating compound (such as an antagonist). The expression level of a protein encoded by a Jak 1, Jak 2, Stat 1 or Stat 2 gene or Jak 1, Jak 2, Stat 1 or Stat 2 mRNA nucleic acid sequences in the cell in cells can be determined by methods previously described.

For binding assays, the test compound can be a small molecule which binds to and occupies the binding site of a polypeptide encoded by a Jak 1, Jak 2, Stat 1 or Stat 2 gene, or a variant thereof. This can make the ligand binding site inaccessible to substrate such that normal biological activity is prevented. Examples of such small molecules include, but are not limited to, small peptides or peptide-like molecules. In binding assays, either the test compound or a polypeptide encoded by a Jak 1, Jak 2, Stat 1 or Stat 2 gene can comprise a detectable label, such as a fluorescent, radioisotopic, chemiluminescent, or enzymatic label (for example, alkaline phosphatase, horseradish peroxidase, or luciferase). Detection of a test compound which is bound to a polypeptide encoded by a Jak 1, Jak 2, Stat 1 or Stat 2 gene can then be determined via direct counting of radio emmission, by scintillation counting, or by determining conversion of an appropriate substrate to a detectable product.

Determining the ability of a test compound to bind to a Jak 1, Jak 2, Stat 1 or Stat 2 protein also can be accomplished using real-time Biamolecular Interaction Analysis (BIA) [McConnell et al., 1992, Science 257, 1906-1912; Sjolander, Urbaniczky, 1991, Anal. Chem. 63, 2338-2345]. BIA is a technology for studying biospecific interactions in real time, without labeling any of the interactants (for example, BIA-core™). Changes in the optical phenomenon surface plasmon resonance (SPR) can be used as an indication of real-time reactions between biological molecules.

To identify other proteins which bind to or interact with a Jak 1, Jak 2, Stat 1 or Stat 2 protein and modulate its activity, a polypeptide encoded by a Jak 1, Jak 2, Stat 1 or Stat 2 gene can be used as a bait protein in a two-hybrid assay or three-hybrid assay (Szabo et al., 1995 , Curr. Opin. Struct. Biol. 5, 699-705; U.S. Pat. No. 5,283,317), according to methods practiced in the art. The two-hybrid system is based on the modular nature of most transcription factors, which consist of separable DNA-binding and activation domains.

Functional Assays. Test compounds can be tested for the ability to increase or decrease the activity of a Jak 1, Jak 2, Stat 1 or Stat 2 protein, or a variant thereof. Activity can be measured after contacting a purified Jak 1, Jak 2, Stat 1 or Stat 2 protein, a cell membrane preparation, or an intact cell with a test compound. A test compound that decreases the activity of a Jak 1, Jak 2, Stat 1 or Stat 2 protein by at least about 10%, at least about 20%, at least about 30%, at least about 40%, at least about 50%, at least about 60%, at least about 70%, at least about 75%, at least about 80%, at least about 90%, at least about 95% or 100% is identified as a potential agent for decreasing the activity of a Jak 1, Jak 2, Stat 1 or Stat 2 protein, for example an antagonist. A test compound that increases the activity of a Jak 1, Jak 2, Stat 1 or Stat 2 protein by at least about 10%, at least about 20%, at least about 30%, at least about 40%, at least about 50%, at least about 60%, at least about 70%, at least about 75%, at least about 80%, at least about 90%, at least about 95% or 100% is identified as a potential agent for increasing the activity of a Jak 1, Jak 2, Stat 1 or Stat 2 protein, for example an agonist.

### Treatment and Prevention

The disclosure also relates to a method for treating or preventing a hair-loss disorder in a subject. In one embodiment, the method comprises detecting the presence of an alteration in a Jak 1, Jak 2, Stat 1 or Stat 2 gene in a sample from the subject, the presence of the alteration being indicative of a hair-loss disorder, or the predisposition to a hair-loss disorder, and, administering to the subject in need a therapeutic treatment against a hair-loss disorder. The therapeutic treatment can be a drug administration (for example, a pharmaceutical composition comprising a siRNA directed to a Jak 1, Jak 2, Stat 1 or Stat 2 nucleic acid). In one embodiment, the therapeutic molecule to be administered comprises a polypeptide encoded by a Jak 1, Jak 2, Stat 1 or Stat 2 gene, comprising at least about 75%, at least about 80%, at least about 85%, at least about 90%, at least about 93%, at least about 95%, at least about 97%, at least about 98%, at least about 99%, or 100% of the amino acid sequence of SEQ ID NO: 1, 3, 5, or 7, and exhibits the function of decreasing expression of a protein encoded by a Jak 1, Jak 2, Stat 1 or Stat 2 gene. This can restore the capacity to initiate hair growth in cells derived from hair follicles or skin. In another embodiment, the therapeutic molecule to be administered comprises a nucleic acid sequence comprising a Jak 1, Jak 2, Stat 1 or Stat 2 gene that encodes a polypeptide, comprising at least about 75%, at least about 80%, at least about 85%, at least about 90%, at least about 93%, at least about 95%, at least about 97%, at least about 98%, at least about 99%, or 100% of the nucleic acid sequence of SEQ ID NO: 2, 4, 6, or 8, and encodes a polypeptide with the function of decreasing expression of a protein encoded by a Jak 1, Jak 2, Stat 1 or Stat 2 gene, thus restoring the capacity to initiate hair growth in cells derived from hair follicle cells or skin.

The alteration can be determined at the level of the DNA, RNA, or polypeptide. Optionally, detection can be determined by performing an oligonucleotide ligation assay, a confirmation based assay, a hybridization assay, a sequencing assay, an allele-specific amplification assay, a microsequencing assay, a melting curve analysis, a denaturing high performance liquid chromatography (DHPLC) assay (for example, see Jones et al, (2000) Hum Genet., 106(6):663-8), or a combination thereof. In another embodiment, the detection is performed by sequencing all or part of a Jak 1, Jak 2, Stat 1 or Stat 2 gene or by selective hybridization or amplification of all or part of a Jak 1, Jak 2, Stat 1 or Stat 2 gene. A Jak 1, Jak 2, Stat 1 or Stat 2 gene specific amplification can be carried out before the alteration identification step.

An alteration in a chromosome region occupied by a Jak 1, Jak 2, Stat 1 or Stat 2 gene can be any form of mutation(s), deletion(s), rearrangement(s) and/or insertions in the coding and/or non-coding region of the locus, alone or in various combination(s). Mutations can include point mutations. Insertions can encompass the addition of one or several residues in a coding or non-coding portion of the gene locus. Insertions can comprise an addition of between 1 and 50 base pairs in the gene locus. Deletions can encompass any region of one, two or more residues in a coding or non-coding portion of the gene locus, such as from two residues up to the entire gene or locus. Deletions can affect smaller regions, such as domains (introns) or repeated sequences or fragments of less than about 50 consecutive base pairs, although larger deletions can occur as well. Rearrangement includes inversion of sequences. The alteration in a chromosome region occupied by a Jak 1, Jak 2, Stat 1 or Stat 2 gene can result in amino acid substitutions, RNA splicing or processing, product instability, the creation of stop codons, frame-shift mutations, and/or truncated polypeptide production. The alteration can result in the production of a polypeptide encoded by a Jak 1, Jak 2, Stat 1 or Stat 2 gene with altered function, stability, targeting or structure. The alteration can also cause a reduction, or even an increase in protein expression. In one embodiment, the alteration in the chromosome region occupied by a Jak 1, Jak 2, Stat 1 or Stat 2 gene can comprise a point mutation, a deletion, or an insertion in a Jak 1, Jak 2, Stat 1 or Stat 2 gene or corresponding expression product. In another embodiment, the alteration can be a deletion or partial deletion of a Jak 1, Jak 2, Stat 1 or Stat 2 gene. The alteration can be determined at the level of the DNA, RNA, or polypeptide.

In another embodiment, the method can comprise detecting the presence of altered RNA expression. Altered RNA expression includes the presence of an altered RNA sequence, the presence of an altered RNA splicing or processing, or the presence of an altered quantity of RNA. These can be detected by various techniques known in the art, including sequencing all or part of the RNA or by selective hybridization or selective amplification of all or part of the RNA. In a further embodiment, the method can comprise detecting the presence of altered expression of a polypeptide encoded by a Jak 1, Jak 2, Stat 1 or Stat 2 gene. Altered polypeptide expression includes the presence of an altered polypeptide sequence, the presence of an altered quantity of polypeptide, or the presence of an altered tissue distribution. These can be detected by various techniques known in the art, including by sequencing and/or binding to specific ligands (such as antibodies).

Various techniques known in the art can be used to detect or quantify altered gene or RNA expression or nucleic acid sequences, which include, but are not limited to, hybridization, sequencing, amplification, and/or binding to specific ligands (such as antibodies). Other suitable methods include allele-specific oligonucleotide (ASO), oligonucleotide ligation, allele-specific amplification, Southern blot (for DNAs), Northern blot (for RNAs), single-stranded conformation analysis (SSCA), PFGE, fluorescent in situ hybridization (FISH), gel migration, clamped denaturing gel electrophoresis, denaturing HLPC, melting curve analysis, heteroduplex analysis, RNase protection, chemical or enzymatic mismatch cleavage, ELISA, radio-immunoassays (RIA) and immuno-enzymatic assays (IEMA). Some of these approaches (such as SSCA and CGGE) are based on a change in electrophoretic mobility of the nucleic acids, as a result of the presence of an altered sequence. According to these techniques, the altered sequence is visualized by a shift in mobility on gels. The fragments can then be sequenced to confirm the alteration. Some other approaches are based on specific hybridization between nucleic acids from the subject and a probe specific for wild type or altered gene or RNA. The probe can be in suspension or immobilized on a substrate. The probe can be labeled to facilitate detection of hybrids. Some of these approaches are suited for assessing a polypeptide sequence or expression level, such as Northern blot, ELISA and RIA. These latter require the use of a ligand specific for the polypeptide, for example, the use of a specific antibody.

Sequencing. Sequencing can be carried out using techniques well known in the art, using automatic sequencers. The sequencing can be performed on the complete Jak 1, Jak 2, Stat 1 or Stat 2 gene or on specific domains thereof, such as those known or suspected to carry deleterious mutations or other alterations.

Amplification. Amplification is based on the formation of specific hybrids between complementary nucleic acid sequences that serve to initiate nucleic acid reproduction. Amplification can be performed according to various techniques known in the art, such as by polymerase chain reaction (PCR), ligase chain reaction (LCR), strand displacement amplification (SDA) and nucleic acid sequence based amplification (NASBA). These techniques can be performed using commercially available reagents and protocols. Useful techniques in the art encompass real-time PCR, allele-specific PCR, or PCR-SSCP. Amplification usually requires the use of specific nucleic acid primers, to initiate the reaction. Nucleic acid primers useful for amplifying sequences from a Jak 1, Jak 2, Stat 1 or Stat 2 gene or locus are able to specifically hybridize with a portion of a Jak 1, Jak 2, Stat 1 or Stat 2 gene locus that flank a target region of the locus, wherein the target region is altered in certain subjects having a hair-loss disorder. In one embodiment, amplification can comprise using forward and reverse PCR primers comprising nucleotide sequences of SEQ ID Nos. 2, 4, 6 or 8.

The disclosure provides for a nucleic acid primer, wherein the primer can be complementary to and hybridize specifically to a portion of a Jak 1, Jak 2, Stat 1 or Stat 2 coding sequence (e.g., gene or RNA) altered in certain subjects having a hair-loss disorder. Primers can be specific for altered sequences in a Jak 1, Jak 2, Stat 1 or Stat 2 gene or RNA. By using such primers, the detection of an amplification product indicates the presence of an alteration in a Jak 1, Jak 2, Stat 1 or Stat 2 gene or the absence of such gene. Primers can also be used to identify single nucleotide polymorphisms (SNPs) located in or around a Jak 1, Jak 2, Stat 1 or Stat 2 gene locus; SNPs can comprise a single nucleotide change, or a cluster of SNPs in and around a Jak 1, Jak 2, Stat 1 or Stat 2 gene. Examples of primers can be single-stranded nucleic acid molecules of about 5 to 60 nucleotides in length, or about 8 to about 25 nucleotides in length. The sequence can be derived directly from the sequence of a Jak 1, Jak 2, Stat 1 or Stat 2 gene. Perfect complementarity is useful to ensure high specificity; however, certain mismatch can be tolerated. For example, a nucleic acid primer or a pair of nucleic acid primers as described above can be used in a method for detecting the presence of or a predisposition to a hair-loss disorder in a subject.

Amplification methods include, e.g., polymerase chain reaction, PCR (PCR PROTOCOLS, A GUIDE TO METHODS AND APPLICATIONS, ed. Innis, Academic Press, N.Y., 1990 and PCR STRATEGIES, 1995, ed. Innis, Academic Press, Inc., N.Y., ligase chain reaction (LCR) (see, e.g., Wu, Genomics 4:560, 1989; Landegren, Science 241:1077, 1988; Barringer, Gene 89:117, 1990); transcription amplification (see, e.g., Kwoh, Proc. Natl. Acad. Sci. USA 86:1173, 1989); and, self-sustained sequence replication (see, e.g., Guatelli, Proc. Natl. Acad. Sci. USA 87:1874, 1990); Q Beta replicase amplification (see, e.g., Smith, J. Clin. Microbiol. 35:1477-1491, 1997), automated Q-beta replicase amplification assay (see, e.g., Burg, Mol. Cell. Probes 10:257-271, 1996) and other RNA polymerase mediated techniques (e.g., NASBA, Cangene, Mississauga, Ontario); see also Berger, Methods Enzymol. 152:307-316, 1987; Sambrook; Ausubel; U.S. Pat. Nos. 4,683,195 and 4,683,202; Sooknanan, Biotechnology 13:563-564, 1995.

Selective Hybridization. Hybridization detection methods are based on the formation of specific hybrids between complementary nucleic acid sequences that serve to detect nucleic acid sequence alteration(s). A detection technique involves the use of a nucleic acid probe specific for wild type or altered gene or RNA, followed by the detection of the presence of a hybrid. The probe can be in suspension or immobilized on a substrate or support (for example, as in nucleic acid array or chips technologies). The probe can be labeled to facilitate detection of hybrids. For example, a sample from the subject can be contacted with a nucleic acid probe specific for a wild type Jak 1, Jak 2, Stat 1 or Stat 2 gene or an altered Jak 1, Jak 2, Stat 1 or Stat 2 gene, and the formation of a hybrid can be subsequently assessed. In one embodiment, the method comprises contacting simultaneously the sample with a set of probes that are specific, respectively, for a wild type Jak 1, Jak 2, Stat 1 or Stat 2 gene and for various altered forms thereof. Thus, it is possible to detect directly the presence of various forms of alterations in a Jak 1, Jak 2, Stat 1 or Stat 2 gene in the sample. Also, various samples from various subjects can be treated in parallel.

A probe can be a polynucleotide sequence which is complementary to and can specifically hybridize with a (target portion of a) Jak 1, Jak 2, Stat 1 or Stat 2 gene or RNA, and that is suitable for detecting polynucleotide polymorphisms associated with alleles of a Jak 1, Jak 2, Stat 1 or Stat 2 gene (or genes) which predispose to or are associated with a hair-loss disorder. Useful probes are those that are complementary to a Jak 1, Jak 2, Stat 1 or Stat 2 gene, RNA, or target portion thereof. Probes can comprise single-stranded nucleic acids of between 8 to 1000 nucleotides in length, for instance between 10 and 800, between 15 and 700, or between 20 and 500. Longer probes can be used as well. A useful probe is a single stranded nucleic acid molecule of between 8 to 500 nucleotides in length, which can specifically hybridize to a region of a Jak 1, Jak 2, Stat 1 or Stat 2 gene or RNA that carries an alteration. For example, the probe can be directed to a chromosome region occupied by a Jak 1, Jak 2, Stat 1 or Stat 2 gene.

The sequence of the probes can be derived from the sequences of a Jak 1, Jak 2, Stat 1 or Stat 2 gene and RNA as provided herein. Nucleotide substitutions can be performed, as well as chemical modifications of the probe. Such chemical modifications can be accomplished to increase the stability of hybrids (e.g., intercalating groups) or to label the probe. Some examples of labels include, without limitation, radioactivity, fluorescence, luminescence, and enzymatic labeling.

A guide to the hybridization of nucleic acids is found in e.g., Sambrook, ed., Molecular Cloning: A Laboratory Manual (3rd Ed.), Vols. 1-3, Cold Spring Harbor Laboratory, 2001; Current Protocols in Molecular Biology, Ausubel, ed. John Wiley & Sons, Inc., New York, 1997; Laboratory Techniques in Biochemistry and Molecular Biology: Hybridization with Nucleic Acid Probes, Part I. Theory and Nucleic Acid Preparation, Tijssen, ed. Elsevier, N.Y., 1993.

### Specific Ligand Binding.

As discussed herein, alteration in a chromosome region occupied by a Jak 1, Jak 2, Stat 1 or Stat 2 gene or alteration in expression of a Jak 1, Jak 2, Stat 1 or Stat 2 gene, can also be detected by screening for alteration(s) in a sequence or expression level of a polypeptide encoded by a Jak 1, Jak 2, Stat 1 or Stat 2 gene. Different types of ligands can be used, such as specific antibodies. In one embodiment, the sample is contacted with an antibody specific for a polypeptide encoded by a Jak 1, Jak 2, Stat 1 or Stat 2 gene and the formation of an immune complex is subsequently determined. Various methods for detecting an immune complex can be used, such as ELISA, radioimmunoassays (RIA) and immuno-enzymatic assays (IEMA).

For example, an antibody can be a polyclonal antibody, a monoclonal antibody, as well as fragments or derivatives thereof having substantially the same antigen specificity. Fragments include Fab, Fab'2, or CDR regions. Derivatives include single-chain antibodies, humanized antibodies, or poly-functional antibodies. An antibody specific for a polypeptide encoded by a Jak 1, Jak 2, Stat 1 or Stat 2 gene can be an antibody that selectively binds such a polypeptide, namely, an antibody raised against a polypeptide encoded by a Jak 1, Jak 2, Stat 1 or Stat 2 gene or an epitope-containing fragment thereof. Although non-specific binding towards other antigens can occur, binding to the target polypeptide occurs with a higher affinity and can be reliably discriminated from non-specific binding. In one embodiment, the method can comprise contacting a sample from the subject with an antibody specific for a wild type or an altered form of a polypeptide encoded by a Jak 1, Jak 2, Stat 1 or Stat 2 gene, and determining the presence of an immune complex. Optionally, the sample can be contacted to a support coated with antibody specific for the wild type or altered form of a polypeptide encoded by a Jak 1, Jak 2, Stat 1 or Stat 2 gene. In one embodiment, the sample can be contacted simultaneously, or in parallel, or sequentially, with various antibodies specific for different forms of a polypeptide encoded by a Jak 1, Jak 2, Stat 1 or Stat 2 gene, such as a wild type and various altered forms thereof.

### Gene Therapy and Protein Replacement Methods

Delivery of nucleic acids into viable cells can be effected ex vivo, in situ, or in vivo by use of vectors, such as viral vectors (e.g., lentivirus, adenovirus, adeno-associated virus, or a retrovirus), or ex vivo by use of physical DNA transfer methods (e.g., liposomes or chemical treatments). Non-limiting techniques suitable for the transfer of nucleic acid into mammalian cells *in vitro* include the use of liposomes, electroporation, microinjection, cell fusion, DEAE-dextran, and the calcium phosphate precipitation method (See, for example, Anderson, Nature, supplement to vol. 392, no. 6679, pp. 25-20 (1998)). Introduction of a nucleic acid or a gene encoding a polypeptide disclosed herein can also be accomplished with extrachromosomal substrates (transient expression) or artificial chromosomes (stable expression). Cells can also be cultured ex vivo in the presence of therapeutic compositions of the present invention in order to proliferate or to produce a desired effect on or activity in such cells. Treated cells can then be introduced in vivo for therapeutic purposes.

Nucleic acids can be inserted into vectors and used as gene therapy vectors. A number of viruses have been used as gene transfer vectors, including papovaviruses, e.g., SV40 (Madzak et al., 1992), adenovirus (Berkner, 1992; Berkner et al., 1988; Gorziglia and Kapikian, 1992; Quantin et al., 1992; Rosenfeld et al., 1992; Wilkinson et al., 1992; Stratford-Perricaudet et al., 1990), vaccinia virus (Moss, 1992), adeno-associated virus (Muzyczka, 1992; Ohi et al., 1990), herpesviruses including HSV and EBV (Margolskee, 1992; Johnson et al., 1992; Fink et al., 1992; Breakfield and Geller, 1987; Freese et al., 1990), and retroviruses of avian (Biandyopadhyay and Temin, 1984; Petropoulos et al., 1992), murine (Miller, 1992; Miller et al., 1985; Sorge et al., 1984; Mann and Baltimore, 1985; Miller et al., 1988), and human origin (Shimada et al., 1991; Helseth et al., 1990; Page et al., 1990; Buchschacher and Panganiban, 1992). Non-limiting examples of *in vivo* gene transfer techniques include transfection with viral (e.g., retroviral) vectors (see U.S. Pat. No. 5,252,479,) and viral coat protein-liposome mediated transfection (Dzau et al., Trends in Biotechnology 11:205-210 (1993). For example, naked DNA vaccines are generally known in the art; see Brower, Nature Biotechnology, 16:1304-1305 (1998). Gene therapy vectors can be delivered to a subject by, for example, intravenous injection, local administration (see, e.g., U.S. Pat. No. 5,328,470) or by stereotactic injection (see, e.g., Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells that produce the gene delivery system.

For reviews of gene therapy protocols and methods see Anderson et al., Science 256:808-813 (1992); U.S. Pat. Nos. 5,252,479, 5,747,469, 6,017,524, 6,143,290, 6,410,010 6,511,847; and U.S. Application Publication Nos. 2002/0077313 and 2002/00069. For additional reviews of gene therapy technology, see Friedmann, Science, 244:1275-1281 (1989); Verma, Scientific American: 68-84 (1990); Miller, Nature, 357: 455-460 (1992); Kikuchi et al., J Dermatol Sci. 2008 May;50(2):87-98; Isaka et al., Expert Opin Drug Deliv. 2007 Sep;4(5):561-71; Jager et al., Curr Gene Ther. 2007 Aug;7(4):272-83; Waehler et al., Nat Rev Genet. 2007 Aug;8(8):573-87; Jensen et al., Ann Med. 2007;39(2):108-15; Herweijer et al., Gene Ther. 2007 Jan;14(2):99-107; Eliyahu et al., Molecules, 2005 Jan 31;10(1):34-64; and Altaras et al., Adv Biochem Eng Biotechnol. 2005;99:193-260.

Protein replacement therapy can increase the amount of protein by exogenously introducing wild-type or biologically functional protein by way of infusion. A replacement polypeptide can be synthesized according to known chemical techniques or can be produced and purified via known molecular biological techniques. Protein replacement therapy has been developed for various disorders. For example, a wild-type protein can be purified from a recombinant cellular expression system (e.g., mammalian cells or insect cells-see U.S. Pat. No. 5,580,757 to Desnick et al.; U.S. Pat. Nos. 6,395,884 and 6,458,574 to Selden et al.; U.S. Pat. No. 6,461,609 to Calhoun et al.; U.S. Pat. No. 6,210,666 to Miyamura et al.; U.S. Pat. No. 6,083,725 to Selden et al.; U.S. Pat. No. 6,451,600 to Rasmussen et al.; U.S. Pat. No. 5,236,838 to Rasmussen et al. and U.S. Pat. No. 5,879,680 to Ginns et al.), human placenta, or animal milk (see U.S. Pat. No. 6,188,045 to Reuser et al.), or other sources known in the art. After the infusion, the exogenous protein can be taken up by tissues through non-specific or receptor-mediated mechanism.

A polypeptide encoded by a Jak 1, Jak 2, Stat 1 or Stat 2 gene can also be delivered in a controlled release system. For example, the polypeptide can be administered using intravenous infusion, an implantable osmotic pump, a transdermal patch, liposomes, or other modes of administration. In one embodiment, a pump can be used (see is Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target thus requiring only a fraction of the systemic dose (see, e.g., Goodson, in Medical Applications of Controlled Release, supra, vol. 2, pp. 115-138 (1984)). Other controlled release systems are discussed in the review by Langer (Science 249:1527-1533 (1990)).

### Pharmaceutical Compositions and Administration for Therapy

Jak 1, Jak 2, Stat 1 or Stat 2 proteins and Jak 1, Jak 2, Stat 1 or Stat 2 modulating compounds can be administered to the subject once (e.g., as a single injection or deposition). Alternatively, Jak 1, Jak 2, Stat 1 or Stat 2 proteins and Jak 1, Jak 2, Stat 1 or Stat 2 modulating compounds can be administered once or twice daily to a subject in need thereof for a period of from about two to about twenty-eight days, or from about seven to about ten days. Jak 1, Jak 2, Stat 1 or Stat 2 proteins and Jak 1, Jak 2, Stat 1 or Stat 2 modulating compounds can also be administered once or twice daily to a subject for a period of 1, 2, 3, 4, 5, 6, 7, 8, 9, 10, 11, 12 times per year, or a combination thereof. Furthermore, Jak 1, Jak 2, Stat 1 or Stat 2 proteins and Jak 1, Jak 2, Stat 1 or Stat 2 modulating compounds of the invention can be co-administrated with another therapeutic. Where a dosage regimen comprises multiple administrations, the effective amount of the Jak 1, Jak 2, Stat 1 or Stat 2 proteins and Jak 1, Jak 2, Stat 1 or Stat 2 modulating compounds administered to the subject can comprise the total amount of gene product administered over the entire dosage regimen.

Jak 1, Jak 2, Stat 1 or Stat 2 proteins and Jak 1, Jak 2, Stat 1 or Stat 2 modulating compounds can be administered to a subject by any means suitable for delivering the Jak 1, Jak 2, Stat 1 or Stat 2 proteins and Jak 1, Jak 2, Stat 1 or Stat 2 modulating compounds to cells of the subject, such as the dermis, epidermis, dermal papilla cells, or hair follicle cells. For example, Jak 1, Jak 2, Stat 1 or Stat 2 proteins and Jak 1, Jak 2, Stat 1 or Stat 2 modulating compounds can be administered by methods suitable to transfect cells. Transfection methods for eukaryotic cells are well known in the art, and include direct injection of the nucleic acid into the nucleus or pronucleus of a cell; electroporation; liposome transfer or transfer mediated by lipophilic materials; receptor mediated nucleic acid delivery, bioballistic or particle acceleration; calcium phosphate precipitation, and transfection mediated by viral vectors.

The compositions of this invention can be formulated and administered to reduce the symptoms associated with a hair-loss disorder by any means that produces contact of the active ingredient with the agent's site of action in the body of a subject, such as a human or animal (e.g., a dog, cat, or horse). They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

A therapeutically effective dose of Jak 1, Jak 2, Stat 1 or Stat 2 modulating compounds can depend upon a number of factors known to those or ordinary skill in the art. The dose(s) of the Jak 1 or Jak 2 modulating compounds can vary, for example, depending upon the identity, size, and condition of the subject or sample being treated, further depending upon the route by which the composition is to be administered, if applicable, and the effect which the practitioner desires the Jak 1 or Jak 2modulating compounds to have upon the nucleic acid or polypeptide disclosed herein. These amounts can be readily determined by a skilled artisan. Any of the therapeutic applications described herein can be applied to any subject in need of such therapy, including, for example, a mammal such as a dog, a cat, a cow, a horse, a rabbit, a monkey, a pig, a sheep, a goat, or a human.

Pharmaceutical compositions for use in accordance with the invention can be formulated in conventional manner using one or more physiologically acceptable carriers or excipients. The therapeutic compositions of the invention can be formulated for a variety of routes of administration, including systemic and topical or localized administration. Techniques and formulations generally can be found in Remmington's Pharmaceutical Sciences, Meade Publishing Co., Easton, Pa (20th Ed., 2000). For systemic administration, an injection is useful, including intramuscular, intravenous, intraperitoneal, and subcutaneous. For injection, the therapeutic compositions of the invention can be formulated in liquid solutions, for example in physiologically compatible buffers such as Hank's solution or Ringer's solution. In addition, the therapeutic compositions can be formulated in solid form and redissolved or suspended immediately prior to use. Lyophilized forms are also included. Pharmaceutical compositions of the present invention are characterized as being at least sterile and pyrogen-free. These pharmaceutical formulations include formulations for human and veterinary use.

According to the invention, a pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

The disclosure also relates to a kit that comprises a pharmaceutically acceptable carrier and a Jak 1, Jak 2, Stat 1 or Stat 2 modulating compound identified using the screening assays disclosed herein packaged with instructions for use. For modulators that are antagonists of the activity of a Jak 1, Jak 2, Stat 1 or Stat 2 protein, or which reduce the expression of a Jak 1, Jak 2, Stat 1 or Stat 2 protein, the instructions would specify use of the pharmaceutical composition for promoting the loss of hair on the body surface of a mammal (for example, arms, legs, bikini area, face).

For Jak 1 or Jak 2 modulating compounds that are agonists of the activity of a Jak 1 or Jak 2protein or increase the expression of one or more proteins encoded by Jak 1 or Jak 2genes, the instructions would specify use of the pharmaceutical composition for regulating hair growth. In one embodiment, the instructions would specify use of the pharmaceutical composition for the treatment of hair loss disorders. In a further embodiment, the instructions would specify use of the pharmaceutical composition for restoring hair pigmentation. For example, administering an agonist can reduce hair graying in a subject.

A pharmaceutical composition containing a Jak 1 or Jak 2modulating compound can be administered in conjunction with a pharmaceutically acceptable carrier, for any of the therapeutic effects discussed herein. Such pharmaceutical compositions can comprise, for example antibodies directed to polypeptides encoded by genes comprising a Jak 1 or Jak 2 gene, or variants thereof, or agonists and antagonists of a polypeptide encoded by a Jak 1 or Jak 2 gene. The compositions can be administered alone or in combination with at least one other agent, such as a stabilizing compound, which can be administered in any sterile, biocompatible pharmaceutical carrier including, but not limited to, saline, buffered saline, dextrose, and water. The compositions can be administered to a patient alone, or in combination with other agents, drugs or hormones.

Sterile injectable solutions can be prepared by incorporating the Jak 1 or Jak 2, modulating compound (e.g., a polypeptide or antibody) in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

In some embodiments, the Jak 1 or Jak 2 modulating compound can be applied via transdermal delivery systems, which slowly releases the active compound for percutaneous absorption. Permeation enhancers can be used to facilitate transdermal penetration of the active factors in the conditioned media. Transdermal patches are described in for example, U.S. Pat. No. 5,407,713; U.S. Pat. No. 5,352,456; U.S. Pat. No. 5,332,213; U.S. Pat. No. 5,336,168; U.S. Pat. No. 5,290,561; U.S. Pat. No. 5,254,346; U.S. Pat. No. 5,164,189; U.S. Pat. No. 5,163,899; U.S. Pat. No. 5,088,977; U.S. Pat. No. 5,087,240; U.S. Pat. No. 5,008,110; and U.S. Pat. No. 4,921,475.

Various routes of administration and various sites of cell implantation can be utilized, such as, subcutaneous or intramuscular, in order to introduce the aggregated population of cells into a site of preference. Once implanted in a subject (such as a mouse, rat, or human), the aggregated cells can then stimulate the formation of a hair follicle and the subsequent growth of a hair structure at the site of introduction. In another embodiment, transfected cells (for example, cells expressing a protein encoded by a Jak 1 or Jak 2 gene are implanted in a subject to promote the formation of hair follicles within the subject. In further embodiments, the transfected cells are cells derived from the end bulb of a hair follicle (such as dermal papilla cells or dermal sheath cells). Aggregated cells (for example, cells grown in a hanging drop culture) or transfected cells (for example, cells produced as described herein) maintained for 1 or more passages can be introduced (or implanted) into a subject (such as a rat, mouse, dog, cat, human, and the like).

"Subcutaneous" administration can refer to administration just beneath the skin (i.e., beneath the dermis). Generally, the subcutaneous tissue is a layer of fat and connective tissue that houses larger blood vessels and nerves. The size of this layer varies throughout the body and from person to person. The interface between the subcutaneous and muscle layers can be encompassed by subcutaneous administration.

This mode of administration can be feasible where the subcutaneous layer is sufficiently thin so that the factors present in the compositions can migrate or diffuse from the locus of administration and contact the hair follicle cells responsible for hair formation. Thus, where intradermal administration is utilized, the bolus of composition administered is localized proximate to the subcutaneous layer.

Administration of the cell aggregates (such as DP or DS aggregates) is not restricted to a single route, but can encompass administration by multiple routes. For instance, exemplary administrations by multiple routes include, among others, a combination of intradermal and intramuscular administration, or intradermal and subcutaneous administration. Multiple administrations can be sequential or concurrent. Other modes of application by multiple routes will be apparent to the skilled artisan.

In other embodiments, this implantation method will be a one-time treatment for some subjects. In further embodiments, multiple cell therapy implantations will be required. In some embodiments, the cells used for implantation will generally be subject-specific genetically engineered cells. In another embodiment, cells obtained from a different species or another individual of the same species can be used. Thus, using such cells can require administering an immunosuppressant to prevent rejection of the implanted cells. Such methods have also been described in United States Patent Application Publication 2004/0057937 and PCT application publication WO 2001/32840.

### Inhibitors

Cytokines are produced to activate neighboring cells to communicate danger signals to one another and spread and amplify the inflammatory response. Over the years, it was learned how to both neutralize these cytokines with blocking antibodies and inhibit their signaling in responding cells by small molecule protein tyrosine kinase inhibitors FDA approved drugs exist for both approaches, e.g., IL-2 and TNF blocking antibodies and the small orally available molecule Gleevec™ that blocks cytokine signaling. Whenever possible, the center will pursue topical small molecule formulations that should improve efficacy while limiting systemic toxicity (improved therapeutic indexes) encouraging clinical evaluation in AA of other small molecule inhibitors in the biopharmaceutical pipeline.

To block signaling of cytokine receptors, topical/oral JAK1/2 inhibitor (Incyte), which has already demonstrated safety and efficacy in patients with psoriaisis and RA, can be used.

The inhibitors can comprise peptides (such as antibodies or fragments thereof), small molecules, nucleic acids (such as siRNA or antisense RNA), or other agents) that can bind to a polypeptide molecule encoded by a gene of interest and/or molecules that have an inhibitory effect on the biological activity of a protein of interest or its expression.

As used herein, a "Jak 1 inhibitor" refers to a compound that interacts with a Jak 1 gene or a Jak 1 protein or polypeptide and inhibits its activity and/or its expression. The compound can decrease the activity or expression of a protein encoded by Jak 1.

In one embodiment, a Jak 1 inhibitor can be a peptide fragment that binds a protein comprising SEQ ID NO: 1. For example, the fragment can encompass any portion of at least about 8 consecutive amino acids of SEQ ID NO: 1. The fragment can comprise at least about 10 consecutive amino acids, at least about 20 consecutive amino acids, at least about 30 consecutive amino acids, at least about 40 consecutive amino acids, at least about 50 consecutive amino acids, at least about 60 consecutive amino acids, or at least about 75 consecutive amino acids of SEQ ID NO: 1. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England).

As used herein, a "Jak 2 inhibitor" refers to a compound that interacts with a Jak 2 gene or a Jak 2 protein or polypeptide and inhibits its activity and/or its expression. The compound can decrease the activity or expression of a protein encoded by Jak 2.

In one embodiment, a Jak 2 inhibitor can be a peptide fragment that binds a protein comprising SEQ ID NO: 3. For example, the fragment can encompass any portion of at least about 8 consecutive amino acids of SEQ ID NO: 3. The fragment can comprise at least about 10 consecutive amino acids, at least about 20 consecutive amino acids, at least about 30 consecutive amino acids, at least about 40 consecutive amino acids, at least about 50 consecutive amino acids, at least about 60 consecutive amino acids, or at least about 75 consecutive amino acids of SEQ ID NO: 3. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England).

As used herein, a "Stat 1 inhibitor" refers to a compound that interacts with a Stat 1 gene or a Stat 1 protein or polypeptide and inhibits its activity and/or its expression. The compound can decrease the activity or expression of a protein encoded by Stat 1.

In one embodiment, a Stat 1 inhibitor can be a peptide fragment that binds a protein comprising SEQ ID NO: 5. For example, the fragment can encompass any portion of at least about 8 consecutive amino acids of SEQ ID NO: 5. The fragment can comprise at least about 10 consecutive amino acids, at least about 20 consecutive amino acids, at least about 30 consecutive amino acids, at least about 40 consecutive amino acids, at least about 50 consecutive amino acids, at least about 60 consecutive amino acids, or at least about 75 consecutive amino acids of SEQ ID NO: 5. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England).

As used herein, a "Stat 2 inhibitor" refers to a compound that interacts with a Stat 2 gene or a Stat 2 protein or polypeptide and inhibits its activity and/or its expression. The compound can decrease the activity or expression of a protein encoded by Stat 2.

In one embodiment, a Stat 2 nhibitor can be a peptide fragment that binds a protein comprising SEQ ID NO: 7. For example, the fragment can encompass any portion of at least about 8 consecutive amino acids of SEQ ID NO: 7. The fragment can comprise at least about 10 consecutive amino acids, at least about 20 consecutive amino acids, at least about 30 consecutive amino acids, at least about 40 consecutive amino acids, at least about 50 consecutive amino acids, at least about 60 consecutive amino acids, or at least about 75 consecutive amino acids of SEQ ID NO: 7. Fragments include all possible amino acid lengths between and including about 8 and about 100 amino acids, for example, lengths between about 10 and about 100 amino acids, between about 15 and about 100 amino acids, between about 20 and about 100 amino acids, between about 35 and about 100 amino acids, between about 40 and about 100 amino acids, between about 50 and about 100 amino acids, between about 70 and about 100 amino acids, between about 75 and about 100 amino acids, or between about 80 and about 100 amino acids. These peptide fragments can be obtained commercially or synthesized via liquid phase or solid phase synthesis methods (Atherton et al., (1989) Solid Phase Peptide Synthesis: a Practical Approach. IRL Press, Oxford, England).

As used herein, a "Jak/Stat inhibitor" refers to a compound that interacts with a Jak1/Jak2/Stat1/Stat2 gene or a Jak1/Jak2/Stat1/Stat2 protein or polypeptide and inhibits its activity and/or its expression. The compound can decrease the activity or expression of a protein encoded by Jak1/Jak2/Stat1/Stat2.

An inhibitor can be a protein, such as an antibody (monoclonal, polyclonal, humanized, chimeric, or fully human), or a binding fragment thereof, directed against a polypeptide encoded by SEQ ID NO: 1, 3, 5 or 7. An antibody fragment can be a form of an antibody other than the full-length form and includes portions or components that exist within full-length antibodies, in addition to antibody fragments that have been engineered. Antibody fragments can include, but are not limited to, single chain Fv (scFv), diabodies, Fv, and (Fab')₂, triabodies, Fc, Fab, CDR1, CDR2, CDR3, combinations of CDR's, variable regions, tetrabodies, bifunctional hybrid antibodies, framework regions, constant regions, and the like (*see*, Maynard et al., (2000) Ann. Rev. Biomed. Eng. 2:339-76; Hudson (1998) Curr. Opin. Biotechnol. 9:395-402). Antibodies can be obtained commercially, custom generated, or synthesized against an antigen of interest according to methods established in the art (Janeway et al., (2001) Immunobiology, 5th ed., Garland Publishing).

An inhibitor can also be a small molecule that binds to a protein and disrupts its function. Small molecules are a diverse group of synthetic and natural substances generally having low molecular weights. They can be isolated from natural sources (for example, plants, fungi, microbes and the like), are obtained commercially and/or available as libraries or collections, or synthesized. Candidate small molecules that modulate a protein can be identified via *in silico* screening or high-through-put (HTP) screening of combinatorial libraries. Most conventional pharmaceuticals, such as aspirin, penicillin, and many chemotherapeutics, are small molecules, can be obtained commercially, can be chemically synthesized, or can be obtained from random or combinatorial libraries (Werner et al., (2006) Brief Funct. Genomic Proteomic 5(1):32-6). In some embodiments, the agent is a small molecule that binds, interacts, or associates with a target protein or RNA. Such a small molecule can be an organic molecule that, when the target is an intracellular target, is capable of penetrating the lipid bilayer of a cell to interact with the target. Small molecules include, but are not limited to, toxins, chelating agents, metals, and metalloid compounds. Small molecules can be attached or conjugated to a targeting agent so as to specifically guide the small molecule to a particular cell.

### Pharmaceutical Compositions and Administration for Therapy

An inhibitor or agonist disclosed herein can be incorporated into pharmaceutical compositions suitable for administration, for example the inhibitor and a pharmaceutically acceptable carrier.

A pharmaceutically acceptable carrier can comprise any and all solvents, dispersion media, coatings, antibacterial and antifungal agents, isotonic and absorption delaying agents, and the like, compatible with pharmaceutical administration. The use of such media and agents for pharmaceutically active substances is well known in the art. Any conventional media or agent that is compatible with the active compound can be used. Supplementary active compounds can also be incorporated into the compositions.

Any of the therapeutic applications described herein can be applied to any subject in need of such therapy, including, for example, a mammal such as a dog, a cat, a cow, a horse, a rabbit, a monkey, a pig, a sheep, a goat, or a human.

A pharmaceutical composition can be administered in conjunction with a pharmaceutically acceptable carrier, for any of the therapeutic effects discussed herein. Such pharmaceutical compositions can comprise, for example antibodies directed to polypeptides. The compositions can be administered alone or in combination with at least one other agent, such as a stabilizing compound, which can be administered in any sterile, biocompatible pharmaceutical carrier including, but not limited to, saline, buffered saline, dextrose, and water. The compositions can be administered to a patient alone, or in combination with other agents, drugs or hormones.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it can be useful to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the inhibitor (e.g., a polypeptide or antibody or small molecule) or agonist in the required amount in an appropriate solvent with one or a combination of ingredients enumerated herein, as required, followed by filtered sterilization. Generally, dispersions are prepared by incorporating the active compound into a sterile vehicle which contains a basic dispersion medium and the required other ingredients from those enumerated herein. In the case of sterile powders for the preparation of sterile injectable solutions, examples of useful preparation methods are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention.

Those skilled in the art will recognize, or be able to ascertain, using no more than routine experimentation, numerous equivalents to the specific substances and procedures described herein. Such equivalents are considered to be within the scope of this invention, and are covered by the following claims.

Publications and references cited herein are not admitted to be prior art.

### EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

### EXAMPLE 1

Interferon γ has been shown to be a target in murine AA (*see* Nakamura, et al. 2008, Am J Pathol, 172(3): 650-658; *see also* Freyschmidt-Paul et al., Br J Dermatol., 155(3):515-521; *see also* Gilhar et al., Journal Invest. Dermatol., 124(1):288-289). In human AA, AA PBMCs show Th1 skewing, AA skin shows IFN signature and GWAS genes (SOCS/IFN-g/).but also IL-2/6/13/21/26.

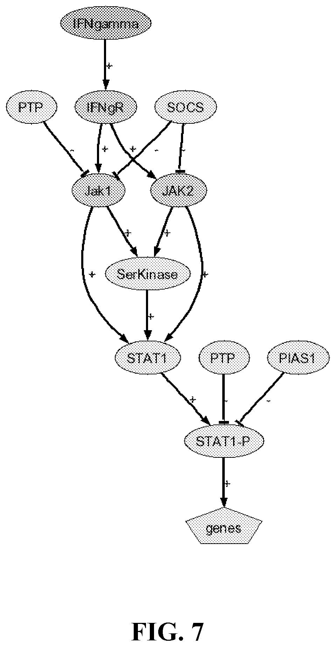

The interferon γ pathway (**FIG. 7**) induces NKG2DL in target HF cells, enhances antigen presentation by DCs, promotes Th1 cellular autoimmunity and augments NK and CTL mediated- cytolysis.

JAK1/2 inhibitors in clinical development include a) INCB018424, topical and oral; 5nM activity (Incyte); b) CEP-701 (Cephalon); and c) TG101348.
a) 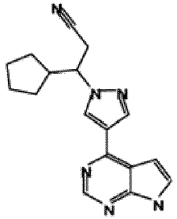
b) 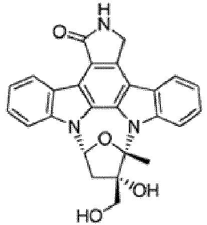
c)

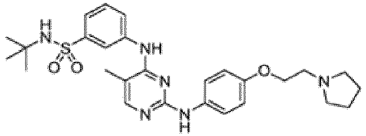

**Table 1. INCB18424-203: Treatment-Emergent Adverse Events in Descending Order (all casualties) (oral delivery is considered an acceptable profile)**

| Medra Preferred Term | Vehicle (n=50) | Active (n=149) |
|---|---|---|
| Application site irritation, pruritus | 7 (14.0) | 14 (9.3) |
| Nasopharyngitis | 1 (2.0) | 10 (6.7) |
| Upper respiratory tract infection | 3 (6.0) | 10 (6.7) |
| Sinusitis | 1 (2.0) | 6 (4.0) |
| Back Pain | 0 (0.0) | 5 (3.4) |
| Headache | 0 (0.0) | 4 (2.7) |
| Influenza | 2 (4.0) | 4 (2.7) |
| Abdominal pain | 0 (0.0) | 3 (2.0) |
| Six SAEs, none treatment related: prostate ca, prolonged hospitalization for cholecystitis, cholelithiasis (1.5%); herniated disc repair, coronary occlusion with hx ASHD (0.5%). CVA (vehicle) | | |

Clinical programs in AA include the following:
- Mild-Moderate AA: Phase 2/3 Psoriasis and oral Phase 3 Myelofibrosis, using a topical JAK inhibitor

A population-based cohort evaluated cases ascertained through National Alopecia Areata Registry vs. controls ascertained through New York City Cancer project. They were matched for Northern European ancestry using AIMS.
- Cases: n=1080 (Alopecia Universalis=482; Alopecia Totalis=120; Alopecia Areata Transient=176; Alopecia Areata Patchy=302)
- Controls: n=1053

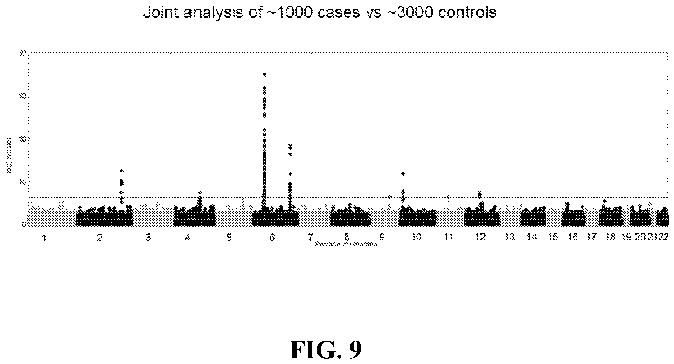

**FIG. 9** presents a Genomewide Association Study (GWAS) in alopecia areata.

**Table 2: List of Genes implicated in AA.**

| **Region** | **Gene** | **Function** | **Stongest association (pvalue)** | **Maximum odds ratio** |
|---|---|---|---|---|
| 2q33.2 | CTLA4 | T-cell proliferation | 3.55x10⁻¹³ | 1.44 |
| | ICOS | T-cell proliferation | 4.33x10⁻⁰⁸ | 1.32 |
| 4q27 | IL21/IL2 | T-, B- and NK-cell proliferation | 4.27x10⁻⁰⁸ | 1.34 |
| 6q25.1 | ULBP6 | NKG2D activating ligand | 4.49x10⁻¹⁹ | 1.65 |
| | ULBP3 | NKG2D activating ligand | 4.43x10⁻¹⁷ | 1.52 |
| 9q31.1 | STX17 | premature hair graying | 3.60x10⁻⁰⁷ | 1.33 |
| 10p15.1 | IL2RA (CD25) | T-cell proliferation | 1.74x10⁻¹² | 1.41 |
| 11q13 | PRDX5 | antioxidant enzyme | 4.14x10⁻⁰⁷ | 1.33 |
| 12q13 | Eos (IKZF4) | T-cell proliferation | 3.21x10⁻⁰⁸ | 1.34 |
| | ERBB3 | epidermal growth factor receptor | 1.27x10⁻⁰⁷ | 1.34 |
| 6p21.32 (HLA) | MICA | NKG2D activating ligand | 1.19x10⁻¹⁷ | 1.44 |
| | NOTCH4 | T-cell differentiation | 1.03x10⁻⁰⁸ | 1.61 |
| | BTNL2 | T-cell proliferation | 2.11x10⁻²⁶ | 2.70 |
| | HLA-DRA | Antigen presentation | 2.93x10⁻³¹ | 2.62 |
| | HLA-DQA1 | Antigen presentation | 3.60x10⁻¹⁷ | 2.15 |
| | HLA-DQA2 | Antigen presentation | 1.38x10⁻³⁵ | 5.43 |
| | HLA-DQB2 | Antigen presentation | 1.73x10⁻¹³ | 1.60 |
| | HLA-DOB | Antigen presentation | 2.07x10⁻⁰⁸ | 1.72 |

**Table 3: Common Cause Model of Skin Autoimmune Diseases. See also http://www.genome.gov/gwastudies (for psoriasis) and Jin et al., 2010, NEJM, 362: 1686-1697.**

| **Region** | **Gene** | **Stongest association (pvalue)** | **Maximum odds ratio** | | **Involved in other autoimmune disease** |
|---|---|---|---|---|---|
| 2q33.2 | CTLA4 | 3.55x10⁻¹³ | 1.44 | | T1D, RA, CeD, MS, SLE, GD |
| | ICOS | 4.33x10⁻⁰⁸ | 1.32 | | T1D, RA, CeD, MS, SLE, GD |
| 4q27 | IL21/IL2 | 4.27x10⁻⁰⁸ | 1.34 | ● | T1D, RA, CeD, PS |
| 6q25.1 | ULBP6 | 4.49x10⁻¹⁹ | 1.65 | | *none* |
| | ULBP3 | 4.43x10⁻¹⁷ | 1.52 | | *none* |
| 9q31.1 | STX17 | 360x10⁻⁰⁷ | 1.33 | | *none* |
| 10p15.1 | IL2RA (CD25) | 1.74x10⁻¹² | 1.41 | 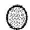 | T1D, MS, GD, GV |
| 11q13 | PRDX5 | 4.14x10⁻⁰⁷ | 1.33 | | MS |
| 12q13 | Eos (IKZF4) | 3.21x10-⁰⁸ | 1.34 | | T1D, SLE |
| | ERBB3 | 1.27x10⁻⁰⁷ | 1.34 | | T1D, SLE |
| 6p21.32 (HLA) | MICA | 1.19x10⁻⁰⁷ | 1.44 | ● | T1D, RA, CeD, UC, PS, SLE |
| | NOTCH4 | 1.03x10⁻⁰⁸ | 1.61 | | T1D, RA, MS |
| | C6orf10 | 1.45x10⁻¹⁶ | 2.36 | 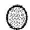 ● | T1D, RA, PS, GV |
| | BTNL2 | 2.11x10⁻²⁶ | 2.70 | 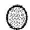 | T1D, RA, UC, CD, SLE, MS, GV |
| | HLA-DRA | 2.93x10⁻³¹ | 2.62 | 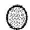 | T1D, RA, CeD, MS, GV |
| | HLA-DQA1 | 3.60x10⁻¹⁷ | 2.15 | 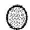 ● | T1D, RA, CeD, MS. SLE, PS, CD, UC, GD, GV |
| | HLA-DQA2 | 1.38x10⁻³⁵ | 5.43 | | T1D, RA |
| | HLA-DQB2 | 1.73x10⁻¹³ | 1.60 | | RA |
| | HLA-DOB | 2.07x10⁻⁰⁸ | 1.72 | | |
| Type I diabetes (T1D), rheumatoid arthritis (RA), celiac disease (CeD), multiple sclerosis (MS), system lupus erythematosus (SLE), Graves disease (GD), psoriasis (PS), Generalized Vitiligo (GV). | | | | | |

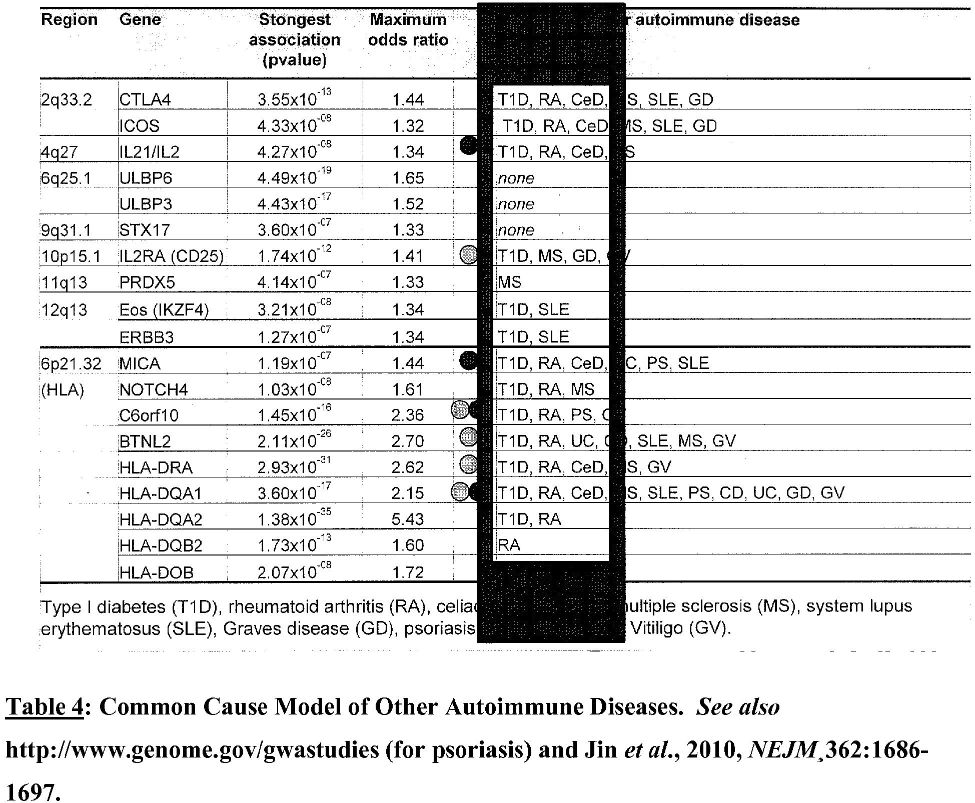

**Table 5: List of Genes implicated in AA. Black circles indicate genes that affect NKG2D; gray circles indicate genes that affect T-cell regulation; black circles with star indicate genes that affect end organs**

| **Region** | | **Gene** | **Function** | **Stongest association (pvalue)** | **Maximum odds ratio** |
|---|---|---|---|---|---|
| 2q33.2 | 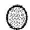 | CTLA4 | T-cell proliferation | 3.55x10⁻¹³ | 1.44 |
| | | ICOS | T-cell proliferation | 4.33x10⁻⁰⁸ | 1.32 |
| 4q27 | 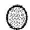 | IL21/IL2 | T-, Band NK-cell proliferation | 4.27x10⁻⁰⁸ | 1.34 |
| 6q25.1 | ● | ULBP6 | NKG2D activating ligand | 4.49x10⁻¹⁹ | 1.65 |
| | ● | ULBP3 | NKG2D activating ligand | 4.43x10⁻¹⁷ | 1.52 |
| 9q31.1 | 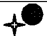 | STX17 | premature hair graying | 3.60x10⁻⁰⁷ | 1.33 |
| 10p15.1 | 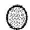 | IL2RA (CD25) | T-cell proliferation | 1.74x10⁻¹² | 1.41 |
| 11q13 | 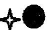 | PRDX5 | antioxidant enzyme | 4.14x10⁻⁰⁷ | 1.33 |
| 12q13 | 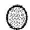 | Eos (IKZF4) | T-cell proliferation | 3.21x10⁻⁰⁸ | 1.34 |
| | | ERBB3 | epidermal growth factor receptor | 1.27x10⁻⁰⁷ | 1.34 |
| 6p21.32 | ● | MICA | NKG2D activating ligand | 1.19x10⁻⁰⁷ | 1.44 |
| (HLA) | | NOTCH4 | T-cell differentiation | 1.03x10⁻⁰⁸ | 1.61 |
| | | C6orf10 | | 1.45x10⁻¹⁶ | 2.36 |
| | | BTNL2 | T-cell proliferation | 2.11x10⁻²⁶ | 2.70 |
| | | HLA-DRA | Antigen presentation | 2.93x10⁻³¹ | 2.62 |
| | | HLA-DQA1 | Antigen presentation | 3.60x10⁻¹⁷ | 2.15 |
| | | HLA-DQA2 | Antigen presentation | 1.38x10⁻³⁵ | 5.43 |
| | | HLA-DQB2 | Antigen presentation | 1.73x10⁻¹³ | 1.60 |
| | | HLA-DOB | Antigen presentation | 2.07x10⁻⁰⁸ | 1.72 |

Common pathways with other autoimmune diseases, including NK ligands in the target organs include the following:
- In RA, synoviocytes aberrantly express MIC ligands, leading to autoreactive T cell stimulation.
- In Celiac disease, MIC ligands are overexpressed in the gut epithelium during active disease.
- In Type 1 diabetes, Islet cells in prediabetic NOD mice express RAE-1 ligand.
The aberrant expression of NK activating ligands in genetically predisposed individuals can induce or exacerbate disease.

Candidate pathways in AA that present new opportunities for therapy include the following:
- Members of the costimulatory receptor family, which have complementary effects on T cell activation, and their balance controls the overall outcome of immune and autoimmune responses.
- Genes which enhance the NK cell cytotoxic response; triggers T cells to produce proinflammatory cytokines; and induces CD4(+) T cells to differentiate into Th17 cells.
- Genes which pivotal role in regulating the adaptive immune system by controlling the survival and proliferation of regulatory T (Treg) cells, which are required for the maintenance of immune tolerance.
- Activating receptors expressed on natural killer (NK) cells, NK1.1 (+) T cells, and T cells. NKG2D ligands include MHC class I chain-related (MIC)A, MICB.

Candidate compounds for treatment of AA include compounds directed to Jak/Stat signaling are described in Ivanenkov et al., Mini Rev Med Chem. 2011 Jan;11(1):55-78.

### EXAMPLE 2

The following genetic studies will be performed:
- A replication study with another 1000 AA patients
- Deep sequencing of candidate regions
- Collection of 10,000 patients
- Common Genetic Studies T1D, CeO and AA
An immunology study using NK activating ligands will be performed using ULBP3, ULBP6 - inducible transgenics, new mouse models.

**Table 6: Genes with Nominal Significance on the GWAS studies**

| | ***Gene*** | ***Mb*** | ***Count of SNPs <1x10⁻⁴*** | ***Min p-value observed*** | ***Min p-value imputed*** | ***Autoimmune Reports*** | **GO *classification*** |
|---|---|---|---|---|---|---|---|
| **Chromosome 2** | | | | | | | |
| | HDAC4 | 240.03 | 1 | 8.10E-05 | 5.59E-05 | | inflammatory response |

| **Chromosome 3** | | | | | | | |
|---|---|---|---|---|---|---|---|
| | CACNA2D3 | 55.02 | 1 | 7.28E-05 | 1.47E-05 | CeD | |

| **Chromosome 5** | | | | | | | |
|---|---|---|---|---|---|---|---|
| | IL13 | 132.02 | 2 | 1.87E-06 | | Asthma | immune response |

| **Chromosome 6** | | | | | | | |
|---|---|---|---|---|---|---|---|
| | HLA-G | 29.94 | 1 | 1.07E-04 | 4.54E-06 | RA, MS, SLE, PS, T1D, Asthma, | immune response |
| | HLA-A | 30.01 | 1 | 1.00E-04 | 2.72E-05 | MS, T1D, PS, GD, Asthma, Vitilago | immune response |
| | MICB | 31.59 | 2 | 1.89E-05 | 1.97E-05 | MS, T1D, UC, RA, CeD, Asthma | immune response |
| | TAP2 | 32.91 | 1 | 6.42E-06 | 1.28E-05 | T1D, RA, SLE, PS, GD | immune response |

| **Chromosome 7** | | | | | | | |
|---|---|---|---|---|---|---|---|
| | IL6 | 22.72 | 2 | 7.72E-05 | 4.34E-05 | RA, T1D, CeD | inflammatory response |
| | CHCHD3 | 132.44 | 1 | 3.07E-05 | 2.02E-05 | CeD | |

| **Chromosome 8** | | | | | | | |
|---|---|---|---|---|---|---|---|
| | CSMD1 | 3.02 | 1 | 8.65E-05 | 8.38E-05 | CeD, MS, PS | |

| **Chromosome 12** | | | | | | | |
|---|---|---|---|---|---|---|---|
| | IFNG | 66.84 | 1 | 1.55E-05 | 1.29E-05 | CeD, T1D, RA, MS, SLE, PS, GD, Asthma | |
| | IL26 | 66.87 | 2 | 7_{.}18E-05 | 6.45E-05 | MS, Asthma | immune response |

| **Chromosome 16** | | | | | | | |
|---|---|---|---|---|---|---|---|
| | KIAA0350 (CLEC16A) | 11.11 | 6 | 1.77E-05 | 1.15E-05 | T1D, MS, Thyroid Disease | |
| | SOCS1 | 11.24 | 2 | 1.16E-05 | 8.66E-06 | CeD, T1D, Asthma | |

| **Chromosome 18** | | | | | | | |
|---|---|---|---|---|---|---|---|
| | ANKRD12 | 9.25 | 2 | 3.59E-05 | 1.55E-05 | | |
| | PTPN2 | 12.80 | 1 | 4.09E-06 | 3.38E-07 | CD, T1D | |

**Table 7: Pathway analysis of nominal genes (see http://david.abcc.ncifcrf.gov/)**

| **Category** | **Term** | **Genes** |
|---|---|---|
| hsa05332 | Graft-versus-host disease | IL6, IFNG, HLA-A, HLA-DMA, HLA-G |
| hsa04612 | Antigen processing and presentation | HSPA1L, TAP2, HLA-A, HLA-DMA, HLA-G |
| hsa04630 | Jak-STAT signaling pathway | SPRY2, IL6, SOCS1, IFNG, IL26, IL13 |
| hsa05330 | Allograft rejection | IFNG, HLA-A, HLA-DMA, HLA-G |
| hsa04940 | Type I diabetes mellitus | IFNG, HLA-A, HLA-DMA, HLA-G |
| hsa04650 | Natural killer cell mediated cytotoxicity | MICB, IFNG, HLA-A, HLA-G |
| hsa05320 | Autoimmune thyroid disease | HLA-A, HLA-DMA, HLA-G |
| hsa05416 | Viral myocarditis | HLA-A, HLA-DMA, HLA-G |
| hsa04060 | Cytokine-cytokine receptor interaction | IL6, IFNG, IL26, IL13 |

### EXAMPLE 3- Study samples, genotyping, quality control and population stratification

Alopecia areata (AA) is a major medical problem and is the most prevalent autoimmune disease in the US (**Table 8**), affecting approximately 4.6 million people, including males and females across all ethnic groups, with a lifetime risk of 1.7% (Kyriakis KP, Paltatzidou K, Kosma E, Sofouri E, Tadros A, Rachioti E. Alopecia areata prevalence by gender and age. J Eur Acad Dermatol Venereol. 23:572-3, 2009). Additionally, AA represents the second most common form of human hair loss, second only to androgenetic alopecia, and causes significant disfigurement and psychological distress to affected individuals (**FIG. 1**). AA affects more individuals than most other autoimmune diseases *combined,* including lupus erythematosus (LE), type 1 diabetes (T1D), psoriasis, multiple sclerosis (MS) and rheumatoid arthritis (RA) (**Table 8**).

**Table 8. Automimmune Disease Prevalence**

| **Autoimmune Disease** | **Rate per 100,000** |
|---|---|
| Alopecia Areata | 1700 |
| Psoriasis | 696-1527 |
| Rheumatoid arthritis | 310-800 |
| Type 1 diabetes | 227-355 |
| Multiple sclerosis | 177-358 |
| Systemic lupus erythematosus | 34-150 |

In stark contrast to these other conditions, research into the pathogenesis and the development of innovative therapies in AA has lagged far behind. This can be due in part to the perception that AA is merely a cosmetic disorder. In reality, AA carries one of the highest burdens among any skin diseases, particularly among children and adolescents whose self-image is so closely linked to their appearance (Bickers DR, Lim HW, Margolis D, Weinstock MA, Goodman C, Faulkner E, Gould C, Gemmen E, Dall T; American Academy of Dermatology Association; Society for Investigative Dermatology. The burden of skin diseases: 2004 a joint project of the American Academy of Dermatology Association and the Society for Investigative Dermatology. J Am Acad Dermatol. 55:490-5, 2006).

Despite its high prevalence, there are no evidence-based treatments for AA. A comprehensive Cochrane analysis assessment of seventeen randomized clinical trials (RCTs) involving a total of 540 participants found no proven treatment of AA (Delamere FM, Sladden MM, Dobbins HM, Leonardi-Bee J. Interventions for alopecia areata. Cochrane Database Syst Rev. 2:CD004413, 2008). Each trial included from 6 to 85 participants and assessed a range of interventions that included topical and oral corticosteroids, topical cyclosporin, photodynamic therapy and topical minoxidil. Overall, none of the interventions showed significant treatment benefit in terms of hair growth when compared with placebo. It was concluded that, few, (if any), treatments for AA are proven to be effective. No RCTs on the use of diphencyprone, dinitrochlorobenzene, intralesional corticosteroids or dithranol were found, although these drugs are commonly used for the treatment of AA. Similarly, although topical corticosteroids and minoxidil are widely prescribed and appear to be safe, there is no convincing evidence that they are beneficial in the long-term. Most trials have been poorly reported and/or are so small that any important clinical benefits are inconclusive. These observations underscore the importance of defining the genetic basis of AA and determining its pathogenesis, so that rational therapeutic approaches can be developed and translational research can begin.

### Pre-clinical Evaluation of Jak/Stat Targeting in Alopecia Areata

The NKG2D receptor (NKG2D), functions to eliminate cells emitting danger signals and dysregulation of this recognition process often leads to development of autoimmunity. The influences of JAK and NF-kb inhibition will be pursued, where, without being bound by theory, NKG2DL expression will be inhibited at the transcriptional level. INCB18424, a JAK1/JAK2 inhibitor (po/topical), is in Phase II Clinical Trials for Myeloproliferative/ Myelofibrosis Disorders and in Psoriasis.

**Table 9: Small Molecule Inhibitors of Jak1/Jak2**

| Small molecules | | | | |
|---|---|---|---|---|
| JAK1/JAK2 Inhibitor (INCB18424) | PTKi: ATP-Binding Cleft Competitive Antagonist | Interferon Signaling | Topical | Phase II/Psoriasis |

GWAS studies revealed a number of risk loci shared by with other forms of autoimmunity, such as rheumatoid arthritis (RA), type I diabetes (T1D), celiac disease (CeD), systemic lupus erythematosus (SLE), multiple sclerosis (MS) and psoriasis (PS), in particular, CTLA-4, IL2/IL2RA, IL21 and genes critical to Treg maintenance.

A genetic basis of AA provide avenues of exploration for therapies based on the underlying mechanisms of AA. Such therapies will focus not only on T cell subsets and mechanisms common to other forms of autoimmunity, but also on unique mechanisms that involve signaling pathways of Jak1/Jak2 and downstream effectors.

The origin of autoimmunity in AA can reside in the hair follicle itself. The studies herein are focused on defining putative danger signals in the hair follicle that contribute to the pathogenesis of AA. Pathogenic alleles that reside within the MHC, which can contribute to immune dysregulation driving the pathogenesis of AA, will also be identified. This can be due in part to the importance of antigen-presenting cells (APCs) in the presentation of danger signals as neoantigens to the immune system.

The mechanisms of Jak1/Jak2 signaling will be investigated, and the importance of Jak1/Jak2/Stat1/Stat2 interactions as drivers of disease induction will be evaluated. Pharmacologic approaches interrupting the Jak1/Jak2/Stat1/Stat2 signaling will be pursued.

C3H IFN-γ deficient mice are protected from AA development. It is shown that IFNs are capable of upregulating NKG2DL in cultured human dermal keratinocytes and fibroblasts. Further NKG2D-cross-linking induces IFN-γ production by innate NK, NKT and γδ T cells, thus creating a potential positive feedback loop, driving adaptive autoreactive immunity. Thus adoptive transfer and antibody depletion/blockade experiments will define the NKG2D-bearing cells involved.

Pharmacologic interventions aimed at interruption of the CD8 NKG2D axis will be examined. Interfering with the upregulation of NKG2DL or the activation of NKG2D-bearing cells are both rational approaches that will be pursued. It is shown that the IFN → JAK/STAT pathway can induce NKG2DL expression.

Whether the IFN→JAK/STAT pathway is pivotal to NKG2DL induction in normal and diseased skin will be assessed. The IFN JAK/STAT signaling pathway can be effectively inhibited with topical JAK1/JAK2 inhibitors, including an agent that has had early clinical success in Psoriasis. If the Type I interferon response underlies NKG2D ligand upregulation, JAK1/JAK2 inhibitors can block this induction, as well as block the IFN-gamma dependent components of the adaptive immune response.

### References

1. Safavi KH, Muller SA, Suman VJ, Moshell AN, Melton LJ, 3rd. Incidence of alopecia areata in Olmsted County, Minnesota, 1975 through 1989. Mayo Clin Proc 1995;70:628-33.
2. Jelinek JE. Sudden whitening of the hair. Bull N Y Acad Med 1972;48:1003-13.
3. Ito T, Ito N, Saatoff M, et al. Maintenance of hair follicle immune privilege is linked to prevention of NK cell attack. The Journal of investigative dermatology 2008; 128:1196-206.
4. Todes-Taylor N, Turner R, Wood GS, Stratte PT, Morhenn VB. T cell subpopulations in alopecia areata. J Am Acad Dermatol 1984; 11:216-23.
5. Gilhar A, Landau M, Assy B, et al. Transfer of alopecia areata in the human scalp graft/Prkdc(scid) (SCID) mouse system is characterized by a TH1 response. Clin Immunol 2003;106:181-7.
6. Gilhar A, Shalaginov R, Assy B, Serafimovich S, Kalish RS. Alopecia areata is a T-lymphocyte mediated autoimmune disease: lesional human T-lymphocytes transfer alopecia areata to human skin grafts on SCID mice. J Investig Dermatol Symp Proc 1999;4:207-10.
7. Zoller M, McElwee KJ, Engel P, Hoffmann R. Transient CD44 variant isoform expression and reduction in CD4(+)/CD25(+) regulatory T cells in C3H/HeJ mice with alopecia areata. The Journal of investigative dermatology 2002; 118:983-92.
8. McElwee KJ, Freyschmidt-Paul P, Hoffmann R, et al. Transfer of CD8(+) cells induces localized hair loss whereas CD4(+)/CD25(-) cells promote systemic alopecia areata and CD4(+)/CD25(+) cells blockade disease onset in the C3H/HeJ mouse model. The Journal of investigative dermatology 2005;124:947-57.
9. Ito T, Meyer KC, Ito N, Paus R. Immune privilege and the skin. Curr Dir Autoimmun 2008;10:27-52.
10. McDonagh AJ, Tazi-Ahnini R. Epidemiology and genetics of alopecia areata. Clin Exp Dermatol 2002;27:405-9.
11. van der Steen P, Traupe H, Happle R, Boezeman J, Strater R, Hamm H. The genetic risk for alopecia areata in first degree relatives of severely affected patients. An estimate. Acta Derm Venereol 1992;72:373-5.
12. Jackow C, Puffer N, Hordinsky M, Nelson J, Tarrand J, Duvic M. Alopecia areata and cytomegalovirus infection in twins: genes versus environment? J Am Acad Dermatol 1998;38:418-25.
13. Martinez-Mir A, Zlotogorski A, Gordon D, et al. Genomewide scan for linkage reveals evidence of several susceptibility loci for alopecia areata. Am J Hum Genet 2007;80:316-28.
14. Nakamura et al., 2008, Controlled Delivery of T-box21 Small Interfering RNA Ameliorates Autoimmune Alopecia (Alopecia Areata) in a C3H/HeJ Mouse Model, Am J Pathol., 172(3): 650-658.
15. Freyschmidt-Paul, K.J. McElwee, R. Hoffmann1, J.P. Sundberg, M.Vitacolonna, S. Kissling, M. Zöller, 2006, Interferon-gamma-deficient mice are resistant to the development of alopecia areata, Br J Dermatol., 155(3):515-21.
16. Gilhar A., Kam Y., Assy B. and Kalish R., 2005, Alopecia areata induced in C3H/HeJ mice by interferon-gamma: evidence for loss of immune privilege, Journal Invest. Dermatol., 124(1):288-9.
17. Wong et al., 2005, Inhibitors of the tyrosine kinase signaling cascade for asthma, Curr. Opin. Pharmacol., 5(3):264-71.
18. Mourich and Iverson, 2009, Splicing in the immune system: Potential targets for therapeutic intervention by antisense-mediated alternative splicing, Curr. Opinion Mol. Ther., 11(2): 124-132.
19. Jin et al., 2010, Variant of TYR and Autoimmunity Susceptibility Loci in Generalized Vitiligo, NEJM, 362:1686-1697.

### EXAMPLE 4

Specific autoimmune mechanisms underlying Alopecia Areata (AA) have remained obscure and therefore clinical investigation of AA has historically lagged behind other autoimmune diseases. Despite the large number of new biotherapeutics available for clinical evaluation in autoimmunity, intralesional or systemic steroids remain the standard of care and only two small randomized clinical trials have been published in the last decade. The insights provided by the discovery of GWAS genes provide a roadmap for closing this gap, by investigating GWAS-identified immunological pathways both for mechanistic and therapeutic relevance.

Identify Effective, Clinically-Relevant, Therapies in Mouse Models of Alopecia Areata. In the normal state, hair follicles (HF) represent an immuneprivileged (IP) sanctuary, expressing little or no classical HLA class I molecules. In active AA, the IP status is lost, since HLA class I expression and NKG2D ligand expression are markedly upregulated. Given the strong genetic association of NKG2D ligand loci with human AA, without being bound by theory, aberrant NKG2DL upregulation and persistent NKG2D activation on immune effector cells drives inflammatory AA initiation and progression. Without being bound by theory, in AA, as for Celiac Disease, IL-15 can drive CD8 T cell effector differentiation enabling promiscuous "NK-type" CD8 cytotoxicity. Indeed, the local HF IL-15/NKG2DL inflammatory signals are accompanied by a dense infiltrate of CD8⁺ T cells expressing NK markers, likely the critical AA immune effectors responsible for IFNγ production and HF cytotoxicity. These collective observations, made in both the human and mouse, invite therapeutic evaluation.

The therapeutic potential of blocking NK-like reprogramming and cytotoxicity by interfering with relevant cytokines (IFN-γ and relevant immunoreceptor signaling pathways (NKG2D). A graft model of alopecia in which all grafted C3H mice develop AA in a timely manner, within 12 weeks, will be used. This greatly accelerates drug screening since evaluation of prevention/reversal of spontaneous disease in C3H/HeJ is otherwise impractical given the late onset (>6 months of age) and low cumulative incidence (15%). Both small molecule approaches, that have the advantage of potential topical delivery, and biotherapeutic proteins (antibodies) that have superior biologic specificity, will be evaluated. The overarching goals are to use biotherapeutics to establish critical immune pathways that might be targeted by more clinically tenable, small molecule topical approaches.

Identify disease-associated AA biomarkers and their reversal with effective therapy in C3H mice. Approaches using GWAS and transcriptional profiling, for example, as well as approaches using immunostains and FACs of T cell subsets, for example, have been taken to identify several pathogenic AA biomarkers in the skin and blood of AA mice. These mouse studies will continue to inform developing a translational biomarker platform in human AA. In the skin, the studies in alopecic C3H/Hej mice demonstrated that IFN-induced genes are dramatically upregulated, including the chemokines CXCL9-11, which are likely responsible for recruitment of a dense infiltrate of HF-associated IFN-γ-producing CXCR3⁺ NKG2D-bearing CD8 T effectors. Spectratype data provide striking evidence that these circulating NKG2D⁺ CD8 T effectors are *bona fide* autoantigen-driven effector T cells that increase with disease progression. Temporal assessment of these serologic, cutaneous and cellular biomarkers in mice during treatment will enable one to identify dynamic, mechanistically important, prescient inflammatory biomarkers that predict therapeutic outcome and inform clinical biomarker discovery/utilization.

Preclinical validation of targeted approaches using AA human tissues ex vivo. To expedite clinical development of treatments that demonstrate "proof-of-concept" in the AA mouse model, human AA tissues will be used for target validation and correlative studies. The "crawl-outs" system will provide a convenient *ex vivo* human bioassay to validate that agents that reverse mouse AA translate to the human, effectively inhibiting human AA T cell survival/expansion/ function.

Just as the human DNA from thousands of alopecic subjects provided GWAS genes and unique insight into the human disease, human tissues, blood and skin from AA subjects will be used to continue to interrogate these genetically implicated immune pathways ultimately for therapeutic utility. Conceptually, the mouse model of alopecia areata this human pursuit, providing the pre-clinical testing grounds for therapeutic approaches in alopecia areata.

Autoimmune diseases affect an estimated 23 million individuals in the US (1) and although the prevalence of AA is not established, it is one of the most prevalent autoimmune diseases (2, 3). In 1999-2000, there were an estimated 2.4 million office visits for AA, half of which by patients in their 20s and 30s. AA causes significant disfigurement and psychological distress to affected individuals and carries one of the highest burdens among any skin diseases, particularly among children and adolescents whose self-image is so closely linked to their appearance (4). At present, the prognosis of AA is unpredictable and there is no definitive treatment. Current therapies include steroids and topical immune therapy, and induce durable remissions in only one third of patients (5, 6). Despite its high prevalence, a comprehensive Cochrane analysis assessment concluded that there are *no* evidence-based treatments for AA (7).

In stark contrast to other autoimmune conditions, research into the pathogenesis and the development of innovative therapies in AA has lagged far behind. In the era of targeted immunological therapies, only two clinical studies evaluating protein biotherapeutics in AA have been reported (8, 9) (both targeting T cell trafficking). In part this has been due to the limited understanding of specific AA immune pathways that would prompt evaluation of rational therapeutic approaches. Based on new understanding of the genetic and pathogenic basis of AA pre-clinical proof-of-concept data that will prompt clinical evaluation of rational therapeutic approaches in this disease will be obtained.

The GWAS (10) study revealed a number of risk loci shared by with other forms of autoimmunity, such as RA, T1D, celiac disease (CeD), SLE, MS and PS, in particular, CTLA4, IL2/IL2RA, IL21, NKG2D Ligands and genes critical to Treg function (Eos). The genetic commonality with RA, T1D, and CeD is especially noteworthy in light of the pathogenic significance of the expression of an NK ligand in the end organ (synovium, islet, gut and skin), and the involvement of the NKG2DL/NKG2D pathway in the pathogenesis of each of these three diseases (11, 14). One advantage of immunotherapeutic studies in the skin is the relative ease of access of the target organ. Thus, the studies herein examining the skin can provide important insight into NKG2D and IL-15 triggered CD8 cytotoxicity, IFN-triggered injury, etc., that impact the understanding of these other related human diseases in which the target organ is not accessible. Indeed positive studies in any one of these autoimmune diseases that share a common cause can serve as the basis for common treatments.

Specific autoimmune mechanisms underlying AA have remained obscure beyond the prevailing view that it is the result of T cell mediated attack of the hair follicle. In the normal state, hair follicles represent an immuneprivileged (IP) sanctuary (15-17), expressing little or no classical HLA class I molecules and instead express the inhibitory HLA ligands HLA-E and HLA-G, thus simultaneously avoiding immune recognition by class I restricted CD8 cells while negatively regulating NK cells, that might otherwise react to "missing self" (16, 18-21). In active AA, the IP status of the normal hair follicle is not operative, since HLA class I expression is markedly upregulated. Previous studies have noted both CD4 and CD8 T cell infiltrates surround the hair follicle in alopecic areas of skin in the mouse and human (22, 23). Indeed transfer of total T cells, but not B cells or sera, can transfer disease from an alopecic mouse or human to a normal WT (24) or SCID mouse (25), implicating effector T cells as the pathogenic cells. However molecular mediators of site-specific inflammation have not been previously identified. Guided by the GWAS findings and previous immunobiological studies, new insight into this common autoimmune disorder is provided, that will lead to the development of innovative, rationally targeted treatments to fill the unmet needs of patients with AA.

AA Immunological Pathways Identified by GWAS Studies The most highly-significant genetic loci found associated with alopecia areata are shown in **Table 10**: (the gray area denotes "nominally significant" genes). These seminal studies have been validated in a confirmatory study in a distinct population. Underlined genes are HF expressed genes while **bold** genes are genes expressed in immune cells. Overall, many susceptible loci are shared by other tissue-specific autoimmune states, with Type I diabetes exhibiting the most striking overlap (right column). The commonality with RA, T1D and CeD is particularly noteworthy, as NKG2D has been shown to have a significant role in the pathogenesis of each of these three diseases (11-14). Thus insight in any one of these disease/model systems is likely to shed light on common pathogenic pathways shared by multiple autoimmune states. The most highly significant alleles identified (other than HLA) were four NKG2D ligands (UBLP-3/UBLP-6/MICA/MICB in **Bold** *italics*) and one inhibitory ligand (HLA-G). The NKG2D receptor (NKG2D), functions to eliminate cells emitting danger signals and dysregulation of this recognition process often leads to development of autoimmunity (26). Other HF expressed genes that contribute to risk include HLA, antigen processing genes (TAP) and PRDX/STX17.

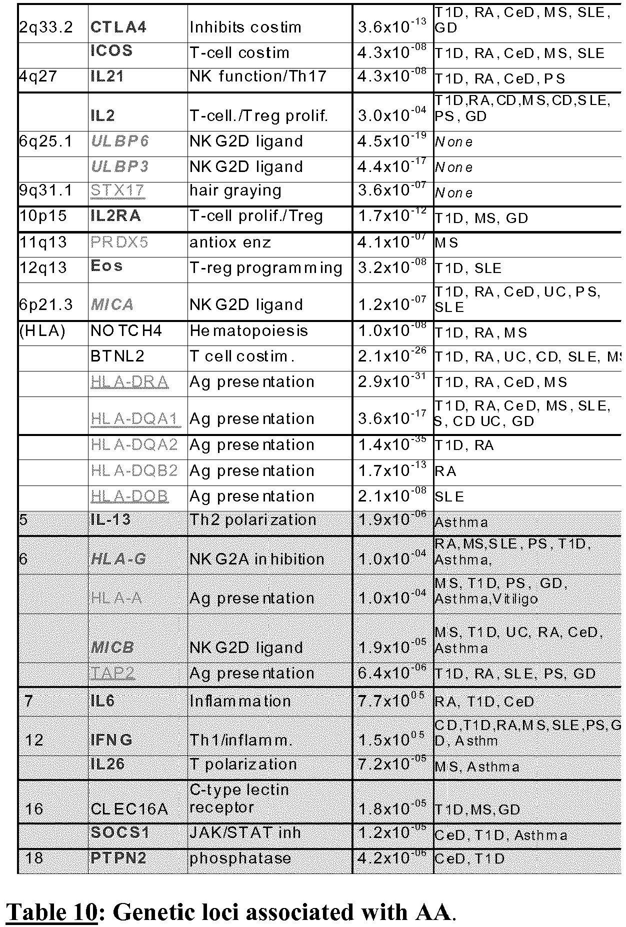

IFN-γ production by activated NKG2D-bearing CD8 effectors can reverse immunologic privilege and perpetuate the inflammatory loop.

IFN-γ related pathways can be undermined with existing therapeutics under clinical investigation, expediting the path to translation. Over the last ten years, several small molecule inhibitors of protein tyrosine kinases (PTKi) have been successfully developed clinically both for oral and topical delivery. PTKi approaches will be pursued to blocking CD8 mediated inflammatory responses in AA, namely inhibition of JAK/STAT signaling, responsible for cytokine responsiveness, downstream of the CD8 effector cytokine IFN-γ.

### Identify clinically-relevant therapies targeting effector T cell responses in alopecic mice.

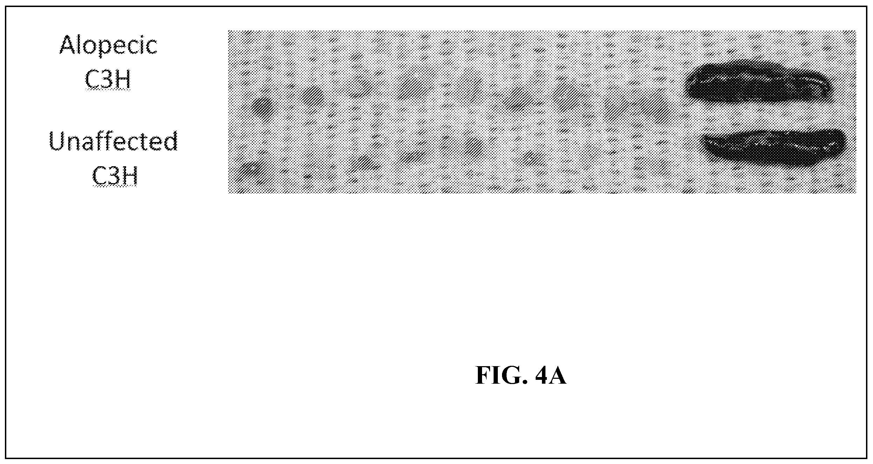

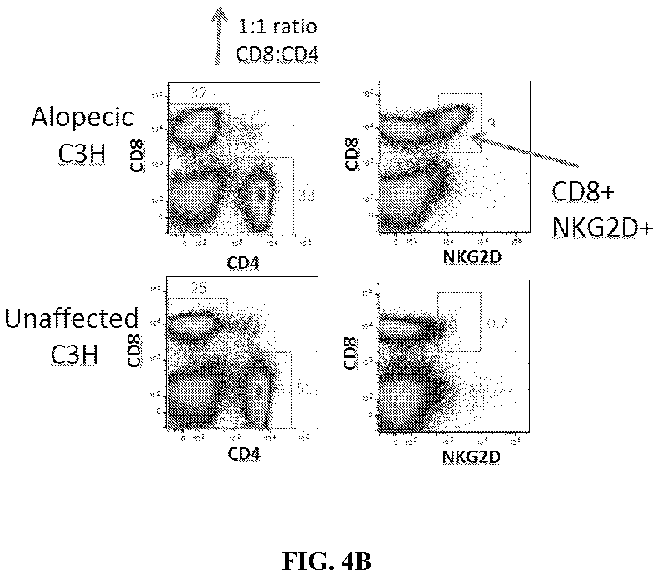

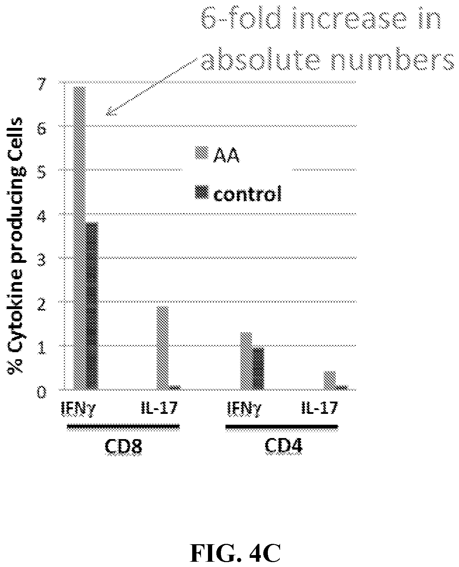

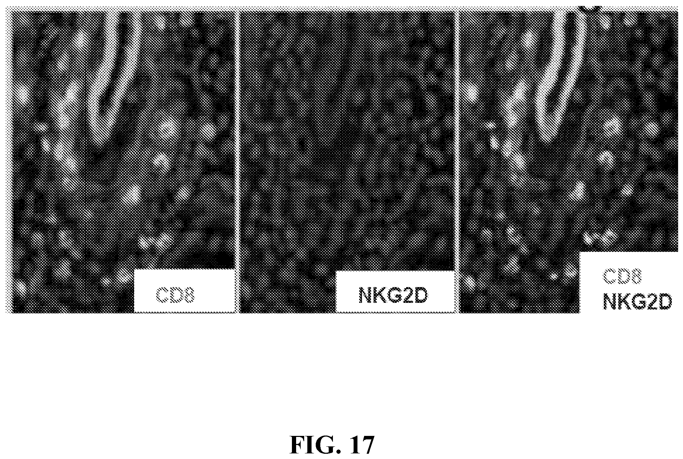

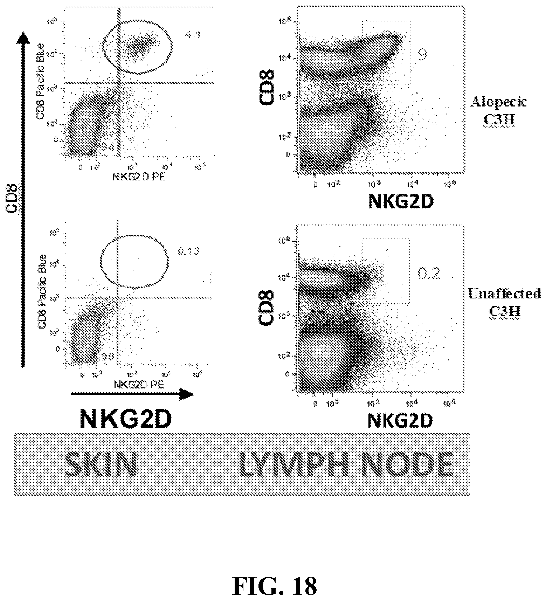

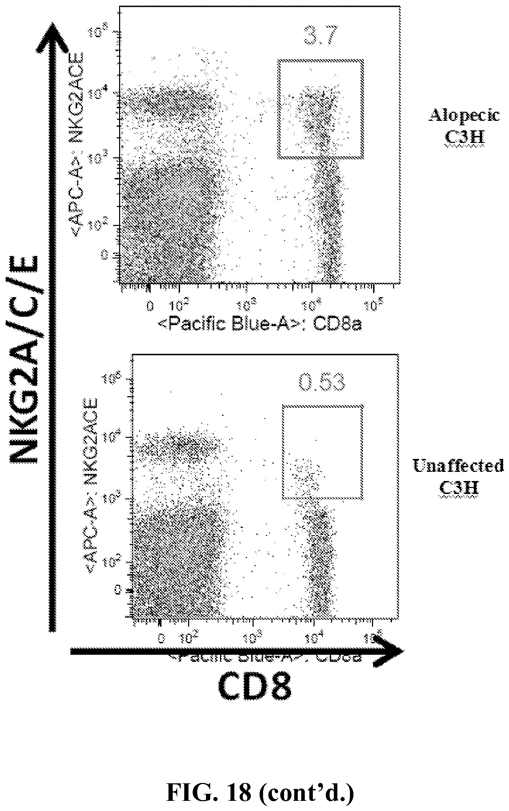

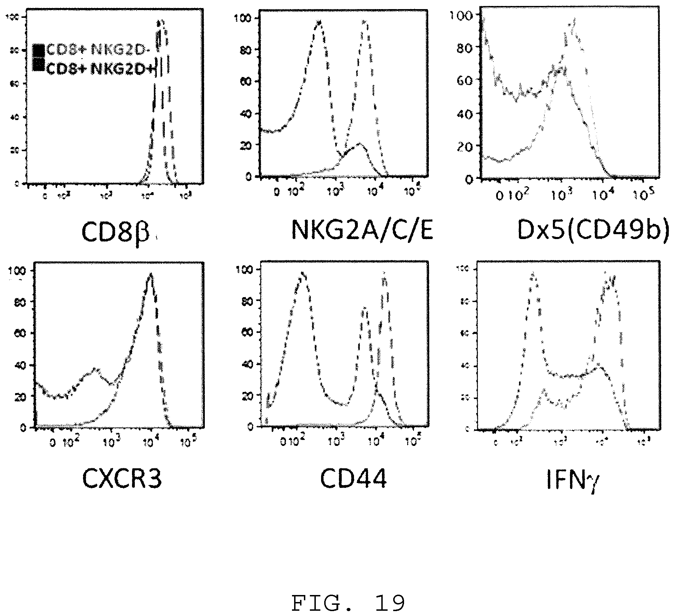

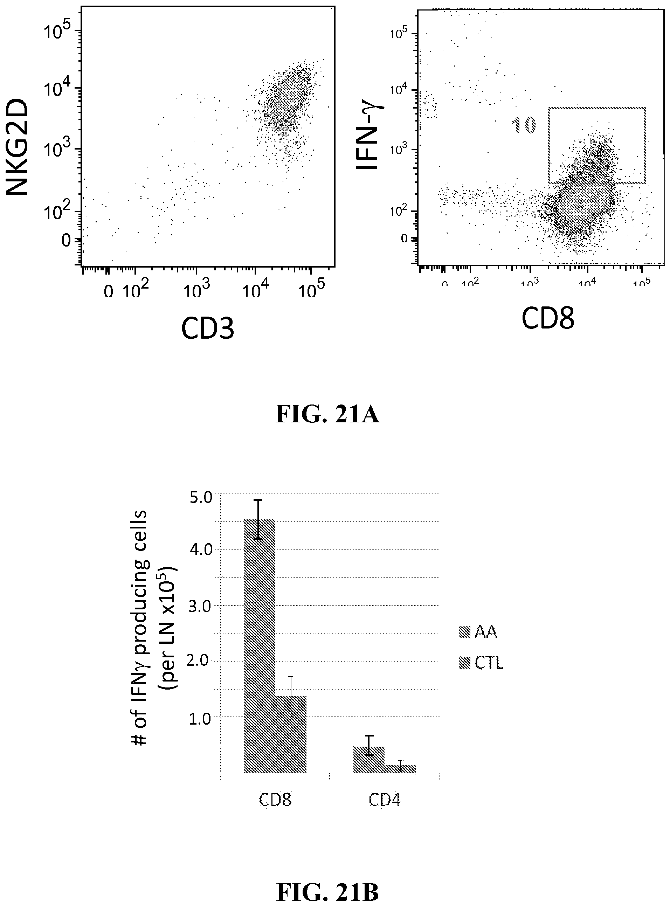

Using immunostaining and flow cytometric analysis of total cellular populations from AA skin, CD8⁺NKG2D⁺ T cells were found infiltrating the hair follicle (**FIG. 17**) and expanded in AA but not normal skin (**FIG. 18** and **FIG. 4A**). Furthermore AA mice exhibit cutaneous lymphadenopathy, at least in part due to dramatic systemic expansion of the CD8⁺NKG2D⁺ population, in both the AA cutaneous lymph node and in the blood, representing fully 6+/-1% of total LN cells are 2.4+/-1.3% of total PBMCs, each more than ten-fold higher than that seen in normal C3H mice. These CD8+NKG2D⁺ T cells are potently cytotoxic against HF dermal sheath cells (**FIG. 21**). Further immunophenotypic analysis of CD8⁺NKG2D⁺ T cells revealed that this population expressed the T memory marker CD44⁺ and multiple "NK markers", including DX5, NKG2A/C/E, and NKp46 (**FIG. 19**).

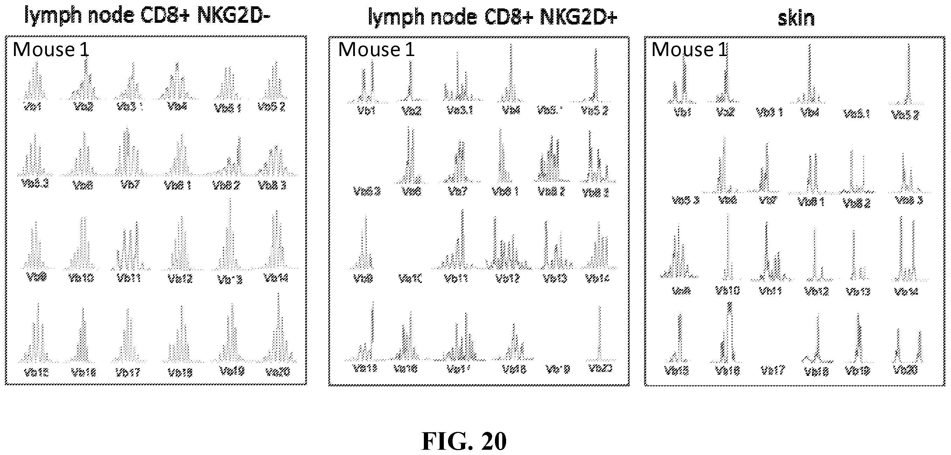

To provide proof that the circulating CD8⁺NKG2D⁺ population represented the bona fide "AA-specific" effector population, without being bound by theory, the CD8⁺ NKG2D⁺ T cells found in the lymph node can express a similar TCR repertoire to the total T cells found infiltrating AA skin. **FIG. 20** shows that CD8⁺NKG2D⁻ lymph node populations exhibit a normal distribution of CDR lengths, a pattern reflective of an unrestricted polyclonal T cell population. In contrast, the TCR repertoire of skin T cells are highly restricted with nearly all Vβ family members dominated by a small number of CDR lengths, indicative of an oligoclonal population of antigen-driven T cells. CD8⁺NKG2D⁺ T cells from the cutaneous lymph node cells are also highly restricted and show a strikingly similar pattern with total cutaneous T cells.

These data confirm that CD8⁺NKG2D⁺ T cells contain most of the oligoclonal T cell populations found in the skin. Moreover the evidence for the expansion of common oligoclonal T cells in the skin and lymph node argues against the possibility that these LN populations (or even markers) are expanded/activated as a reactive process to inflammatory events. For instance, Treg cells (CD4⁺CD25⁺FoxP3⁺) are also increased in numbers in AA lymph nodes, however, this is likely a reactive process, since their spectratype is diverse and unlike those present in the skin. Note that some TCR/clones are found in skin but not in draining cutaneous lymph nodes, e.g., Vβ10. These likely include CD4⁺ T cell populations found in AA skin but not present in the *sorted* CD8+ LN fraction. These data establish that the CD8⁺NKG2D⁺ T cells are the pathogenic NKG2D⁺ effectors in mouse AA, establishing a parallel of the mouse model to human AA.

### Pre-clinical AA model to establish proof of concept for therapeutic approaches in vivo

C3H/HeJ Skin Graft Model: The C3H/HeJ mouse model for alopecia areata has been studied since it was first reported this model in 1994 (29, 30, 31). While other mouse models for alopecia areata were found (32), this model remains the most studied and used in the field today. Having done a variety of drug trials with this model over the years (33), a variety of protocols have been optimized (34).

Full thickness skin grafting from alopecic C3H/HeJ mice to 2-3 month-old unaffected C3H/HeJ female mice results in patchy alopecia within 10 weeks of grafting in all recipients, progressing to generalized alopecia at 20 weeks. A variety of comprehensive and efficient histological scoring systems are employed building on the traditional training of histopathologists where the adjective used in descriptions (normal, 0; mild, 1; moderate, 2; severe, 3; and extreme, 4) are converted to a numerical value for importation into a relational database (Mouse Disease Information System [or MoDIS]: http://research.jax.org/faculty/sundberg/registration.php) (35, 36). This database is exportable via Excel for importation into a statistical analysis or other software program. Alopecia areata is graded based on severity, location of inflammatory cells, types and relative numbers of inflammatory cells (granulocytes, mast cells, lymphocytes), follicular dystrophy, etc. Molecular RNA and protein biomarkers will be incorporated in this panel, and generation and analysis of this multiple variable database will be done.

Protocol#1: In the prevention setting, recipient mice will be treated beginning the first week after grafting.

Protocol#2: For treatment, grafted mice with early-established AA will be treated to reverse AA progression.

Agents will first be examined in the prevention model. Agents that successfully prevent AA will then move forward for secondary evaluation in the setting of established disease, while those that fail the first test will be discarded, permitting entry of other innovative approaches. The following are the initial therapeutic approaches, both specific biologics and small molecules, chosen for preclinical evaluation.

Small molecule JAK protein tryosine kinase inhibitors (PTKis) that target activation pathways involved in the T effector response will be tested. These PTKis are already in Phase III clinical trials easing translation and potential clinical development of "NK-type" T cell-targeted topicals.

### Therapeutic interventions with small molecule PTKi interventions

Topical delivery has obvious advantages in limiting systemic exposures and toxicities. Pre-clinical and clinical studies have demonstrated that small molecule PTKi delivery in cream can overcome the skin barrier and achieve effective local concentrations in the dermis with limited systemic exposure (78). Key PTKs pivotal to effector T responses in AA will be targeted, namely the cytokine IFNγ.

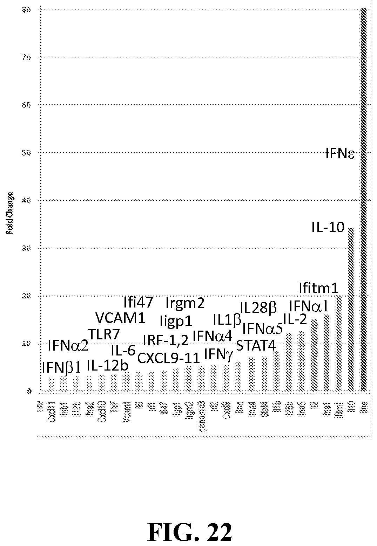

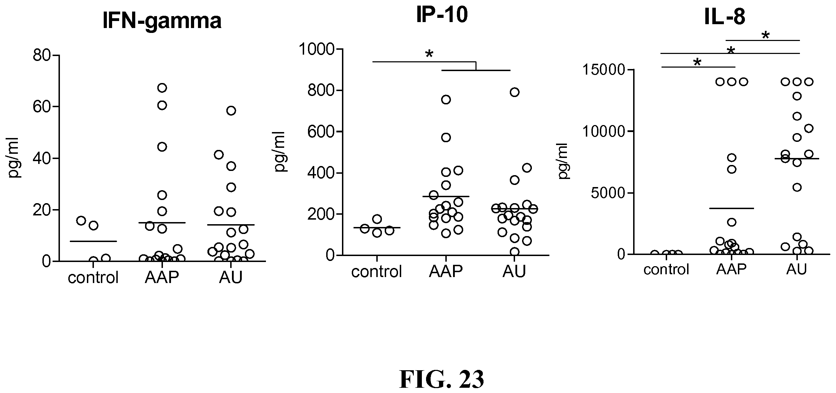

Targeting IFNs with Jak1/2 Inhibitors: Interferons are an attractive therapeutic target in AA since they likely participate at several steps in the inflammatory response; including eliminating HF immunologic privilege and inducing the cellular inflammatory response. Interferons upegulate several relevant pro-inflammatory molecules in the hair follicle end organ, including NKG2DL, adhesion molecules (e.g., ICAM-1), antigenic processing/presentation (TAP1/2, LMP, proteosome, MHCI and II) and moreover drive the immune effector response (increased Th1-type responses, DC activation and IFN-γ-mediated cytotoxicity). In the C3H-HeJ mouse model of AA, IFN-γ is required for pathogenesis (89, 90) and administration of interferon-y accelerates disease (91). Importantly, administration of IFN-γ neutralizing antibodies reverses AA pathogenesis in the C3H-HeJ mouse (92, 93). Likewise, in the human, alopecia areata has been noted in several series as a side-effect of Type I interferon therapy (94 -104) and transcriptional profiles of human AA skin have noted a Type I IFN response in lesional biopsies (105) and Th1 skewing and elevated IFN-response cytokines/chemokines in the peripheral blood (**FIGS. 22****,** **23**) (106-110) and reviewed in (111). T cells obtained from AA skin biopsy explants were uniformly CD8⁺NKG2D+ cells and when stimulated produced IFN-γ (**FIG. 21A**) but not IL-4 or IL-17. In stark contrast, the majority of T cells obtained from normal skin explants are CD4⁺ (112, 113). The situation is similar in AA mice, IFN-γ producing CD8 T cells are expanded in the cutaneous lymph node **(****FIGS. 19****,** **21****).** In the skin, our transcriptional profiling studies of total skin isolated from lesional vs. non-lesional C3H skin has identified an interferon response as the dominant signature with 17 of the top 20 upregulated genes being interferon response genes (**FIG. 22**). Indeed, IFNs upregulate NKG2DL expression on hair follicle targets, closing an IFN-mediated pathogenic circle, in which IFN-γ made by CD8 effectors drive the HF NKG2D ligands that lead to CD8⁺NKG2D⁺ activation.

Clinical JAK1/2 inhibitors: Both blocking antibodies (114, 115) and small molecule PTKis are under clinical development targeting the IFN response in autoimmunity. IFN induced STAT1/2 activation is mediated by Jak1/Jak2/Tyk kinases and clinical data have emerged using oral Jak1/Jak2 PTKis in several autoimmune conditions, including RA and psoriasis. Topical therapies have the added advantage of an improved therapeutic index limiting the potential for systemic immunosuppression. INCB018424 inhibits Jak1 and Jak2 (IC₅₀=1nM) and is the only *topical* JAK inhibitor (78) currently in clinical investigation and its safety profile to date has been acceptable. Proof-of-concept with oral delivery has been demonstrated in a Jak-dependent disease (myelofibrosis) (116) and in a dermatological disease driven by local cytokine production (psoriasis models) (78). In psoriasis subjects, topical INCB018424 induced rapid clinical responses and normalization of cutaneous Th1/Th17 cytokine responses (unpublished data from clinicaltrials.gov/ct2/show/NCT00820950). Taken together, topical INCB018424 is a safe intervention predicted to have efficacy in Alopecia Areata, an inflammatory state dominated by an IFN-signature.

Approach: Protocol #1: Systemic delivery of INCB018424: Twice daily (90mg/kg) orogastric gavage provides good systemic exposure given the t_{1/2} half-life of 3-6 hours in the rodent. In this manner we can assess if JAK1/2 inhibition prevents AA progression.

Protocol #2: Topical delivery of INCB018424 (1.5%): Reversal of patchy AA by topical JAK1/JAK2 inhibitor. Daily topical treatment (78) will begin to new affected patchy areas in grafted mice that begin to emerge 6-10 weeks after grafting. Topical treatment can be restricted to specific areas of mouse skin by appropriate bandaging to prevent systemic exposures from grooming, licking etc. Importantly, by treating affected areas we can assess the clinically relevant question of whether topical exposure can reverse AA progression.

### Identification of murine biomarkers of AA disease activity that inform clinical investigation

The identification of immunological events that accompany treatment outcome in these pre-clinical studies, is important, both to validate the proposed mechanism of action and to provide useful biomarkers that will inform potential clinical evaluation of these same therapies in AA patients. Since in clinical evaluation, biospecimens will be limited to blood and skin samples, the focus will be on mouse biomarkers in the mouse that are easily sampled from these sites. Approaches using GWAS and transcriptional profiling, for example, as well as approaches using immunostains and FACs of T cell subsets, for example, have been taken to identify several pathogenic biomarkers in the skin and blood of AA mice. The studies in alopecic C3H/HeJ mice have already identified several skin biomarkers upregulated in AA, such as IFN-induced genes (**FIG. 22**). Spectratype data (**FIG. 20**) has provided striking evidence that these circulating NKG2D⁺ CD8 T effectors are *bona fide* autoantigen-driven effector T cells. Temporal assessment of these serologic, cutaneous and cellular biomarkers in mice, with and without treatment, will enable one to identify dynamic, mechanistically important, prescient inflammatory biomarkers that predict therapeutic outcome and inform clinical biomarker discovery/utilization.

Cellular biomarkers of circulating immune effectors in AA: "NK-type" CD8 T cells are expanded in the skin, blood and lymph node in AA mice and are a valuable tool to investigate pathogenesis. Moreover, the cellular subset appears directly relevant to the human disease.

*Spectratype-based imunomonitoring:* NK-type CD8 T cells will be identified in AA mice in the circulation by flow cytometry of 50 µl of whole blood (**FIG. 18**). The total numbers and spectratype of this CD8⁺NKG2D⁺ sorted T cell subset, will be assessed every three weeks after grafting to monitor the evolution of clonal dominance/epitope spreading within a single mouse. Spectratype analysis will be used to confirm that immunophenotypic loss of CD8⁺NKG2D⁺ T cells is due to treatment-related elimination of the circulating alopecic T cell clones. This non-invasive immunomonitoring approach will be used to follow mice on all treatments. For instance, 3 weeks of treatment treatment with the JAK1/2 inhibitor INCB018424 did not. Without being bound by theory, JAK1/2 inhibitors are expected to abrogate the downstream inflammatory consequences of T cell derived effector cytokines (IFN-γ, IL-17) in the target tissue.

Immune "proteomic" signatures in serum: Cytokines and chemokines in the serum are a reflection of the inflammatory milieu in the target organ. The data in the human demonstrate elevations of several cytokines and chemokine some of which correlate with disease activity (for instance IL-8), extending the observations of prior studies (106, 107, 109). Consistent with our overall findings implicating IL-15 and IFN-γ in AA pathogenesis, it is seen that IFN-γ and the well-known IFN-γ inducible chemokine IP-10 (CXCL10) are upregulated in human AA sera. Functionally relevant inflammatory cytokine/chemokine biomarkers will be identified in the sera from AA mice using a multianalyte approach (Luminex platform). The advantage of biomarker development in the mouse, besides genetic homogeneity, is the relative ease of longitudinal studies to identify biomarkers that occur early or late in disease progression and conversely with treatment response. At the RNA level, C3H mouse skin IFN microarrays demonstrated that both Type I IFNs (IFNα₁₋₃, ε□ and type II IFN (IFN-γ□ were upregulated as well several IFN-associated chemokines (CXCL9-11, CCL5). Cytokine/chemokines production will be examined in sera using custom multianalyte Luminex assay (R&D), comprehensively addressing serum levels of 9 chemokines (CCL2-5, CCL11, CXCL5, CXCL9, CXCL10, CXCL11) and 14 cytokines (IFNα, IFNγ, IL-1, IL-2, IL-4, IL-5, IL-6, IL-10, IL-12, IL-13, IL-15, IL-17, TNF, GM-CSF). We will include antibody pairs to detect serum levels of NKG2DLs (117) and IL-15Rα.

Transcriptional profiling of skin: In human AA, published studies of small series of patients have noted a Type I IFN response in lesional biopsies (105) and Th1 skewing and elevated IFN-response cytokines/chemokines in the peripheral blood (106-110) and reviewed in (111). These data are mirrored in the analysis of the C3H mouse. Transcriptional profiling of RNA isolated from alopecic mouse skin revealed a dominant IFN response. 18 of the top 21 upregulated genes in AA skin were IFN response genes, which was confirmed by real-time PCR (**FIG. 22**). The upregulation of the IFN-response genes CXCL9-11 are notable since the CD8⁺NKG2D⁺ cells also uniformly express their shared chemokine receptor, CXCR3, a receptor upregulated on short-lived immune effectors (118-120). These chemokines are also upregulated in the sera of human AA subjects and thus can be a functionally relevant AA skin biomarker in the skin (mRNA) and in the blood (protein). Work in this area will continue, building a database of skin RNA signatures collected before and during ongoing treatment, that can be used to tailor treatment plans and as early indicators of treatment response. Towards these goals, parallel studies will be performed in the mouse with both intralesional steroids and CTLA4-Ig, as well as with therapeutic interventions, to provide a transcriptional profile database that will be useful in cross-referencing the human treatment studies (comparative genomic analysis between studies and for potential clinical development of more novel approaches).

### Human Subjects

Skin biopsies from the scalp will be collected from a minimum of 100 Alopecia Areata patients and approximately 100 controls. The subject population will consist of AA patients and unaffected control subjects receiving hair transplantation. Approval for collection of these samples underwent expedited review/exemption since they are discarded tissue. A protocol for collection of scalp biopsies from AA patients is in preparation. A minimum of 100 AA subjects will be recruited, but collection of greater than 100 will be beneficial and increase the power of this approach.

In order to achieve maximum homogeneity among samples for generating the expression interactome, hair follicles will be collected exclusively from middle aged Caucasian male subjects with AA, since the matching control donors are most commonly from this group. Therefore, despite the fact that AA shows no ethnic or gender predilection, the AA patients will be selected to match in age and gender to the controls for the purpose of this study (middle aged Caucasian males). Patients with active disease (ie: with some remaining hair follicles) will also be selected so that they can be efficiently microdissected.

### Vertebrate Animals

Young C3H/HeJ mice (2-3 months) and retired breeders showing visible hair loss will be acquired from Jackson Labs. The animals will be kept under standard conditions in the animal facilities.

The mice will be administered drugs or antibodies by either systemic injections or topically. For topical applications, the mice skin will be shaved on the dorsal surface with electric clippers. One week after shaving, mice will receive topical application of the chemicals dissolved in acetone or 10% v/v in propylene glycol as vehicles. Injections will be given either intraperitonealy or subcutaneously. The dosage as well as frequency of administration will be followed as per established protocols. The mice will be monitored daily for signs of distress throughout the duration of treatment.

### References

1. NIH Biennial Report of the Director, National Institutes of Health Fiscal Years 2006 & 2007, Summary of Activities by Disease Category, Autoimmune Diseases. (2007).
2. Tobias, L. A Briefing Report on Autoimmune Diseases and AARDA: Past, Present, and Future, (2010).
3. Safavi, K.H., Muller, S.A., Suman, V.J., Moshell, A.N. & Melton, L.J., 3rd Incidence of alopecia areata in Olmsted County, Minnesota, 1975 through 1989. Mayo Clin Proc 70, 628-633 (1995).
4. Wasserman, D., Guzman-Sanchez, D.A., Scott, K. & McMichael, A. Alopecia areata. Int J Dermatol 46, 121-131 (2007).
5. Alkhalifah, A., Alsantali, A., Wang, E., McElwee, K.J. & Shapiro, J. Alopecia areata update: part II. Treatment. J Am Acad Dermatol 62, 191-202, quiz 203-194.
6. Alkhalifah, A., Alsantali, A., Wang, E., McElwee, K.J. & Shapiro, J. Alopecia areata update: part I. Clinical picture, histopathology, and pathogenesis. J Am Acad Dermatol 62, 177-188, quiz 189-190.
7. Delamere, F.M., Sladden, M.M., Dobbins, H.M. & Leonardi-Bee, J. Interventions for alopecia areata. Cochrane Database Syst Rev, CD004413 (2008).
8. Kaelin, U., Hassan, A.S., Braathen, L.R. & Yawalkar, N. Treatment of alopecia areata partim universalis with efalizumab. J Am Acad Dermatol 55, 529-532 (2006).
9. Strober, B.E. et al. Alefacept for severe alopecia areata: a randomized, double-blind, placebo-controlled study. Arch Dermatol 145, 1262-1266 (2009).
10. Petukhova, L. et al. Genome-wide association study in alopecia areata implicates both innate and adaptive immunity. Nature 466, 113-117 (2010).
11. Abadie, V., Sollid, L.M., Barreiro, L.B. & Jabri, B. Integration of genetic and immunological insights into a model of celiac disease pathogenesis. Annu Rev Immunol 29, 493-525 (2011).
12. Champsaur, M. & Lanier, L.L. Effect of NKG2D ligand expression on host immune responses. Immunol Rev 235, 267-285 (2010).
13. Van Belle, T.L. & von Herrath, M.G. The role of the activating receptor NKG2D in autoimmunity. Mol Immunol 47, 8-11 (2009).
14. Caillat-Zucman, S. How NKG2D ligands trigger autoimmunity? Hum Immunol 67, 204-207 (2006).
15. Paus, R., Ito, N., Takigawa, M. & Ito, T. The hair follicle and immune privilege. J Investig Dermatol Symp Proc 8, 188-194 (2003).
16. Paus, R., Nickoloff, B.J. & Ito, T. A 'hairy' privilege. Trends Immunol 26, 32-40 (2005).
17. Paus, R., Slominski, A. & Czarnetzki, B.M. Is alopecia areata an autoimmune-response against melanogenesis-related proteins, exposed by abnormal MHC class I expression in the anagen hair bulb? Yale J Biol Med 66, 541-554 (1993).
18. Gilhar, A., Paus, R. & Kalish, R.S. Lymphocytes, neuropeptides, and genes involved in alopecia areata. J Clin Invest 117, 2019-2027 (2007).
19. Ito, T. et al. Collapse and restoration of MHC class-I-dependent immune privilege: exploiting the human hair follicle as a model. Am J Pathol 164, 623-634 (2004).
20. Ito, T. et al. Maintenance of hair follicle immune privilege is linked to prevention of NK cell attack. J Invest Dermatol 128, 1196-1206 (2008).
21. Ito, T., Meyer, K.C., Ito, N. & Paus, R. Immune privilege and the skin. Curr Dir Autoimmun 10, 27-52 (2008).
22. Sundberg, J.P., Boggess, D., Montagutelli, X., Hogan, M.E. & King, L.E., Jr. C3H/HeJ mouse model for alopecia areata. J Invest Dermatol 104, 16S-17S (1995).
23. Sundberg, J.P., Oliver, R.F., McElwee, K.J. & King, L.E., Jr. Alopecia areata in humans and other mammalian species. J Invest Dermatol 104, 32S-33S (1995).
24. McElwee, K.J. et al. Transfer of CD8(+) cells induces localized hair loss whereas CD4(+)/CD25(-) cells promote systemic alopecia areata and CD4(+)/CD25(+) cells blockade disease onset in the C3H/HeJ mouse model. J Invest Dermatol 124, 947-957 (2005).
25. Gilhar, A. et al. Transfer of alopecia areata in the human scalp graft/Prkdc(scid) (SCID) mouse system is characterized by a TH1 response. Clin Immunol 106, 181-187 (2003).
26. Van Belle, T.L. & von Herrath, M.G. The role of the activating receptor NKG2D in autoimmunity. Molecular Immunology 47, 8-11 (2009).
29. Sun, J., Silva, K.A., McElwee, K.J., King, L.E., Jr. & Sundberg, J.P. The C3H/HeJ mouse and DEBR rat models for alopecia areata: review of preclinical drug screening approaches and results. Exp Dermatol 17, 793-805 (2008).
30. Sundberg, J.P., Cordy, W.R. & King, L.E., Jr. Alopecia areata in aging C3H/HeJ mice. J Invest Dermatol 102, 847-856 (1994).
31. Sundberg, J.P., Cordy, W.R. & King, L.E. Alopecia areata in aging C3H/HeJ mice. J Invest Dermatol 102, 847-856 (1994).
32. McElwee, K., Boggess, D., Miller, J., King, L. & Sundberg, J. Spontaneous alopecia areata-like hair loss in one congenic and seven inbred laboratory mouse strains. J Invest Dermatol Symp Proc 4, 202-206 (1999).
33. Sun, J., Silva, K.A., McElwee, K.J., King, L.E. & Sundberg, J.P. The C3H/HeJ mouse and DEBR rat models for alopecia areata: preclinical drug screening tools Exp Dermatol 17, 793-805 (2008).
34. Sundberg, J.P., Silva, K.A., McPhee, C. & King, L.E. Skin diseases in laboratory mice: approaches to drug target identification and efficacy screening. Methods Mol Biol 602, 193-213 (2010).
35. Sundberg, B.A., Schofield, P.N., Gruenberger, M. & Sundberg, J.P. A data capture tool for mouse pathology phenotyping. Vet Pathol 46, 1230-1240 (2009).
36. Sundberg, J.P., Sundberg, B.A. & Schofield, P.N. Integrating mouse anatomy and pathology ontologies into a diagnostic/phenotyping database: tools for record keeping and teaching. Mammalian Genome 19, 413-419 (2008).
74. Dandekar, A.A., O'Malley, K. & Perlman, S. Important roles for gamma interferon and NKG2D in gammadelta T-cell-induced demyelination in T-cell receptor beta-deficient mice infected with a coronavirus. J Virol 79, 9388-9396 (2005).
78. Fridman, J.S. et al. Preclinical evaluation of local JAK1 and JAK2 inhibition in cutaneous inflammation. J Invest Dermatol 131, 1838-1844 (2011).
81. Karaman, M.W. et al. A quantitative analysis of kinase inhibitor selectivity. Nat Biotechnol 26, 127-132 (2008).
89. Freyschmidt-Paul, P. et al. Interferon-gamma-deficient mice are resistant to the development of alopecia areata. Br J Dermatol 155, 515-521 (2006).
90. Hirota, R. et al. Induction of hair regrowth in the alopecia site of IFN-gamma knockout mice by allografting and IFN-gamma injection into the transplantation site. J Interferon Cytokine Res 23, 433-439 (2003).
91. Gilhar, A., Kam, Y., Assy, B. & Kalish, R.S. Alopecia areata induced in C3H/HeJ mice by interferon-gamma: evidence for loss of immune privilege. J Invest Dermatol 124, 288-289 (2005).
92. Skurkovich, S., Korotky, N.G., Sharova, N.M. & Skurkovich, B. Treatment of alopecia areata with anti-interferon-gamma antibodies. J Investig Dermatol Symp Proc 10, 283-284 (2005).
93. Nakamura, M., Jo, J., Tabata, Y. & Ishikawa, O. Controlled delivery of T-box21 small interfering RNA ameliorates autoimmune alopecia (Alopecia Areata) in a C3H/HeJ mouse model. Am J Pathol 172, 650-658 (2008).
94. Mistry, N., Shapero, J. & Crawford, R.I. A review of adverse cutaneous drug reactions resulting from the use of interferon and ribavirin. Can J Gastroenterol 23, 677-683 (2009).
95. Maticic, M., Poljak, M., Lunder, T., Rener-Sitar, K. & Stojanovic, L. Lichen planus and other cutaneous manifestations in chronic hepatitis C: pre- and post-interferon-based treatment prevalence vary in a cohort of patients from low hepatitis C virus endemic area. J Eur Acad Dermatol Venereol 22, 779-788 (2008).
96. Kartal, E.D., Alpat, S.N., Ozgunes, I. & Usluer, G. Adverse effects of high-dose interferon-alpha-2a treatment for chronic hepatitis B. Adv Ther 24, 963-971 (2007).
97. Kartal, E.D., Alpat, S.N., Ozgunes, I. & Usluer, G. Reversible alopecia universalis secondary to PEG-interferon alpha-2b and ribavirin combination therapy in a patient with chronic hepatitis C virus infection. Eur J Gastroenterol Hepatol 19, 817-820 (2007).
98. Demirturk, N., Aykin, N., Demirdal, T. & Cevik, F. Alopecia universalis: a rare side effect seen on chronic hepatitis C treatment with peg-IFN and ribavirin. Eur J Dermatol 16, 579-580 (2006).
99. Yu, M.L. et al. A randomised study of peginterferon and ribavirin for 16 versus 24 weeks in patients with genotype 2 chronic hepatitis C. Gut 56, 553-559 (2007).
100. Taliani, G. et al. Reversible alopecia universalis during treatment with PEG-interferon and ribavirin for chronic hepatitis C. J Chemother 17, 212-214 (2005).
101. Radny, P. et al. Alopecia areata induced by adjuvant treatment with alpha-interferon in malignant melanoma? Dermatology 209, 249-250 (2004).
102. Agesta, N., Zabala, R. & Diaz-Perez, J.L. Alopecia areata during interferon alpha-2b/ribavirin therapy. Dermatology 205, 300-301 (2002).
103. Kernland, K.H. & Hunziker, T. Alopecia areata induced by interferon alpha? Dermatology 198, 418-419 (1999).
104. Lang, A.M., Norland, A.M., Schuneman, R.L. & Tope, W.D. Localized interferon alfa-2b-induced alopecia. Arch Dermatol 135, 1126-1128 (1999).
105. Ghoreishi, M., Martinka, M. & Dutz, J.P. Type 1 interferon signature in the scalp lesions of alopecia areata. Br J Dermatol.
106. Barahmani, N. et al. Serum T helper 1 cytokine levels are greater in patients with alopecia areata regardless of severity or atopy. Clin Exp Dermatol (2009).
107. Kuwano, Y. et al. Serum chemokine profiles in patients with alopecia areata. Br J Dermatol 157, 466-473 (2007).
108. Arca, E., Musabak, U., Akar, A., Erbil, A.H. & Tastan, H.B. Interferon-gamma in alopecia areata. Eur J Dermatol 14, 33-36 (2004).
109. Benoit, S., Toksoy, A., Goebeler, M. & Gillitzer, R. Selective expression of chemokine monokine induced by interferon-gamma in alopecia areata. J Invest Dermatol 121, 933-935 (2003).
110. Hoffmann, R. et al. Cytokine mRNA levels in Alopecia areata before and after treatment with the contact allergen diphenylcyclopropenone. J Invest Dermatol 103, 530-533 (1994).
111. Gregoriou, S. et al. Cytokines and other mediators in alopecia areata. Mediators Inflamm 2010, 928030.
112. Clark, R.A. et al. The vast majority of CLA+ T cells are resident in normal skin. J Immunol 176, 4431-4439 (2006).
113. Clark, R.A. et al. A novel method for the isolation of skin resident T cells from normal and diseased human skin. J Invest Dermatol 126, 1059-1070 (2006).
114. Bissonnette, R. et al. A randomized, double-blind, placebo-controlled, phase I study of MEDI-545, an anti-interferon-alfa monoclonal antibody, in subjects with chronic psoriasis. JAm Acad Dermatol 62, 427-436.
115. Hommes, D.W. et al. Fontolizumab, a humanised anti-interferon gamma antibody, demonstrates safety and clinical activity in patients with moderate to severe Crohn's disease. Gut 55, 1131-1137 (2006).
116. Verstovsek, S. et al. Safety and efficacy of INCB018424, a JAK1 and JAK2 inhibitor, in myelofibrosis. N Engl J Med 363, 1117-1127 (2010).
117. Nuckel, H. et al. The prognostic significance of soluble NKG2D ligands in B-cell chronic lymphocytic leukemia. Leukemia 24, 1152-1159 (2010).
118. Hu, J.K., Kagari, T., Clingan, J.M. & Matloubian, M. Expression of chemokine receptor CXCR3 on T cells affects the balance between effector and memory CD8 T-cell generation. Proc Natl Acad Sci U S A 108, E118-127.
119. Kohlmeier, J.E. et al. Inflammatory chemokine receptors regulate CD8(+) T cell contraction and memory generation following infection. J Exp Med 208, 1621-1634.
120. Kurachi, M. et al. Chemokine receptor CXCR3 facilitates CD8(+) T cell differentiation into short-lived effector cells leading to memory degeneration. J Exp Med 208, 1605-1620.
125. Ramsborg, C.G. & Papoutsakis, E.T. Global transcriptional analysis delineates the differential inflammatory response interleukin-15 elicits from cultured human T cells. Exp Hematol 35, 454-464 (2007).
126. Chiossone, L. et al. Molecular analysis of the methylprednisolone-mediated inhibition of NK-cell function: evidence for different susceptibility of IL-2-versus IL-15-activated NK cells. Blood 109, 3767-3775 (2007).
127. Creed, T.J. et al. The effects of cytokines on suppression of lymphocyte proliferation by dexamethasone. J Immunol 183, 164-171 (2009).
128. Goleva, E., Kisich, K.O. & Leung, D.Y. A role for STAT5 in the pathogenesis of IL-2-induced glucocorticoid resistance. J Immunol 169, 5934-5940 (2002).
129. Rentzos, M. et al. Circulating interleukin-15 and RANTES chemokine in MS patients: effect of treatment with methylprednisolone in patients with relapse. Neurol Res 32, 684-689 (2009).
130. Tliba, O. et al. Cytokines induce an early steroid resistance in airway smooth muscle cells: novel role of interferon regulatory factor-1. Am J Respir Cell Mol Biol 38, 463-472 (2008).
131. Xu, Q., Goleva, E., Ou, L.S., Li, L.B. & Leung, D.Y. CD56+ cells induce steroid resistance in B cells exposed to IL-15. J Immunol 172, 7110-7115 (2004).

### REFERENCE EXAMPLE 5

Alopecic effector T cells were identified in the lymph node and blood of alopecic mice using spectratyping of flow sorted cells. A blood-based assay was developed to monitor these alopecic T cells during treatment. Cytotoxic assays have been developed for evaluating functional components of the hair follicle (HF) interactions with CTLs, in both human and mice.

Using human biospecimens obtained from AA subjects, it was demonstrated that circulating T cells express high levels of NKG2D and primary T cells obtained from the skin are dominated by a CD8⁺NKG2D⁺ IFN-γ producing cells establishing parallels between the mouse and human disease.

### Immunophenotyping and Skin Immunobiology

To study pathways in biospecimens requires analysis of heterogeneous cell populations in the skin and in the blood. In the clinical investigation of human autoimmunity, alopecia areata provides a unique opportunity because of the accessibility of the end-organ enabling study/isolation of the pathogenic immune effectors in their relevant microenvironment. Analytic tools and personnel required will be provided for optimal study of these precious end-organ skin biospecimens, and their cellular/serological counterparts in the blood obtained from the same individual.

### Preparation and analysis of biospecimens:

The following will be provided to assist in the analysis of blood:
i) Multianalyte cytokine/chemokine analysis from serum samples.
ii) Flow cytometric immunophenotyping, including cytokine analysis of human PBMCs from AA subjects.
iii) Flow sorting for downstream applications using T cell subsets (RNA profiling, spectratyping) .

The following will be provided to assist in the analysis of skin:
i) Preparation/analysis of viable cellular fractions of both primary hair follicle and T cell components for functional (cytotoxicity) and analytic studies (T cell cloning, spectratyping).
ii) Immunostaining and transcriptional profiling of whole skin
iii) Flow cytometric immunophenotyping of dermal lymphocytes from human and mouse AA skin.

### Scientific Experiments

a. Pathogenic HF-specific cellular subsets in the peripheral blood of AA subjects will be identified. To this end, spectratype analysis will be used as a tool to identify circulating human alopecic T cells populations, as was done for the mouse model, by matching the spectratype found in total skin T cells with the spectratypes of specific sorted peripheral blood T cell subsets in the peripheral blood obtained from the same patients.
b. Th1-predominance in human AA will be established and the rationale and biomarker platform for Th1-targeted therapies will be developed. Therapeutic targeting of disease-specific Th-pathways has succeeded in other human skin inflammatory diseases, most notably in psoriasis a prototypical Th17 disease. There is substantial data in the AA animal model for AA as a predominant Th1 disease. The Th-profile of infiltrating dermal AA T cells and relevant circulating T cell subsets in the blood will be addressed using a multi-prong approach, including multianalyte, RNA profiling, immunstaining and intracellular flow cytometric analysis from the skin and blood.

An integrative approach to translational research begins with basic studies identifying targetable pathways in AA, then testing these pathways therapeutically in preclinical models, longitudinal biomarker assessment in clinical trials and pursuing population-based research based on genetic studies. The pre-clinical therapeutic effects of interventions will be evaluated in the grafted AA mouse models, treatment effects on the inflammatory responses will be assessed in the skin and blood and human biospecimens will be provided for validation of human relevance in ex vivo studies.

Monitoring the pathogenic interactions between immune cells and target cells in the skin in humans requires sophisticated processing and analysis of primary human tissues and blood.

Flow cytometry has become the primary tool for the identification of cell populations according to specific parameters, and is therefore employed by an ever-growing number of biomedical scientists.

The immunobiology of the skin presents specific characteristics and challenges; the special architecture of the tissue including its barrier functions, its associated unique immune cell populations and its close proximity/interactions with microbial flora, and the practical difficulties of isolating infiltrating lymphocytes, are all part of the complexities needed to be understood in the approach to immunological diseases of the skin. Services that provide assistance with tissue processing and staining, as well as cell isolation, and essential to insuring high quality consistent results are available.

Flow cytometry is a tool for clinical investigators and a working knowledge of its use is required for all translational immunologists. Four flow cytometers, including two workhorse instruments (FACs Canto and FACs Caliber) a 6-laser LSR II and a 4-laser BD Influx are available. The LSR II and BD Influx have been designed to have comparable lasers/detectors to facilitate transition from analysis to preparative sorting. The LSR II is equipped with 6 lasers, capable of detecting in total 19 colors simultaneously providing versatility to detect both fluorescent proteins (mBanana, GFP, BFP, RFP/dsRed, mCherry mRasberry) and fluorescent dyes/chemical fluorochromes. LSR II can analyze diverse cell types within heterogeneous cell populations from tissues using a wide array of organic and inorganic fluorochromes. These additional fluorescent parameters allow detection of coordinated functional events in specific cell types in mixed populations (e.g. intracellular IFN-gamma, IL-10 production and phosphoprotein detection in T cell populations from cutaneous tissues and draining lymph nodes). The basic LSR II instrument is equipped with three lasers; blue (488nm), red (633nm), violet (405nm). The custom designed LSR II contains in addition, three additional lasers permitting up to 20-color detection and a 96-well plate reader. "Leave one out" 10-color panel designs for T cell subsets (for example, Tregs), B cells and monocyte have been designed to take advantage of the 100W yellow-green 594 laser which has exquisite sensitivity for "red" Alexa 594. Thus, investigators may adopt a "leave-one out" design to sensitively examine expression for a specific marker of interest by staining with Alexa 594 conjugated antibodies.

Quality: Panel development for immunophenotyping lymphocyte populations in human peripheral blood and other experiments will be conducted. Because of the capacity of the 6 laser system of the LSR II, a common "easy-to use" set of colors that can be used without the need for compensation was established. The ability to do a 5/6-color experiment without the need for fluorescent compensation gives increased data resolution as the fluorochromes as far apart from each other in the spectrum, and excited independently by each laser line. This allows for the ability of populations to clearly separate, something that is rare when compensation is applied. The current selected fluorochrome combination is DAPI (gated on the DAPI negative cells) Pacific Blue, FITC, PE, Alexa Fluor 594 and APC, however other combinations of fluors are currently being tested and used. This 5-flourochrome set serves as the base platform for panels, to which additional fluorochromes can be added. In a proof-of-concept experiment, the use of 5 flurochromes was shown simultaneously without the need for compensation. C57BL/6 splenocytes were stained with CD8 Pacific Blue, CD3 FITC, CD45R (B220) PE, CD11b Bio+Streptavidin Alexa Fluor 594, and CD4 APC.

Maintenance and Quality control for Skin Immunobiology: The procedure used for the isolation of lymphocytes from skin biopsies has been adapted from a method developed by Clark and Kupper (2) in which biopsies are culture on 3-dimensional tantalum-coated cellfoam matrices to promote migration of T cells from the biopsies. Of each isolated cell populations a minor fraction will be used for CD45/CD3 staining to assess the number of leucocytes / T lymphocytes by flow cytometry prior to further studies or cryopreservation. For re-use, the matrices will be soaked for 30 min in 10% bleach, then rinsed in water and transferred into a solution of Enzyte enzyme cleaner (Decon Labs). The matrices will be left in this solution on a hot plate with stirrer for 24 hrs, rinsed with distilled water and let dry before autoclaving.

Whole skin analytic techniques (e.g., immunostaining) will be provided. The following will be provided: 1) critical expertise in skin immunobiology for the preparation/analysis of viable cellular fractions of both primary hair follicles and cutaneous T cells; 2) biomarker development capabilities for clinical biospecimens including multianalyte analysis (Luminex) and transcriptional profiling of precious clinical biospecimens (skin and blood from subjects enrolled in clinical trials). The tools to investigate biological processes and monitor the inflammatory AA mechanisms in pre-clinical and clinical samples from both the blood and the AA target end organ, the skin will be provided.

Capabilities in microarray data analysis and storage, sequence and pathway analysis are available for the studies herein, and extend all the way to the most recent algorithms for regulatory network reverse engineering. A number of widely-used databases are created and maintained. In addition, all important sequence and structure databases are maintained centrally. This allows direct large-scale searches, if need be using custom algorithms and cluster computing. Many of the research methods developed are packaged into software applications and computational services, freely available to the scientific community (http://www.c2b2.columbia.edu/page.php?pageid=10).

The following services are available:

### 1. Immunomonitoring:

### a. Multianalyte cytokine & chemokine analysis from serum samples

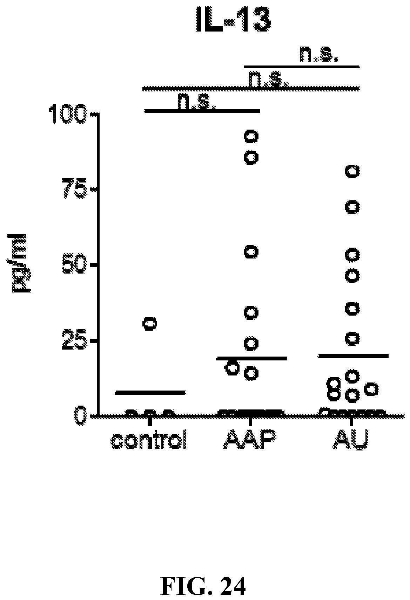

Two approaches are available: a) The multiplex bead based immunoassays systems (e.g. CBA/FlowCytomix) provides multiple analytes that allow 10 or more cytokines/chemokines to be assessed on a single read of a small volume (50 µl) sample. The LSR II 96 well high throughput capacity provides ease of use combined with efficiency. Software ("snap-to" gating) is available in house for data analysis. A multiplex bead-based assay system will also be developed to quantify soluble human and murine NKG2DL in the sera, for which specific antibody sets are commercially available. b) Multiplex analysis using the Luminex platform is available in the CTSA for analysis of commercially available mouse and human cytokine and chemokine arrays (**FIG. 24**). The Luminex assays are provided on a fee-for-service basis, with reagents purchased by the user. Once an AA serum biomarker platform bulk purchase of selected analytes will enable cost-savings.

### Flow cytometric immunophenotyping

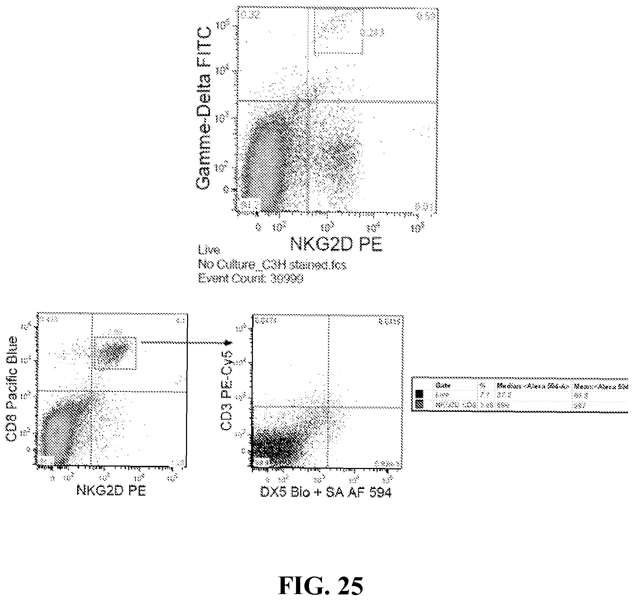

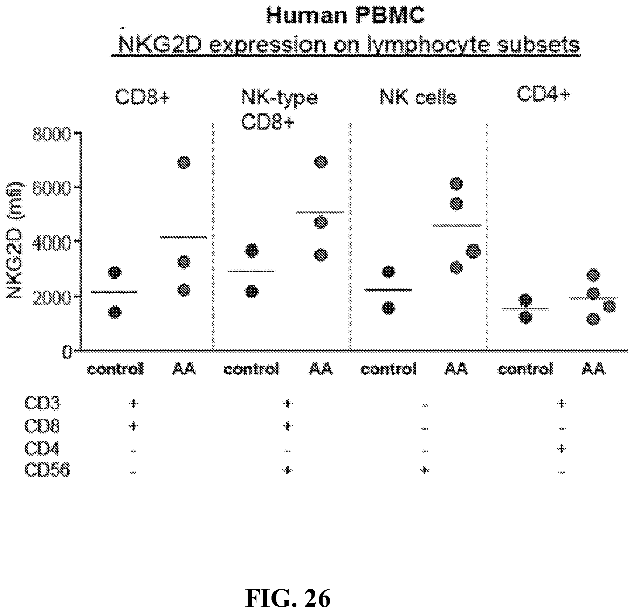

It has been established using multiparameter flow that CD8+NKG2D+ cells dominate the dermal leukocyte population harvested from the skin of mice with spontaneous alopecia (**FIG. 25**). To address the human situation, flow panels have been developed to define quantitative alterations in any of 30 PBMC subsets focusing on NK and CD4 and CD8 T cell populations using 3 FACS tubes (T cell subset tube, NK- marker tube for CD4, CD8 and NK cells and a third non-T cell tube); Lineage Markers: CD3, CD4, CD8α, CD8β, CD14, CD19, CD20, CD56, CD64; T cell subset Markers: CD45Ra, CD45Ro, CD56, CD62L, CCR7, CD127, FoxP3, Helio; Cutaneous Chemokine Receptor Analysis: CCR4, CCR8, CCR10, CLA, E and P-selectin; NK immunoreceptor family members: Inhibitory: KIR/CD158, CD94/NKG2A, LILR/ILT, LAIR1 NKR-P1 Activatory: NKp46, NKp30, NKp44, NKG2C, NKG2D. In the initial evaluation of PBMCs from four AA subjects, increased NKG2D expression was observed on CD56+ CD8+T cells and NK cells, but not on CD4+ T cells (**FIG. 26**).

Cytokine analysis: Activation of freshly isolated buffy coat peripheral T cells with PMA/ionomycin will drive cytokine production by circulating memory T cells. After a 4 hour incubation with brefeldin, cells are cell surface marker stained, fixed and permeabilized prior to intracellular staining for IL-2, IFN-γ, TNF, IL-4 , IL-17, and FOXP3/helios. These cytokines/transcription factors together with the surface markers CD3, CD4, CD8, CD25, CD56, CD62L, NKG2D, CD45Ra, CD45Ro, CLA/CCR4 will delineate the fraction of Th1/Th2/Th17/Tregs in the total and cutaneous CD4/CD8 naive and memory T cell compartment and their expression of NKG2D. Without being bound by theory, using a LSRII, which enables multi-parametric testing from a single tube, 2-4 million PBMCs will be more than sufficient for this analysis. This will leave enough PBMCs (>20 million cells) from a 30 ml blood draw to provide for other goals.

### Flow sorting for downstream applications using T cell subsets

The goal is to provide potential alopecic T cells for downstream functional analysis. For example, flow sorted T cell subsets will be provided for RNA profiling, for instance, transcriptional profiling of NK-type T cells. For biomarker studies, honing in on the appropriate cellular subset will improve the resolution of the analysis, which will otherwise be diluted by the presence of RNA from the mixed heterogenous PBMC population.

Studies in the AA mouse indicate that the alopecic T cells (**FIG. 24**), are recirculating and can be readily found and isolated from the blood and cutaneous lymph nodes. More than 5 million CD8+NKG2D+ T cells can be isolated by flow sorting from the cutaneous lymph nodes of a single mouse. This is an impressive number of fresh primary "pathogenic" T cells for downstream study and is a substantial improvement on the cell yields one can expect to recover from "crawl-outs" from alopecic skin. The identification of pathogenic T cell subsets in the blood of AA subjects human AA subjects is a scientific goal. Flow isolated T cell subsets will be provided for downstream applications including biomarker studies, functional studies and spectratyping to provide evidence for immuopathogenic relevance of specific T cells subsets.

### i) Spectratype Analysis of TCR repertoire

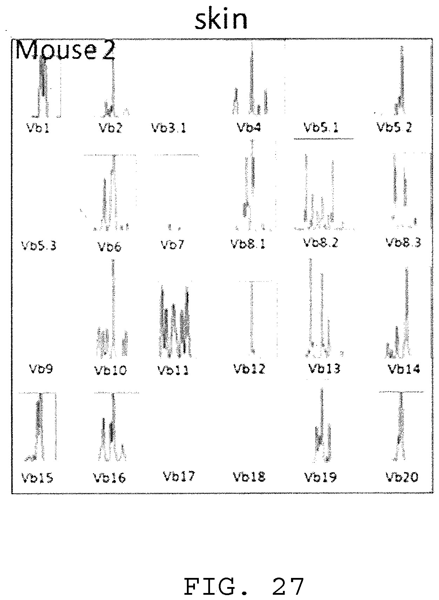

Spectratyping or TCR β-chain length distribution analysis of RNA from tissues or phenotypically separated lymphocyte subsets provides a qualitative portrait of the TCR clonotype utilization/repertoire of the infiltrating T cells. In recent years this technique has been used by others (5-7) as a highly sensitive and accurate method to delineate the proportion of clonally expanded T cells in a sample. Oligoclonality, evidence of expansion of subsets of T cells with restricted TCRs is indicative of antigenic drive in inflammatory processes and can be used as a first step to identify pathogenic T cell clones. Since each TCR rearrangement varies in CDR3 length in multiples of three nucleotides, heterogeneity of CDR3 length within a population of T cells can be used as a measure of TCR diversity. The principle of this technique is to PCR amplify the cDNA obtained from a T cell population using an upstream specific Vβ family member primers and a downstream constant region primer that together span the CDR3 region made by combinatorial and junctional VDJ joining. The PCR products are then fluorochrome-labelled in a primer extension ("run-off") reaction using a fluorescently labeled constant region primer. The products are then run on the ABI PRISM 3700 DNA analyzer. With Gene Mapper software the size of the peaks corresponding to discrete CDR3 lengths can be displayed and analyzed (**FIGS. 20** and **27**). The data are exported and compared to reference polyclonal repertoires, and measures of oligoclonality such as the Hamming distance are calculated. The area of the peaks in each histogram reflects the expression frequency of each CDR3 length within each family. A predominance of clonal expansions in the skin indicates a primary role for dermal antigens in driving disease and will enable focused investigation of pathogenic T cells. In contrast a polyclonal repertoire of unexpanded clones will indicate recruitment by non-clonally specific chemokine receptors. Comparisons of the TCR repertoire in non-lesional skin and blood of the same individuals will indicate whether clonally expanded skin resident T cell populations might also be found in the circulation. Similarly sorted T cell subsets can be used for source RNA material for repertoire analysis. For instance, **FIGS**. **20** and **27** demonstrates that in C3H mice an oligoclonal population is found in the lesional skin, a repertoire that is also over-represented in NKG2D positive but not negative CD8 lymph node populations. These data confirm that CD8+NKG2D+ T cells contain most (but not all) of the oligoclonal T cell populations found in the skin. Note that some TCR/clones are found in skin but not in draining cutaneous lymph nodes, e.g., Vβ10. These likely include CD4+ T cell populations found in AA skin but not present in the sorted CD8+ LN fraction. This approach provides powerful evidence for "antigen-drive", and combined with flow sorting as shown here, identifies pathogenic T cell subsets amongst populations of cells.

This evidence of clonal sharing or oligoclonality will be investigated and validated in mouse and human samples at the level of clonotype resolution by preparing bacterial libraries of the PCR products using topoisomerase-based cloning, and sequencing the TCR β-chain of resulting clones in a high throughput manner using 96-well based techniques as described (5-7). Usually 48 or 96 clones are selected and sequenced for each PCR product. The sequences are exported and aligned in Geneious and the fine structure of VDJ element usage and joining is determined using Vquest. This allows definitive identification of shared clones and lymphocyte trafficking in different regions, as well as precise enumeration of the clonotypic composition of a sample. Furthermore, clonotypic TCRs can be used for gene transduction studies to generate T cells and retrogenic mice expressing alopecic TCRs. This effort can also lead to development of "humanized" transgenic TCR models of alopecia using transgenically expressed human alopecic TCRs in HLA02 transgenic mice.

### ii) Transcriptional biomarkers of circulating immune cells

A transcriptional signature marking aggressive, drug refractory Lupus can be identified within the CD8 T cell compartment, but not when using RNA of total PBMCs (8) Thus combining flow sorting with transcriptional profiling greatly powers the analytic capacity. In alopecia areata the pathogenic cellular subset in the appears to be CD8+NKG2Dhi T cells that envelop the hair follicle and may be of pathogenic relevance broadly in autoimmunity (9) including both celiac disease (4, 10), Type I diabetes (11) and rheumatoid arthritis (12-14). Transcriptional profiling of these cells in celiac disease (4, 10) previously described an NK-like transcriptional programming of these cells that will be similarly assessed in sort-purified circulating and skin-derived CD8 T cells. It will be important to specifically address the transcriptional profiles of these cellular populations to identify biological pathways central to these cells.

Epigenetically regulated autoimmune genes using Illumina-based genomic approaches were identified and DNA was obtained from sorted autoimmune human T cells from patients with Type I diabetes, alopecia areata and celiac disease. Hypermethylation of the promoter regions that downregulate the expression of important immunoregulatory genes including CD3, PD-1 and FasL was identified.

### iii) Cellular Immunology

Flow cytometryic analysis of activated PBMCs can be used for intracellular cytokine analysis and or signaling activation of phosphorylated proteins. Classic antigen specific, mitogenic or mixed lymphocyte responses can be appreciated with CFSE-stained T cells or with thymidine incorporation. Available equipment includes a cell harvester and Microbeta/Trilux plate reader (for thymidine based proliferation and chromium release cytotoxicity assays), ELISA plate readers/washers, ELISPOT readers.

### Skin immunobiology

Preparation of primary hair follicle and T cell components for functional (cytotoxicity), preparative (T cell cloning) and analytic studies (spectratyping, RNA profiling). Biopsy specimens from AA patients on study are precious, with immunostaining and transcriptional profiling studies taking the highest priority given the feasibility and informative power of these two approaches. Routinely human scalp skin is procured from control individuals (hair transplant donors) to establish primary cultures of individual cellular populations within the skin and hair follicle.

### a) Culture of T cells and hair follicle components, hair follicle organ culture and skin organ culture.

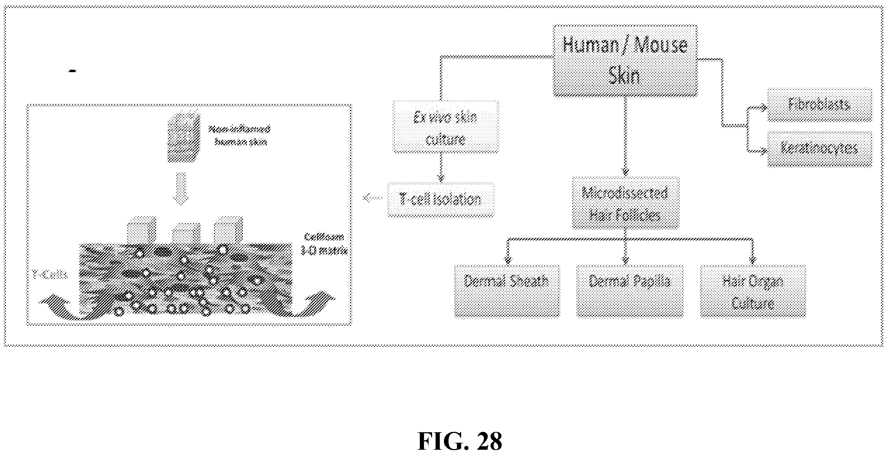

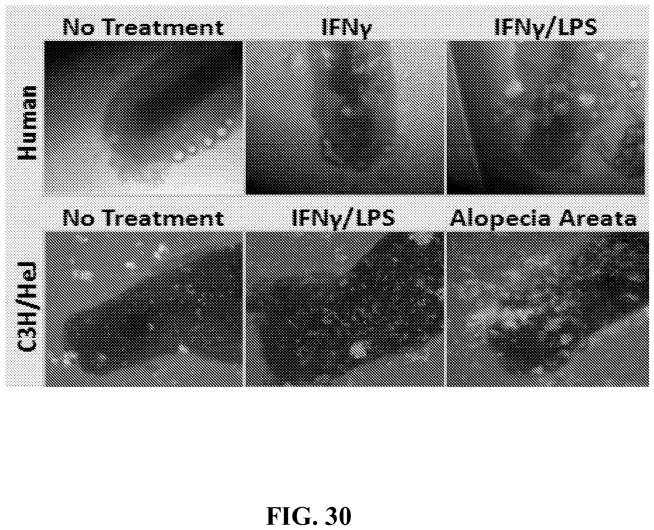

i) HF targets: Human scalp skin procured from control or AA affected individuals will be used to establish primary cultures of individual cellular populations within the skin and hair follicle (**FIG. 28**) to study the regulation of NKG2DL in primary HF populations and to evaluate HF populations as targets in CTL assays. Interfollicular skin will be dispase treated to separate the epidermal and dermal components and enzymatically processed to establish primary cultures of keratinocytes and fibroblasts. The hair follicles will be microdissected to separate the mesenchymal components and further used to culture dermal sheath cells (DSC) and dermal papilla cells (DPC). Serum free hair follicle organ culture established in the laboratory modeled after protocols from Kondo and Philpott et al (15) (**FIG. 30**) will be used to routinely culture individual follicles from control individuals, as well as lesional (when possible) and control skin from AA patients. The follicles are cultured in serum free growth conditions and show normal anagen growth for 7-10 days, followed by catagen entry and disintegration of the matrix (16). For cytotoxicity assays, the follicles will be transferred to media which sustains cytotoxic T-Cells or NK cells (MyeloCult, Stemcell Technologies) for 4-8 hrs. The skin explants maintain normal skin architecture, as well as the immune microenvironment, and will be used for functional studies (17).
ii) T cell effectors: Isolation of sufficient numbers of T cells from enzymatically digested/dispersed human skin can be difficult; however cultured T cells weren expanded using the dermal crawl-out approach developed by Clark (2). Skin explants have been used to expand the resident T-cell population of the skin by culturing the explants on cell foam matrices for 3 weeks in IMDM containing 20% FCS, IL-2 and IL-15. This technique yields 0.3-3.0 x 106 T cells per 4mm biopsy enabling immunophenotyping, T cell cloning, Th-profiling, and other downstream applications (e.g., transcriptional profiling, cytotoxicity and other functional assays). For AA patients on intervention trials, if sufficient T cells are obtained after three weeks of expansion (>1x10⁶) they will be divided, with half the population used for RNA isolation/profiling and the other half used for flow immunophenotyping/Th profiling. If the yields are less than 1x106 total cells, studies will be limited to RNA profiling. For AA patients that are not on study, there will be more latitude for using crawl-out T cells and autologous HF targets for alternative functional studies, including for instance cytolyic assays and studies with antibody blockade/small molecules. As an example of the ability to analyse rare clinical populations of T cells, **FIG. 29** illustrates a T cell 'crawl-out" assay, in which cultured human dermis obtained from punch skin biopsies are used as source material for flow cytometric analysis.

Note that T cells obtained from AA skin biopsies are oligoclonal IFN-γ□ producing CD8+NKG2D+ T cells in striking contrast to the expected polyclonal CD4 population seen in crawl-outs from normal skin (2, 18).

### b.) Interaction of the skin and the immune components in Alopecia Areata models

Established protocols are available for assessing skin-immune interactions for the studies herein. Primary cultured cells, as well as organ-cultured follicles, can be used as targets using cytotoxic T cells from control or patient peripheral blood lymphocytes, or those derived from the skin (**FIG. 29**). The immune effector cells can be co-cultured with CFSE-labeled target cells/hair follicles and the cytolysis will be measured colorimetrically by LDH release, or alternatively, the dead cells will be stained with 7-AAD and the cell numbers counted by flow cytometry. These assays can be used to determine molecular requirements for the interaction of immune effectors and HF targets, including cytotoxicity. For instance as shown here, cytotoxicity required NKG2D engagement and prior sensitization of targets with cytokines/TLR ligands known to upregulate NKG2DL transcription and surface expression.

### Evaluation of skin biomarkers of pathogenesis: Immunostaining and transcriptional profiling

AA shows high correlation with several autoimmune disorders, making the hair follicle a highly accessible organ in which to study basic mechanisms of autoimmunity. There has been substantial interest in identifying early surrogate biological markers of pathogenesis. As such, AA represents a model system for biomarker development which may have relevance to a broad range of diseases. The following biomarkers are currently used to monitor AA development and response to treatment both in humans and in mice.
a) Immunohistochemical Staining for lymphocytic Infiltration. AA is associated with the presence of intrafollicular, and parafollicular immune infiltrate. For immunohistochemistry, tissue will be either fixed in 10% formalin in PBS for 8 hours at room temperature and stored in 70% ethanol for paraffin sections or embedded directly in Cryomatrix (Shandon, Waltham, MA) on dry ice for frozen sections which are stored at - 80°C.
   Paraffin-embedded tissue blocks are cut into 8-µm sections, and hematoxylin and eosin (H&E) staining is carried out for histological studies. Frozen tissue is also sectioned to a thickness of 8 µm and fixed in 4% paraformaldehyde for immunofluorescence staining. Sections are stained with fluorescence-labeled secondary antibodies and immunofluorescence imaging carried out using Zeiss Axioskop microscope. Basic immunohistological/fluorescent staining will include staining with primary antibodies to CD3, CD4, CD8. As an example of the immunostains, evidence that MHC I and II and ICAM-1 are massively upregulated in alopecic HFs which are associated with a dense CD8 dominated infiltrate is provided (**FIG**. **31**).
b) Isolation of total skin RNA and Quantitative PCR for inflammatory markers. The human and mouse skin will be tested for elevation of inflammatory gene transcripts already defined, including IFN-response genes, and gene signatures to be defined, including "NK-type" CD8 T cell transcripts. Total RNA will be extracted from skin using an RNeasy purification kit (Qiagen). DNase-treated total RNA will be reverse-transcribed using SuperScript II reverse transcriptase (Invitrogen, Carlsbad, CA).

RT-PCR shall be performed using SYBR Green Master Mix and an ABI Prism 7000 Sequence Detection System (Applied Biosystems, Foster City, CA). GAPDH and β-actin will be used as internal normalization control genes.

One of the goals of the studies herein is to establish the phenotype of circulating AA effector populations by matching spectratypes of sorted peripheral blood AA T cell subsets with those found in total skin T cells from the same patient. Flow cytometric sorting techniques will be combiened with spectratype analysis to identify pathogenic T cells in the circulation of AA patients. This same approach was used in the mouse model to show that CD8+NKG2D+ lymph node cells contain the majority of the T cell clones found in alopecic skin. In AA subjects, the TCR repertoire will be compared in lesional and non-lesional skin and blood of the same individuals to identify whether clonally expanded skin resident T cell populations are also be found in the circulation. By progressive refinement of the sort criteria to isolate source RNA material from circulating T cell subsets one should be able to (at least partially) match the TCR repertoire found in total skin from the same patient and in this way indentify the immunophenotypic markers of pathogenic CD4 and CD8 T cells. Identification of pathogenic T cell subsets will be enormously valuable for monitoring clinical studies, refining biomarker development and would provide unique cellular materials for bedside-to-bench studies of pathogenesis.

Another goal of the studies herein is to establish the Th profile of AA T cells infiltrating the dermis and circulating in the peripheral blood. Pathogenic AA T cell Effector Differentiation/Cytokine Profile: Therapeutic targeting of specific Th-pathways has succeeded in other human skin inflammatory diseases, most notably in psoriasis a prototypical Th17 disease. There is substantial data in the AA animal model for AA as a predominant Th1 disease (19-23). To establish Th1-predominance in humans and develop the rationale and biomarker platform for Th1-targeted therapies, the Th-profile of infiltrating dermal AA T cells and relevant circulating T cell subsets in the blood will be addressed using a multi-prong approach including multianalyte, RNA profiling, immunostaining and intracellular flow cytometric analysis from the skin and blood. Refining the immunophenotypic markers for AA CD4 and CD8 T cells will of course allow detection of Th-profiles of the potential alopecic-specific T cells amongst the polyclonal circulating population.

Although transcriptional profiling of AA lesions from a small series of subjects are indicative of Th1-type skewing (24), without being bound by theory, AA is not "one disease". The common occurrence in AA subjects of co-morbid immune conditions that reflect both an underlying Th2 or Th1/17 bias that predisposes to diseases with common pathogenic pathways. including hypersensitivity (25) (dermatitis) and other autoimmune conditions (26-33) (see studies described herein) suggests that there may be a Th2-type AA as well as a Th1 or Th17 type of AA. Thus it is important to identify functional T cell responses in PBMCs from the periphery and from AA lesional tissue explants from a large number of patients that might identify subsets of AA with different Th profiles and to correlate these immune function biomarker parameters with the presence or absence of the relevant SNPs.

The T cell immunophenotype of peripheral T cells and lesional T cells will be assessed. 50 AA patients and 50 normal controls will be studied from the GWAS, with attached clinical history. Assessment of Th-profiles of total circulating T cells is straight forward but multi-parametric flow will be applied to T cell subsets for more granular assessment, as aided ultimately by definition of immunophenotypic markers of AA pathogenic populations. Isolation of sufficient numbers of T cells from enzymatically digested/dispersed human skin can be difficult, and maybe altered by culture conditions. Correlation will be performed with transcriptional profiling using whole skin approaches and microdissected skin samples to describe the cytokine profile within the follicular sheath infiltrate as well as from infiltrates in the interfollicular epithelium.

### Human Subjects

Skin biopsies from the scalp will be collected from normal subjects consist of AA patients and unaffected control subjects receiving hair transplantation. Additional biopsies will be obtained from subjects on clinical trials.

The sources of research material will be scalp skin biopsies from AA patients and controls. RNA from small tissue samples, including microdissected hair follicle compartments are routinely obtained. Tissue biopsies from AA patients and controls will be provided.

### Vertebrate Animals

Young C3H/HeJ mice (2-3 months) and retired breeders showing visible hair loss will be acquired from Jackson Labs. The mice strains established by each participating principle investigator will be housed in their respective animal housing suites. Mice will be given free access to water and pellet diet (5010 rodent diet, LabDiet, PMI Nutrition International).

Primary cell culture for individual skin and follicle components as well as hair/skin organ cultures will be established for wild-type and transgenic or knock-out mice. These studies also involve hair follicle and skin tissue characterization. The mice will be euthanised by CO2 asphyxiation followed by cervical dislocation, a veterinary-approved and widely recommended method. It is the quickest and most humane method that is not associated with any pain or stress for the animals. Mice will be shaved on the dorsal surface with electric clipper and then skin tissue will be dissected out. The isolation scheme of individual cellular components of the skin and hair follicle as well the T-cells isolation from skin explants is described in **FIGS. 25****,** **28** **and** **29**. Skin Samples will also be embedded in OCT and paraffin. In addition to skin, spleen and thymus will also be dissected from the animals and further used for flow cytometry. Serum from the animals will also be collected by tail bleeding and used for cytokine profiling.

### References

1. de Jong, A., Pena-Cruz, V., Cheng, T.Y., Clark, R.A., Van Rhijn, I., and Moody, D.B. 2010. CD1a-autoreactive T cells are a normal component of the human alphabeta T cell repertoire. Nat Immunol 11:1102-1109.
2. Clark, R.A., Chong, B.F., Mirchandani, N., Yamanaka, K., Murphy, G.F., Dowgiert, R.K., and Kupper, T.S. 2006. A novel method for the isolation of skin resident T cells from normal and diseased human skin. J Invest Dermatol 126:1059-1070.
3. Bhagat, G., Naiyer, A.J., Shah, J.G., Harper, J., Jabri, B., Wang, T.C., Green, P.H., and Manavalan, J.S. 2008. Small intestinal CD8+TCRgammadelta+NKG2A+ intraepithelial lymphocytes have attributes of regulatory cells in patients with celiac disease. J Clin Invest 118:281-293.
4. Meresse, B., Chen, Z., Ciszewski, C., Tretiakova, M., Bhagat, G., Krausz, T.N., Raulet, D.H., Lanier, L.L., Groh, V., Spies, T., et al. 2004. Coordinated induction by IL15 of a TCR-independent NKG2D signaling pathway converts CTL into lymphokine-activated killer cells in celiac disease. Immunity 21:357-366.
5. Winchester, R., Wiesendanger, M., O'Brien, W., Zhang, H.Z., Maurer, M.S., Gillam, L.D., Schwartz, A., Marboe, C., and Stewart, A.S. 2011. Circulating activated and effector memory T cells are associated with calcification and clonal expansions in bicuspid and tricuspid valves of calcific aortic stenosis. J Immunol 187:1006-1014.
6. Wu, H.D., Maurer, M.S., Friedman, R.A., Marboe, C.C., Ruiz-Vazquez, E.M., Ramakrishnan, R., Schwartz, A., Tilson, M.D., Stewart, A.S., and Winchester, R. 2007. The lymphocytic infiltration in calcific aortic stenosis predominantly consists of clonally expanded T cells. J Immunol 178:5329-5339.
7. Curran, S.A., FitzGerald, O.M., Costello, P.J., Selby, J.M., Kane, D.J., Bresnihan, B., and Winchester, R. 2004. Nucleotide sequencing of psoriatic arthritis tissue before and during methotrexate administration reveals a complex inflammatory T cell infiltrate with very few clones exhibiting features that suggest they drive the inflammatory process by recognizing autoantigens. J Immunol 172:1935-1944.
8. McKinney, E.F., Lyons, P.A., Carr, E.J., Hollis, J.L., Jayne, D.R., Willcocks, L.C., Koukoulaki, M., Brazma, A., Jovanovic, V., Kemeny, D.M., et al. 2010. A CD8+ T cell transcription signature predicts prognosis in autoimmune disease. Nat Med 16:586-591, 581p following 591.
9. Van Belle, T.L., and von Herrath, M.G. 2009. The role of the activating receptor NKG2D in autoimmunity. Mol Immunol 47:8-11.
10. Meresse, B., Curran, S.A., Ciszewski, C., Orbelyan, G., Setty, M., Bhagat, G., Lee, L., Tretiakova, M., Semrad, C., Kistner, E., et al. 2006. Reprogramming of CTLs into natural killer-like cells in celiac disease. J Exp Med 203:1343-1355.
11. Ogasawara, K., Hamerman, J.A., Ehrlich, L.R., Bour-Jordan, H., Santamaria, P., Bluestone, J.A., and Lanier, L.L. 2004. NKG2D blockade prevents autoimmune diabetes in NOD mice. Immunity 20:757-767.
12. Andersson, A.K., Sumariwalla, P.F., McCann, F.E., Amjadi, P., Chang, C., McNamee, K., Tornehave, D., Haase, C., Agerso, H., Stennicke, V.W., et al. 2011. Blockade of NKG2D ameliorates disease in mice with collagen-induced arthritis: A potential pathogenic role in chronic inflammatory arthritis. Arthritis Rheum 63:2617-2629.
13. Groh, V., Bruhl, A., El-Gabalawy, H., Nelson, J.L., and Spies, T. 2003. Stimulation of T cell autoreactivity by anomalous expression of NKG2D and its MIC ligands in rheumatoid arthritis. Proc Natl Acad Sci U S A 100:9452-9457.
14. Killock, D. 2011. Experimental arthritis: NKG2D: a potential therapeutic target in RA? Nat Rev Rheumatol 7:438.
15. Philpott, M.P., Sanders, D.A., and Kealey, T. 1996. Whole hair follicle culture. Dermatol Clin 14:595-607.
16. Kondo, S., Hozumi, Y., and Aso, K. 1990. Organ culture of human scalp hair follicles: effect of testosterone and oestrogen on hair growth. Arch Dermatol Res 282:442-445.
17. Companjen, A.R., van der Wel, L.I., Wei, L., Laman, J.D., and Prens, E.P. 2001. A modified ex vivo skin organ culture system for functional studies. Arch Dermatol Res 293:184-190.
18. Clark, R.A., Chong, B., Mirchandani, N., Brinster, N.K., Yamanaka, K., Dowgiert, R.K., and Kupper, T.S. 2006. The vast majority of CLA+ T cells are resident in normal skin. J Immunol 176:4431-4439.
19. Freyschmidt-Paul, P., McElwee, K.J., Hoffmann, R., Sundberg, J.P., Vitacolonna, M., Kissling, S., and Zoller, M. 2006. Interferon-gamma-deficient mice are resistant to the development of alopecia areata. Br J Dermatol 155:515-521.
20. Hirota, R., Tajima, S., Yoneda, Y., Okada, M., Tashiro, J., Ueda, K., Kubota, T., and Yoshida, R. 2003. Induction of hair regrowth in the alopecia site of IFN-gamma knockout mice by allografting and IFN-gamma injection into the transplantation site. J Interferon Cytokine Res 23:433-439.
21. Gilhar, A., Kam, Y., Assy, B., and Kalish, R.S. 2005. Alopecia areata induced in C3H/HeJ mice by interferon-gamma: evidence for loss of immune privilege. J Invest Dermatol 124:288-289.
22. Skurkovich, S., Korotky, N.G., Sharova, N.M., and Skurkovich, B. 2005. Treatment of alopecia areata with anti-interferon-gamma antibodies. J Investig Dermatol Symp Proc 10:283-284.
23. Nakamura, M., Jo, J., Tabata, Y., and Ishikawa, O. 2008. Controlled delivery of T-box21 small interfering RNA ameliorates autoimmune alopecia (Alopecia Areata) in a C3H/HeJ mouse model. Am J Pathol 172:650-658.
24. Subramanya, R.D., Coda, A.B., and Sinha, A.A. 2010. Transcriptional profiling in alopecia areata defines immune and cell cycle control related genes within disease-specific signatures. Genomics 96:146-153.
25. Barahmani, N., Lopez, A., Babu, D., Hernandez, M., Donely, S.E., and Duvic, M. 2009. Serum T helper 1 cytokine levels are greater in patients with alopecia areata regardless of severity or atopy. Clin Exp Dermatol.
26. Chu, S.Y., Chen, Y.J., Tseng, W.C., Lin, M.W., Chen, T.J., Hwang, C.Y., Chen, C.C., Lee, D.D., Chang, Y.T., Wang, W.J., et al. 2011. Comorbidity profiles among patients with alopecia areata: The importance of onset age, a nationwide population-based study. J Am Acad Dermatol.
27. Du Vivier, A., and Munro, D.D. 1975. Alopecia areata, autoimmunity, and Down's syndrome. Br Med J 1:191-192.
28. Goh, C., Finkel, M., Christos, P.J., and Sinha, A.A. 2006. Profile of 513 patients with alopecia areata: associations of disease subtypes with atopy, autoimmune disease and positive family history. J Eur Acad Dermatol Venereol 20:1055-1060.
29. Kasumagic-Halilovic, E. 2008. Thyroid autoimmunity in patients with alopecia areata. Acta Dermatovenerol Croat 16:123-125.
30. Puavilai, S., Puavilai, G., Charuwichitratana, S., Sakuntabhai, A., and Sriprachya-Anunt, S. 1994. Prevalence of thyroid diseases in patients with alopecia areata. Int J Dermatol 33:632-633.
31. Seyrafi, H., Akhiani, M., Abbasi, H., Mirpour, S., and Gholamrezanezhad, A. 2005. Evaluation of the profile of alopecia areata and the prevalence of thyroid function test abnormalities and serum autoantibodies in Iranian patients. BMC Dermatol 5:11.
32. Wang, S.J., Shohat, T., Vadheim, C., Shellow, W., Edwards, J., and Rotter, J.I. 1994. Increased risk for type I (insulin-dependent) diabetes in relatives of patients with alopecia areata (AA). Am J Med Genet 51:234-239.
33. Yano, S., Ihn, H., Nakamura, K., Okochi, H., and Tamaki, K. 1999. Antinuclear and antithyroid antibodies in 68 Japanese patients with alopecia areata. Dermatology 199:191.

### EXAMPLE 6

In vivo pre-clinical studies of blocking Jak1/Jak2 was performed using the INCB018424 Jak1/Jak2 inhibitor in the grafted animal model of the disease, C3H-HeJ mice. 0/5 mice developed alopecia after treatment with INCB018424, whereas 2/5 placebo treated mice developed AA.

## Claims

1. A Jak1 and/or Jak2 inhibitor selected from the group consisting of: AG490 with the CAS No. 133550-30-8; CYT387 with the CAS No. 1056634-68-4; SB1518 with the CAS No. 937272-79-2; TG101348 with the CAS No. 936091-26-8; BMS-911543 with the CAS No. 1271022-90-2; WP-1034 with the CAS No. 857064- 42-7; CEP-701 with the CAS No. 111358-88-4; INCB 018424 with the CAS No. 941678-49-5; and LY3009104 with the CAS No. 1187594-09-7 for use in a method of treatment of a hair-loss disorder selected from the group consisting of alopecia areata and androgenetic alopecia in a mammalian subject in need thereof.

2. The Jak1 and/or Jak2 inhibitor for use according to claim 1, wherein treatment is achieved by inducing hair growth in the subject.

3. The Jak1 and/or Jak2 inhibitor for use according to claim 1 or 2, wherein the inhibitor is INCB 018424 with the CAS No. 941678-49-5.

4. The Jak1 and/or Jak2 inhibitor for use according to claim 1 or 2, wherein the inhibitor is LY3009104 with the CAS No. 1187594-09-7.

5. The Jak1 and/or Jak2 inhibitor for use according to claim 1 or 2, wherein the inhibitor is AG490 with the CAS No. 133550-30-8; CYT387 with the CAS No. 1056634-68-4; SB1518 with the CAS No. 937272-79-2; TG101348 with the CAS No. 936091-26-8; BMS-911543 with the CAS No. 1271022-90-2; WP-1034 with the CAS No. 857064-42-7, or CEP-701 with the CAS No. 111358-88-4.

6. The Jak1 and/or Jak2 inhibitor for use according to claim 1 or 2, wherein the hair loss disorder is alopecia areata.

7. The Jak1 and/or Jak2 inhibitor for use according to claim 1 or 2, wherein the hair loss disorder is androgenetic alopecia.

8. The Jak1 and/or Jak2 inhibitor for use according to claim 6, wherein the alopecia areata is alopecia totalis or alopecia universalis.

9. The Jak1 and/or Jak2 inhibitor for use according to claim 1, wherein the Jak1 and/or Jak2 inhibitor is in a composition suitable for intravenous, intramuscular, intra-peritoneal, intradermal, subcutaneous, oral, nasal, transdermal, topical, transmucosal, or rectal administration.

10. The Jak1 and/or Jak2 inhibitor for use according to claim 2, wherein the Jak1 and/or Jak2 inhibitor is in a composition suitable for intravenous, intramuscular, intra-peritoneal, intradermal, subcutaneous, oral, nasal, transdermal, topical, transmucosal, or rectal administration.

## Patentansprüche

1. Jak1- und/oder Jak2-Inhibitor, ausgewählt aus der Gruppe bestehend aus: AG490 mit der CAS-Nr. 133550-30-8; CYT387 mit der CAS-Nr. 1056634-68-4; SB1518 mit der CAS-Nr. 937272-79-2; TG101348 mit der CAS-Nr. 936091-26-8; BMS-911543 mit der CAS-Nr. 1271022-90-2; WP-1034 mit der CAS-Nr. 857064-42-7; CEP-701 mit der CAS-Nr. 111358-88-4; INCB 018424 mit der CAS-Nr. 941678-49-5; und LY3009104 mit der CAS-Nr. 1187594-09-7 zur Verwendung bei einem Verfahren zur Behandlung einer aus der Gruppe bestehend aus Alopecia areata und androgenetischer Alopezie ausgewählten Haarausfallerkrankung bei einem eine solche Behandlung benötigenden Säugerindividuum.

2. Jak1- und/oder Jak2-Inhibitor zur Verwendung nach Anspruch 1, wobei die Behandlung durch Induzieren von Haarwuchs bei dem Individuum bewerkstelligt wird.

3. Jak1- und/oder Jak2-Inhibitor zur Verwendung nach Anspruch 1 oder 2, wobei es sich bei dem Inhibitor um INCB 018424 mit der CAS-Nr. 941678-49-5 handelt.

4. Jak1- und/oder Jak2-Inhibitor zur Verwendung nach Anspruch 1 oder 2, wobei es sich bei dem Inhibitor um LY3009104 mit der CAS-Nr. 1187594-09-7 handelt.

5. Jak1- und/oder Jak2-Inhibitor zur Verwendung nach Anspruch 1 oder 2, wobei es sich bei dem Inhibitor um AG490 mit der CAS-Nr. 133550-30-8; CYT387 mit der CAS-Nr. 1056634-68-4; SB1518 mit der CAS-Nr. 937272-79-2; TG101348 mit der CAS-Nr. 936091-26-8; BMS-911543 mit der CAS-Nr. 1271022-90-2; WP-1034 mit der CAS-Nr. 857064-42-7 oder CEP-701 mit der CAS-Nr. 111358-88-4 handelt.

6. Jak1- und/oder Jak2-Inhibitor zur Verwendung nach Anspruch 1 oder 2, wobei es sich bei der Haarausfallerkrankung um Alopecia areata handelt.

7. Jak1- und/oder Jak2-Inhibitor zur Verwendung nach Anspruch 1 oder 2, wobei es sich bei der Haarausfallerkrankung um androgenetische Alopezie handelt.

8. Jak1- und/oder Jak2-Inhibitor zur Verwendung nach Anspruch 6, wobei es sich bei der Alopecia areata um Alopecia totalis oder Alopecia universalis handelt.

9. Jak1- und/oder Jak2-Inhibitor zur Verwendung nach Anspruch 1, wobei der Jak1- und/oder Jak2-Inhibitor in einer Zusammensetzung vorliegt, die zur intravenösen, intramuskulären, intraperitonealen, intradermalen, subkutanen, oralen, nasalen, transdermalen, topischen, transmukosalen oder rektalen Verabreichung geeignet ist.

10. Jak1- und/oder Jak2-Inhibitor zur Verwendung nach Anspruch 2, wobei der Jak1- und/oder Jak2-Inhibitor in einer Zusammensetzung vorliegt, die zur intravenösen, intramuskulären, intraperitonealen, intradermalen, subkutanen, oralen, nasalen, transdermalen, topischen, transmukosalen oder rektalen Verabreichung geeignet ist.

## Revendications

1. Inhibiteur de Jak1 et/ou Jak2 choisi dans le groupe constitué de : AG490 avec CAS n° 133550-30-8 ; CYT387 avec CAS n° 1056634-68-4 ; SB1518 avec CAS n° 937272-79-2 ; TG101348 avec CAS n° 936091-26-8 ; BMS-911543 avec CAS n° 1271022-90-2 ; WP-1034 avec CAS n° 857064-42-7 ; CEP-701 avec CAS n° 111358-88-4 ; INCB 018424 avec CAS n° 941678-49-5 ; et LY3009104 avec CAS n° 1187594-09-7 pour utilisation dans un procédé de traitement d'un trouble de perte des cheveux choisi dans le groupe constitué de la pelade et l'alopécie androgénétique chez un sujet mammifère en ayant besoin.

2. Inhibiteur de Jak1 et/ou Jak2 pour utilisation selon la revendication 1, le traitement étant obtenu par induction de la croissance des cheveux chez le sujet.

3. Inhibiteur de Jak1 et/ou Jak2 pour utilisation selon la revendication 1 ou 2, l'inhibiteur étant INCB 018424 avec CAS n° 941678-49-5.

4. Inhibiteur de Jak1 et/ou Jak2 pour utilisation selon la revendication 1 ou 2, l'inhibiteur étant LY3009104 avec CAS n° 1187594-09-7.

5. Inhibiteur de Jak1 et/ou Jak2 pour utilisation selon la revendication 1 ou 2, l'inhibiteur étant AG490 avec CAS n° 133550-30-8 ; CYT387 avec CAS n° 1056634-68-4 ; SB1518 avec CAS n° 937272-79-2 ; TG101348 avec CAS n° 936091-26-8 ; BMS-911543 avec CAS n° 1271022-90-2 ; WP-1034 avec CAS n° 857064-42-7, ou CEP-701 avec CAS n° 111358-88-4.

6. Inhibiteur de Jak1 et/ou Jak2 pour utilisation selon la revendication 1 ou 2, le trouble de perte des cheveux étant la pelade.

7. Inhibiteur de Jak1 et/ou Jak2 pour utilisation selon la revendication 1 ou 2, le trouble de perte des cheveux étant l'alopécie androgénétique.

8. Inhibiteur de Jak1 et/ou Jak2 pour utilisation selon la revendication 6, la pelade étant la pelade totale ou la pelade universelle.

9. Inhibiteur de Jak1 et/ou Jak2 pour utilisation selon la revendication 1, l'inhibiteur de Jak1 et/ou Jak2 étant dans une composition adaptée pour administration intraveineuse, intramusculaire, intrapéritonéale, intradermique, sous-cutanée, orale, nasale, transdermique, topique, transmuqueuse ou rectale.

10. Inhibiteur de Jak1 et/ou Jak2 pour utilisation selon la revendication 2, l'inhibiteur de Jak1 et/ou Jak2 étant dans une composition adaptée pour administration intraveineuse, intramusculaire, intrapéritonéale, intradermique, sous-cutanée, orale, nasale, transdermique, topique, transmuqueuse ou rectale.
